# EUROPÄISCHE PATENTSCHRIFT

(11) **EP 3 558 387 B1**
(45) Veröffentlichungstag und Bekanntmachung des Hinweises auf die Patenterteilung: **20.10.2021**
(21) Anmeldenummer: 17837872.5
(22) Anmeldetag: 18.12.2017
(51) Int. Cl.: A61K 47/68, A61K 47/65, A61P 35/00

(54) **SPEZIFISCHE ANTIKÖRPER-WIRKSTOFF-KONJUGATE (ADCS) MIT KSP-INHIBITOREN**
SPECIFIC ANTIBODY DRUG CONJUGATES (ADCS) HAVING KSP INHIBITORS
CONJUGUÉS ANTICORPS-PRINCIPE ACTIF (ADC) SPÉCIFIQUES RENFERMANT DES INHIBITEURS DE KSP

(30) Priorität: 21.12.2016 EP 16205870
(43) Veröffentlichungstag der Anmeldung: 30.10.2019
(73) Patentinhaber: Bayer Pharma Aktiengesellschaft, 13353 Berlin (DE)
(72) Erfinder: LERCHEN, Hans-Georg, 51375 Leverkusen (DE); REBSTOCK, Anne-Sophie, 69410 Champagne au Mont d'Or (FR); STELTE-LUDWIG, Beatrix, 42489 Wülfrath (DE); KIRCHHOFF, Dennis, 13086 Berlin (DE); BERNDT, Sandra, 16540 Hohen Neuendorf (DE); DIETZ, Lisa, 42111 Wuppertal (DE); MÄRSCH, Stephan, 50733 Köln (DE); HAMMER, Stefanie, 10247 Berlin (DE)
(74) Vertreter: HGF Europe LLP
(86) Internationale Anmeldenummer: PCT/EP2017/083313
(87) Internationale Veröffentlichungsnummer: WO 2018/114804

(56) Entgegenhaltungen:
- WO-A1-2015/096982
- WO-A1-2016/028573
- WO-A1-2016/201065
- WO-A1-2016/207094
- WO-A1-2016/207098
- WO-A1-2016/207104
- WO-A1-2017/162663
- WO-A1-2017/216028
- WO-A2-2014/160160
- LI BIN ET AL: "Design, synthesis and evaluation of anti-CD123 antibody drug conjugates", BIOORGANIC & MEDICINAL CHEMISTRY, PERGAMON, GB, Bd. 24, Nr. 22, 17. September 2016 (2016-09-17), Seiten 5855-5860, XP029775084, ISSN: 0968-0896, DOI: 10.1016/J.BMC.2016.09.043

### SEQUENCE LISTING

<110> Bayer Pharma Aktiengesellschaft
<120> Specific Antibody-Drug-Conjugates (ADCs) with KSP inhibitors
<130> BHC 16 1 068
<160> 62
<170> PatentIn version 3.5
<210> 1
   <211> 120
   <212> PRT
   <213> Artificial Sequence
<220>
   <223> Antibody Sequence
<400> 1
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Asp Tyr
               20                  25                  30
   Tyr Met Lys Trp Val Arg Gln Ala Pro Gly Gln Gly Leu Glu Trp Met
           35                  40                  45
   Gly Asp Ile Ile Pro Ser Asn Gly Ala Thr Phe Tyr Asn Gln Lys Phe
       50                  55                  60
   Lys Gly Gln Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser His Leu Leu Arg Ala Ser Trp Phe Ala Tyr Trp Gly Gln
               100                 105                 110
   Gly Thr Leu Val Thr Val Ser Ser
           115                 120
<210> 2
   <211> 5
   <212> PRT
   <213> Artificial Sequence
<220>
   <223> Antibody Sequence
<400> 2
   Asp Tyr Tyr Met Lys
   1               5
<210> 3
   <211> 17
   <212> **PRT**
   <213> **Artificial** Sequence
<220>
   <223> Antibody Sequence
<400> 3
   Asp Ile Ile Pro Ser Asn Gly Ala Thr Phe Tyr Asn Gln Lys Phe Lys
   1               5                   10                  15
   Gly
<210> 4
   <211> 11
   <212> **PRT**
   <213> **Artificial** Sequence
<220>
   <223> Antibody Sequence
<400> 4
   Ser His Leu Leu Arg Ala Ser Trp Phe Ala Tyr
   1               5                   10
<210> 5
   <211> 113
   <212> **PRT**
   <213> **Artificial** Sequence
<220>
   <223> Antibody Sequence
<400> 5
   Asp Ile Val Met Thr Gln Ser Pro Asp Ser Leu Ala Val Ser Leu Gly
   1               5                   10                  15
   Glu Arg Ala Thr Ile Asn Cys Glu Ser Ser Gln Ser Val Leu Asn Ser
               20                  25                  30
   Gly Asn Gln Lys Asn Tyr Leu Thr Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Asn
                   85                  90                  95
   Asp Tyr Ser Tyr Pro Tyr Thr Phe Gly Gln Gly Thr Lys Leu Glu Ile
               100                 105                 110
   Lys
<210> 6
   <211> 17
   <212> **PRT**
   <213> **Artificial** Sequence
<220>
   <223> Antibody Sequence
<400> 6
   Glu Ser Ser Gln Ser Val Leu Asn Ser Gly Asn Gln Lys Asn Tyr Leu
   1 5 10 15
   Thr
<210> 7
   <211> 7
   <212> PRT
   <213> Artificial Sequence
<220>
   <223> Antibody Sequence
<400> 7
   Trp Ala Ser Thr Arg Glu Ser
   1 5
<210> 8
   <211> 9
   <212> **PRT**
   <213> **Artificial** Sequence
<220>
   <223> Antibody Sequence
<400> 8
   Gln Asn Asp Tyr Ser Tyr Pro Tyr Thr
   1 5
<210> 9
   <211> **449**
   <212> **PRT**
   <213> **Artificial** Sequence
<220>
   <223> Antibody Sequence
<400> 9
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Asp Tyr
               20                  25                  30
   Tyr Met Lys Trp Val Arg Gln Ala Pro Gly Gln Gly Leu Glu Trp Met
           35                  40                  45
   Gly Asp Ile Ile Pro Ser Asn Gly Ala Thr Phe Tyr Asn Gln Lys Phe
       50                  55                  60
   Lys Gly Gln Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser His Leu Leu Arg Ala Ser Trp Phe Ala Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225 230 235 240
   Pro Ser Val Phe Leu Phe Pro Pro Lys Pro Lys Asp Thr Leu Met Ile
   245 250 255
   Ser Arg Thr Pro Glu Val Thr Cys Val Val Val Asp Val Ser His Glu
   260 265 270
   Asp Pro Glu Val Lys Phe Asn Trp Tyr Val Asp Gly Val Glu Val His
   275 280 285
   Asn Ala Lys Thr Lys Pro Arg Glu Glu Gln Tyr Asn Ser Thr Tyr Arg
   290 295 300
   Val Val Ser Val Leu Thr Val Leu His Gln Asp Trp Leu Asn Gly Lys
   305 310 315 320
   Glu Tyr Lys Cys Lys Val Ser Asn Lys Ala Leu Pro Ala Pro Ile Glu
   325 330 335
   Lys Thr Ile Ser Lys Ala Lys Gly Gln Pro Arg Glu Pro Gln Val Tyr
   340 345 350
   Thr Leu Pro Pro Ser Arg Asp Glu Leu Thr Lys Asn Gln Val Ser Leu
   355 360 365
   Thr Cys Leu Val Lys Gly Phe Tyr Pro Ser Asp Ile Ala Val Glu Trp
   370 375 380
   Glu Ser Asn Gly Gln Pro Glu Asn Asn Tyr Lys Thr Thr Pro Pro Val
   385 390 395 400
   Leu Asp Ser Asp Gly Ser Phe Phe Leu Tyr Ser Lys Leu Thr Val Asp
   405 410 415
   Lys Ser Arg Trp Gln Gln Gly Asn Val Phe Ser Cys Ser Val Met His
   420 425 430
   Glu Ala Leu His Asn His Tyr Thr Gln Lys Ser Leu Ser Leu Ser Pro
   435 440 445
   Gly
<210> 10
   <211> 220
   <212> PRT
   <213> Artificial Sequence
<220>
   <223> Antibody Sequence
<400> 10
   Asp Ile Val Met Thr Gln Ser Pro Asp Ser Leu Ala Val Ser Leu Gly
   1               5                   10                  15
   Glu Arg Ala Thr Ile Asn Cys Glu Ser Ser Gln Ser Val Leu Asn Ser
               20                  25                  30
   Gly Asn Gln Lys Asn Tyr Leu Thr Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Asn
                   85                  90                  95
   Asp Tyr Ser Tyr Pro Tyr Thr Phe Gly Gln Gly Thr Lys Leu Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 11
   <211> 118
   <212> PRT
   <213> Artificial Sequence
<220>
   <223> Antibody Sequence
<400> 11
   Glu Val Gln Leu Leu Glu Ser Gly Gly Gly Leu Val Gln Pro Gly Gly
   1               5                   10                  15
   Ser Leu Arg Leu Ser Cys Ala Val Ser Asp Tyr Ser Ile Thr Ser Gly
               20                  25                  30
   Tyr Tyr Trp Asn Trp Ile Arg Gln Ala Pro Gly Lys Lys Leu Glu Trp
           35                  40                  45
   Met Gly Tyr Ile Ser Tyr Asp Gly Ser Asn Asn Tyr Asn Pro Ser Leu
       50                  55                  60
   Lys Asn Gly Arg Ile Thr Ile Ser Arg Asp Thr Ser Lys Asn Thr Phe
   65                  70                  75                  80
   Tyr Leu Gln Met Asn Ser Leu Arg Ala Glu Asp Thr Ala Val Tyr Tyr
                   85                  90                  95
   Cys Ala Arg Gly Glu Gly Phe Tyr Phe Asp Ser Trp Gly Gln Gly Thr
               100                 105                 110
   Leu Val Thr Val Ser Ser
           115
<210> 12
   <211> 6
   <212> **PRT**
   <213> **Artificial** Sequence
<220>
   <223> Antibody Sequence
<400> 12
   Ser Gly Tyr Tyr Trp Asn
   1               5
<210> 13
   <211> 17
   <212> PRT
   <213> Artificial Sequence
<220>
   <223> Antibody Sequence
<400> 13
   Tyr Ile Ser Tyr Asp Gly Ser Asn Asn Tyr Asn Pro Ser Leu Lys Asn
   1               5                   10                  15
   Gly
<210> 14
   <211> 8
   <212> **PRT**
   <213> **Artificial** Sequence
<220>
   <223> Antibody Sequence
<400> 14
   Gly Glu Gly Phe Tyr Phe Asp Ser
   1               5
<210> 15
   <211> 113
   <212> **PRT**
   <213> **Artificial** Sequence
<220>
   <223> Antibody Sequence
<400> 15
   Glu Ile Val Leu Thr Gln Ser Pro Gly Thr Leu Ser Leu Ser Pro Gly
   1               5                   10                  15
   Glu Arg Ala Thr Leu Ser Cys Lys Ser Ser Gln Ser Leu Phe Phe Gly
               20                  25                  30
   Ser Thr Gln Lys Asn Tyr Leu Ala Trp Tyr Gln Gln Lys Pro Gly Gln
           35                  40                  45
   Ala Pro Arg Leu Leu Ile Tyr Trp Ala Ser Thr Arg Glu Ser Gly Ile
       50                  55                  60
   Pro Asp Arg Phe Ser Gly Ser Gly Ser Gly Thr Asp Phe Thr Leu Thr
   65                  70                  75                  80
   Ile Ser Arg Leu Glu Pro Glu Asp Phe Ala Val Tyr Tyr Cys Gln Gln
                   85                  90                  95
   Tyr Tyr Asn Tyr Pro Trp Thr Phe Gly Gln Gly Thr Lys Leu Glu Ile
               100                 105                 110
   Lys
<210> 16
   <211> 17
   <212> PRT
   <213> Artificial Sequence
<220>
   <223> Antibody Sequence
<400> 16
   Lys Ser Ser Gln Ser Leu Phe Phe Gly Ser Thr Gln Lys Asn Tyr Leu
   1               5                   10                  15
   Ala
<210> 17
   <211> 7
   <212> PRT
   <213> Artificial Sequence
<220>
   <223> Antibody Sequence
<400> 17
   Trp Ala Ser Thr Arg Glu Ser
   1               5
<210> 18
   <211> 9
   <212> PRT
   <213> Artificial Sequence
<220>
   <223> Antibody Sequence
<400> 18
   Gln Gln Tyr Tyr Asn Tyr Pro Trp Thr
   1 5
<210> 19
   <211> 447
   <212> PRT
   <213> Artificial Sequence
<220>
   <223> Antibody Sequence
<400> 19
   Glu Val Gln Leu Leu Glu Ser Gly Gly Gly Leu Val Gln Pro Gly Gly
   1               5                   10                  15
   Ser Leu Arg Leu Ser Cys Ala Val Ser Asp Tyr Ser Ile Thr Ser Gly
               20                  25                  30
   Tyr Tyr Trp Asn Trp Ile Arg Gln Ala Pro Gly Lys Lys Leu Glu Trp
           35                  40                  45
   Met Gly Tyr Ile Ser Tyr Asp Gly Ser Asn Asn Tyr Asn Pro Ser Leu
       50                  55                  60
   Lys Asn Gly Arg Ile Thr Ile Ser Arg Asp Thr Ser Lys Asn Thr Phe
   65                  70                  75                  80
   Tyr Leu Gln Met Asn Ser Leu Arg Ala Glu Asp Thr Ala Val Tyr Tyr
                   85                  90                  95
   Cys Ala Arg Gly Glu Gly Phe Tyr Phe Asp Ser Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
   260 265 270
   Glu Val Lys Phe Asn Trp Tyr Val Asp Gly Val Glu Val His Asn Ala
   275 280 285
   Lys Thr Lys Pro Arg Glu Glu Gln Tyr Asn Ser Thr Tyr Arg Val Val
   290 295 300
   Ser Val Leu Thr Val Leu His Gln Asp Trp Leu Asn Gly Lys Glu Tyr
   305 310 315 320
   Lys Cys Lys Val Ser Asn Lys Ala Leu Pro Ala Pro Ile Glu Lys Thr
   325 330 335
   Ile Ser Lys Ala Lys Gly Gln Pro Arg Glu Pro Gln Val Tyr Thr Leu
   340 345 350
   Pro Pro Ser Arg Asp Glu Leu Thr Lys Asn Gln Val Ser Leu Thr Cys
   355 360 365
   Leu Val Lys Gly Phe Tyr Pro Ser Asp Ile Ala Val Glu Trp Glu Ser
   370 375 380
   Asn Gly Gln Pro Glu Asn Asn Tyr Lys Thr Thr Pro Pro Val Leu Asp
   385 390 395 400
   Ser Asp Gly Ser Phe Phe Leu Tyr Ser Lys Leu Thr Val Asp Lys Ser
   405 410 415
   Arg Trp Gln Gln Gly Asn Val Phe Ser Cys Ser Val Met His Glu Ala
   420 425 430
   Leu His Asn His Tyr Thr Gln Lys Ser Leu Ser Leu Ser Pro Gly
   435 440 445
<210> 20
   <211> 220
   <212> PRT
   <213> Artificial Sequence
<220>
   <223> Antibody Sequence
<400> 20
   Glu Ile Val Leu Thr Gln Ser Pro Gly Thr Leu Ser Leu Ser Pro Gly
   1               5                   10                  15
   Glu Arg Ala Thr Leu Ser Cys Lys Ser Ser Gln Ser Leu Phe Phe Gly
               20                  25                  30
   Ser Thr Gln Lys Asn Tyr Leu Ala Trp Tyr Gln Gln Lys Pro Gly Gln
           35                  40                  45
   Ala Pro Arg Leu Leu Ile Tyr Trp Ala Ser Thr Arg Glu Ser Gly Ile
       50                  55                  60
   Pro Asp Arg Phe Ser Gly Ser Gly Ser Gly Thr Asp Phe Thr Leu Thr
   65                  70                  75                  80
   Ile Ser Arg Leu Glu Pro Glu Asp Phe Ala Val Tyr Tyr Cys Gln Gln
                   85                  90                  95
   Tyr Tyr Asn Tyr Pro Trp Thr Phe Gly Gln Gly Thr Lys Leu Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 21
   <211> 113
   <212> PRT
   <213> Artificial Sequence
<220>
   <223> Antibody Sequence
<400> 21
   Glu Val Gln Leu Val Glu Ser Gly Gly Gly Leu Val Gln Pro Gly Lys
   1               5                   10                  15
   Ser Leu Lys Leu Ser Cys Ser Ala Ser Gly Phe Thr Phe Ser Thr Ser
               20                  25                  30
   Gly Met His Trp Phe Arg Gln Ala Pro Gly Lys Gly Leu Asp Trp Val
           35                  40                  45
   Ala Tyr Ile Ser Ser Ser Ser Gly Phe Val Tyr Ala Asp Ala Val Lys
       50                  55                  60
   Gly Arg Phe Thr Ile Ser Arg Asp Asn Ala Gln Asn Thr Leu Tyr Leu
   65                  70                  75                  80
   Gln Leu Asn Ser Leu Lys Ser Glu Asp Thr Ala Ile Tyr Tyr Cys Ala
                   85                  90                  95
   Arg Ser Glu Ala Ala Phe Trp Gly Gln Gly Thr Leu Val Thr Val Ser
               100                 105                 110
   Ser
<210> 22
   <211> 5
   <212> PRT
   <213> Artificial Sequence
<220>
   <223> Antibody Sequence
<400> 22
   Thr Ser Gly Met His
   1               5
<210> 23
   <211> 16
   <212> PRT
   <213> Artificial Sequence
<220>
   <223> Antibody Sequence
<400> 23
   Tyr Ile Ser Ser Ser Ser Gly Phe Val Tyr Ala Asp Ala Val Lys Gly
   1               5                   10                  15
<210> 24
   <211> 5
   <212> PRT
   <213> Artificial Sequence
<220>
   <223> Antibody Sequence
<400> 24
   Ser Glu Ala Ala Phe
   1               5
<210> 25
   <211> 112
   <212> PRT
   <213> Artificial Sequence
<220>
   <223> Antibody Sequence
<400> 25
   Asp Ile Val Leu Thr Gln Ala Pro Arg Ser Val Ser Val Thr Pro Gly
   1               5                   10                  15
   Glu Ser Ala Ser Ile Ser Cys Arg Ser Asn Lys Ser Arg Leu Ser Arg
               20                  25                  30
   Met Gly Ile Thr Pro Leu Asn Trp Tyr Leu Gln Lys Pro Gly Lys Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Glu Thr Asp Phe Thr Leu Lys Ile
   65                  70                  75                  80
   Ser Lys Val Glu Thr Glu Asp Val Gly Val Tyr Tyr Cys Ala Gln Phe
                   85                  90                  95
   Leu Glu Tyr Pro Pro Thr Phe Gly Ser Gly Thr Lys Leu Glu Ile Lys
               100                 105                 110
<210> 26
   <211> 16
   <212> PRT
   <213> Artificial Sequence
<220>
   <223> Antibody Sequence
<400> 26
   Arg Ser Asn Lys Ser Arg Leu Ser Arg Met Gly Ile Thr Pro Leu Asn
   1 5 10 15
<210> 27
   <211> 7
   <212> PRT
   <213> Artificial Sequence
<220>
   <223> Antibody Sequence
<400> 27
   Arg Met Ser Asn Leu Ala Ser
   1 5
<210> 28
   <211> 9
   <212> PRT
   <213> Artificial Sequence
<220>
   <223> Antibody Sequence
<400> 28
   Ala Gln Phe Leu Glu Tyr Pro Pro Thr
   1 5
<210> 29
   <211> 442
   <212> PRT
   <213> Artificial Sequence
<220>
   <223> Antibody Sequence
<400> 29
   Glu Val Gln Leu Val Glu Ser Gly Gly Gly Leu Val Gln Pro Gly Lys
   1               5                   10                  15
   Ser Leu Lys Leu Ser Cys Ser Ala Ser Gly Phe Thr Phe Ser Thr Ser
               20                  25                  30
   Gly Met His Trp Phe Arg Gln Ala Pro Gly Lys Gly Leu Asp Trp Val
           35                  40                  45
   Ala Tyr Ile Ser Ser Ser Ser Gly Phe Val Tyr Ala Asp Ala Val Lys
       50                  55                  60
   Gly Arg Phe Thr Ile Ser Arg Asp Asn Ala Gln Asn Thr Leu Tyr Leu
   65                  70                  75                  80
   Gln Leu Asn Ser Leu Lys Ser Glu Asp Thr Ala Ile Tyr Tyr Cys Ala
                   85                  90                  95
   Arg Ser Glu Ala Ala Phe Trp Gly Gln Gly Thr Leu Val Thr Val Ser
               100                 105                 110
   Ser Ala Ser Thr Lys Gly Pro Ser Val Phe Pro Leu Ala Pro Ser Ser
           115                 120                 125
   Lys Ser Thr Ser Gly Gly Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Gln
               180                 185                 190
   Thr Tyr Ile Cys Asn Val Asn His Lys Pro Ser Asn Thr Lys Val Asp
           195                 200                 205
   Lys Lys Val Glu Pro Lys Ser Cys Asp Lys Thr His Thr Cys Pro Pro
       210                 215                 220
   Cys Pro Ala Pro Glu Leu Leu Gly Gly Pro Ser Val Phe Leu Phe Pro
   225                 230                 235                 240
   Pro Lys Pro Lys Asp Thr Leu Met Ile Ser Arg Thr Pro Glu Val Thr
                   245                 250                 255
   Cys Val Val Val Asp Val Ser His Glu Asp Pro Glu Val Lys Phe Asn
               260                 265                 270
   Trp Tyr Val Asp Gly Val Glu Val His Asn Ala Lys Thr Lys Pro Arg
           275                 280                 285
   Glu Glu Gln Tyr Asn Ser Thr Tyr Arg Val Val Ser Val Leu Thr Val
       290                 295                 300
   Leu His Gln Asp Trp Leu Asn Gly Lys Glu Tyr Lys Cys Lys Val Ser
   305                 310                 315                 320
   Asn Lys Ala Leu Pro Ala Pro Ile Glu Lys Thr Ile Ser Lys Ala Lys
                   325                 330                 335
   Gly Gln Pro Arg Glu Pro Gln Val Tyr Thr Leu Pro Pro Ser Arg Asp
               340                 345                 350
   Glu Leu Thr Lys Asn Gln Val Ser Leu Thr Cys Leu Val Lys Gly Phe
           355                 360                 365
   Tyr Pro Ser Asp Ile Ala Val Glu Trp Glu Ser Asn Gly Gln Pro Glu
       370             375             380
   Asn Asn Tyr Lys Thr Thr Pro Pro Val Leu Asp Ser Asp Gly Ser Phe
   385             390             395             400
   Phe Leu Tyr Ser Lys Leu Thr Val Asp Lys Ser Arg Trp Gln Gln Gly
                   405             410             415
   Asn Val Phe Ser Cys Ser Val Met His Glu Ala Leu His Asn His Tyr
               420             425             430
   Thr Gln Lys Ser Leu Ser Leu Ser Pro Gly
           435             440
<210> 30
   <211> 219
   <212> PRT
   <213> Artificial Sequence
<220>
   <223> Antibody Sequence
<400> 30
   Asp Ile Val Leu Thr Gln Ala Pro Arg Ser Val Ser Val Thr Pro Gly
   1               5                   10                  15
   Glu Ser Ala Ser Ile Ser Cys Arg Ser Asn Lys Ser Arg Leu Ser Arg
               20                  25                  30
   Met Gly Ile Thr Pro Leu Asn Trp Tyr Leu Gln Lys Pro Gly Lys Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Glu Thr Asp Phe Thr Leu Lys Ile
   65                  70                  75                  80
   Ser Lys Val Glu Thr Glu Asp Val Gly Val Tyr Tyr Cys Ala Gln Phe
                   85                  90                  95
   Leu Glu Tyr Pro Pro Thr Phe Gly Ser Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
   130 135 140
   Tyr Pro Arg Glu Ala Lys Val Gln Trp Lys Val Asp Asn Ala Leu Gln
   145 150 155 160
   Ser Gly Asn Ser Gln Glu Ser Val Thr Glu Gln Asp Ser Lys Asp Ser
   165 170 175
   Thr Tyr Ser Leu Ser Ser Thr Leu Thr Leu Ser Lys Ala Asp Tyr Glu
   180 185 190
   Lys His Lys Val Tyr Ala Cys Glu Val Thr His Gln Gly Leu Ser Ser
   195 200 205
   Pro Val Thr Lys Ser Phe Asn Arg Gly Glu Cys
   210 215
<210> 31
   <211> 120
   <212> PRT
   <213> Artificial Sequence
<220>
   <223> Antibody Sequence
<400> 31
   Gln Val Gln Leu Val Gln Ser Gly Ala Glu Val Lys Lys Pro Gly Ser
   1 5 10 15
   Ser Val Lys Val Ser Cys Lys Ala Ser Gly Gly Thr Phe Ser Asp Tyr
   20 25 30
   Tyr Met Lys Trp Val Arg Gln Ala Pro Gly Gln Gly Leu Glu Trp Met
   35 40 45
   Gly Asp Ile Ile Pro Ser Asn Gly Ala Thr Phe Tyr Asn Gln Lys Phe
   50 55 60
   Lys Gly Arg Val Thr Ile Thr Ala Asp Glu Ser Thr Ser Thr Ala Tyr
   65 70 75 80
   Met Glu Leu Ser Ser Leu Arg Ser Glu Asp Thr Ala Val Tyr Tyr Cys
   85 90 95
   Ala Arg Ser His Leu Leu Arg Ala Ser Trp Phe Ala Tyr Trp Gly Gln
   100 105 110
   Gly Thr Leu Val Thr Val Ser Ser
   115 120
<210> 32
   <211> 5
   <212> PRT
   <213> Artificial Sequence
<220>
   <223> Antibody Sequence
<400> 32
   Asp Tyr Tyr Met Lys
   1 5
<210> 33
   <211> 17
   <212> PRT
   <213> Artificial Sequence
<220>
   <223> Antibody Sequence
<400> 33
   Asp Ile Ile Pro Ser Asn Gly Ala Thr Phe Tyr Asn Gln Lys Phe Lys
   1 5 10 15
   Gly
<210> 34
   <211> 11
   <212> PRT
   <213> Artificial Sequence
<220>
   <223> Antibody Sequence
<400> 34
   Ser His Leu Leu Arg Ala Ser Trp Phe Ala Tyr
   1 5 10
<210> 35
   <211> 113
   <212> PRT
   <213> Artificial Sequence
<220>
   <223> Antibody Sequence
<400> 35
   Asp Ile Val Met Thr Gln Ser Pro Asp Ser Leu Ala Val Ser Leu Gly
   1 5 10 15
   Glu Arg Ala Thr Ile Asn Cys Glu Ser Ser Gln Ser Val Leu Asn Ser
   20 25 30
   Gly Asn Gln Lys Asn Tyr Leu Thr Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Asn
   85 90 95
   Asp Tyr Ser Tyr Pro Tyr Thr Phe Gly Gln Gly Thr Lys Leu Glu Ile
   100 105 110
   Lys
<210> 36
   <211> 17
   <212> PRT
   <213> Artificial Sequence
<220>
   <223> Antibody Sequence
<400> 36
   Glu Ser Ser Gln Ser Val Leu Asn Ser Gly Asn Gln Lys Asn Tyr Leu
   1 5 10 15
   Thr
<210> 37
   <211> 7
   <212> PRT
   <213> Artificial Sequence
<220>
   <223> Antibody Sequence
<400> 37
   Trp Ala Ser Thr Arg Glu Ser
   1 5
<210> 38
   <211> 9
   <212> PRT
   <213> Artificial Sequence
<220>
   <223> Antibody Sequence
<400> 38
   Gln Asn Asp Tyr Ser Tyr Pro Tyr Thr
   1               5
<210> 39
   <211> 449
   <212> PRT
   <213> Artificial Sequence
<220>
   <223> Antibody Sequence
<400> 39
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Ser Asp Tyr
               20                  25                  30
   Tyr Met Lys Trp Val Arg Gln Ala Pro Gly Gln Gly Leu Glu Trp Met
           35                  40                  45
   Gly Asp Ile Ile Pro Ser Asn Gly Ala Thr Phe Tyr Asn Gln Lys Phe
       50                  55                  60
   Lys Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser His Leu Leu Arg Ala Ser Trp Phe Ala Tyr Trp Gly Gln
               100                 105                 110
   Gly Thr Leu Val Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly
<210> 40
   <211> 220
   <212> PRT
   <213> Artificial Sequence
<220>
   <223> Antibody Sequence
<400> 40
   Asp Ile Val Met Thr Gln Ser Pro Asp Ser Leu Ala Val Ser Leu Gly
   1               5                   10                  15
   Glu Arg Ala Thr Ile Asn Cys Glu Ser Ser Gln Ser Val Leu Asn Ser
               20                  25                  30
   Gly Asn Gln Lys Asn Tyr Leu Thr Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Asn
                   85                  90                  95
   Asp Tyr Ser Tyr Pro Tyr Thr Phe Gly Gln Gly Thr Lys Leu Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 41
   <211> 113
   <212> PRT
   <213> Artificial Sequence
<220>
   <223> Antibody Sequence
<400> 41
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Phe Ser Thr Ser
               20                  25                  30
   Gly Met His Trp Phe Arg Gln Ala Pro Gly Lys Gly Leu Glu Trp Val
           35                  40                  45
   Ala Tyr Ile Ser Ser Ser Ser Gly Phe Val Tyr Ala Asp Ala Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Ser Glu Ala Ala Phe Trp Gly Gln Gly Thr Leu Val Thr Val Ser
               100                 105                 110
   Ser
<210> 42
   <211> 5
   <212> PRT
   <213> Artificial Sequence
<220>
   <223> Antibody Sequence
<400> 42
   Thr Ser Gly Met His
   1               5
<210> 43
   <211> 16
   <212> **PRT**
   <213> **Artificial** Sequence
<220>
   <223> Antibody Sequence
<400> 43
   Tyr Ile Ser Ser Ser Ser Gly Phe Val Tyr Ala Asp Ala Val Lys Gly
   1 5 10 15
<210> 44
   <211> 5
   <212> PRT
   <213> Artificial Sequence
<220>
   <223> Antibody Sequence
<400> 44
   Ser Glu Ala Ala Phe
   1 5
<210> 45
   <211> 112
   <212> PRT
   <213> Artificial Sequence
<220>
   <223> Antibody Sequence
<400> 45
   Asp Ile Val Met Thr Gln Ser Pro Leu Ser Leu Pro Val Thr Pro Gly
   1 5 10 15
   Glu Pro Ala Ser Ile Ser Cys Arg Ser Gln Lys Ser Arg Leu Ser Arg
   20 25 30
   Met Gly Ile Thr Pro Leu Asn Trp Tyr Leu Gln Lys Pro Gly Gln Ser
   35 40 45
   Pro Gln Leu Leu Ile Tyr Arg Met Ser Asn Leu Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Ala Gln Phe
   85 90 95
   Leu Glu Tyr Pro Pro Thr Phe Gly Gln Gly Thr Lys Leu Glu Ile Lys
   100 105 110
<210> 46
   <211> 16
   <212> PRT
   <213> Artificial Sequence
<220>
   <223> Antibody Sequence
<400> 46
   Arg Ser Gln Lys Ser Arg Leu Ser Arg Met Gly Ile Thr Pro Leu Asn
   1 5 10 15
<210> 47
   <211> 7
   <212> PRT
   <213> Artificial Sequence
<220>
   <223> Antibody Sequence
<400> 47
   Arg Met Ser Asn Leu Ala Ser
   1 5
<210> 48
   <211> 9
   <212> PRT
   <213> Artificial Sequence
<220>
   <223> Antibody Sequence
<400> 48
   Ala Gln Phe Leu Glu Tyr Pro Pro Thr
   1 5
<210> 49
   <211> 442
   <212> PRT
   <213> Artificial Sequence
<220>
   <223> Antibody Sequence
<400> 49
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Phe Ser Thr Ser
               20                  25                  30
   Gly Met His Trp Phe Arg Gln Ala Pro Gly Lys Gly Leu Glu Trp Val
           35                  40                  45
   Ala Tyr Ile Ser Ser Ser Ser Gly Phe Val Tyr Ala Asp Ala Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Ser Glu Ala Ala Phe Trp Gly Gln Gly Thr Leu Val Thr Val Ser
               100                 105                 110
   Ser Ala Ser Thr Lys Gly Pro Ser Val Phe Pro Leu Ala Pro Ser Ser
           115                 120                 125
   Lys Ser Thr Ser Gly Gly Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Gln
               180                 185                 190
   Thr Tyr Ile Cys Asn Val Asn His Lys Pro Ser Asn Thr Lys Val Asp
           195                 200                 205
   Lys Lys Val Glu Pro Lys Ser Cys Asp Lys Thr His Thr Cys Pro Pro
       210                 215                 220
   Cys Pro Ala Pro Glu Leu Leu Gly Gly Pro Ser Val Phe Leu Phe Pro
   225                 230                 235                 240
   Pro Lys Pro Lys Asp Thr Leu Met Ile Ser Arg Thr Pro Glu Val Thr
                   245                 250                 255
   Cys Val Val Val Asp Val Ser His Glu Asp Pro Glu Val Lys Phe Asn
   260 265 270
   Trp Tyr Val Asp Gly Val Glu Val His Asn Ala Lys Thr Lys Pro Arg
   275 280 285
   Glu Glu Gln Tyr Asn Ser Thr Tyr Arg Val Val Ser Val Leu Thr Val
   290 295 300
   Leu His Gln Asp Trp Leu Asn Gly Lys Glu Tyr Lys Cys Lys Val Ser
   305 310 315 320
   Asn Lys Ala Leu Pro Ala Pro Ile Glu Lys Thr Ile Ser Lys Ala Lys
   325 330 335
   Gly Gln Pro Arg Glu Pro Gln Val Tyr Thr Leu Pro Pro Ser Arg Asp
   340 345 350
   Glu Leu Thr Lys Asn Gln Val Ser Leu Thr Cys Leu Val Lys Gly Phe
   355 360 365
   Tyr Pro Ser Asp Ile Ala Val Glu Trp Glu Ser Asn Gly Gln Pro Glu
   370 375 380
   Asn Asn Tyr Lys Thr Thr Pro Pro Val Leu Asp Ser Asp Gly Ser Phe
   385 390 395 400
   Phe Leu Tyr Ser Lys Leu Thr Val Asp Lys Ser Arg Trp Gln Gln Gly
   405 410 415
   Asn Val Phe Ser Cys Ser Val Met His Glu Ala Leu His Asn His Tyr
   420 425 430
   Thr Gln Lys Ser Leu Ser Leu Ser Pro Gly
   435 440
<210> 50
   <211> 219
   <212> PRT
   <213> Artificial Sequence
<220>
   <223> Antibody Sequence
<400> 50
   Asp Ile Val Met Thr Gln Ser Pro Leu Ser Leu Pro Val Thr Pro Gly
   1 5 10 15
   Glu Pro Ala Ser Ile Ser Cys Arg Ser Gln Lys Ser Arg Leu Ser Arg
               20                  25                  30
   Met Gly Ile Thr Pro Leu Asn Trp Tyr Leu Gln Lys Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Phe
                   85                  90                  95
   Leu Glu Tyr Pro Pro Thr Phe Gly Gln Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 51
   <211> 113
   <212> PRT
   <213> Artificial Sequence
<220>
   <223> Antibody Sequence
<400> 51
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Phe Ser Thr Ser
               20                  25                  30
   Gly Met His Trp Val Arg Gln Ala Pro Gly Lys Gly Leu Glu Trp Val
           35                  40                  45
   Ser Tyr Ile Ser Ser Ser Ser Gly Phe Val Tyr Ala Asp Ala Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Ser Glu Ala Ala Phe Trp Gly Gln Gly Thr Leu Val Thr Val Ser
               100                 105                 110
   Ser
<210> 52
   <211> 5
   <212> PRT
   <213> Artificial Sequence
<220>
   <223> Antibody Sequence
<400> 52
   Thr Ser Gly Met His
   1               5
<210> 53
   <211> 16
   <212> PRT
   <213> Artificial Sequence
<220>
   <223> Antibody Sequence
<400> 53
   Tyr Ile Ser Ser Ser Ser Gly Phe Val Tyr Ala Asp Ala Val Lys Gly
   1               5                   10                  15
<210> 54
   <211> 5
   <212> PRT
   <213> Artificial Sequence
<220>
   <223> Antibody Sequence
<400> 54
   Ser Glu Ala Ala Phe
   1               5
<210> 55
   <211> 112
   <212> PRT
   <213> Artificial Sequence
<220>
   <223> Antibody Sequence
<400> 55
   Asp Ile Val Met Thr Gln Ser Pro Leu Ser Leu Pro Val Thr Pro Gly
   1               5                   10                  15
   Glu Pro Ala Ser Ile Ser Cys Arg Ser Gln Lys Ser Arg Leu Ser Arg
               20                  25                  30
   Met Gly Ile Thr Pro Leu Asn Trp Tyr Leu Gln Lys Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Phe
                   85                  90                  95
   Leu Glu Tyr Pro Pro Thr Phe Gly Gln Gly Thr Lys Leu Glu Ile Lys
               100                 105                 110
<210> 56
   <211> 16
   <212> **PRT**
   <213> **Artificial** Sequence
<220>
   <223> Antibody Sequence
<400> 56
   Arg Ser Gln Lys Ser Arg Leu Ser Arg Met Gly Ile Thr Pro Leu Asn
   1 5 10 15
<210> 57
   <211> 7
   <212> PRT
   <213> Artificial Sequence
<220>
   <223> Antibody Sequence
<400> 57
   Arg Met Ser Asn Leu Ala Ser
   1 5
<210> 58
   <211> 9
   <212> PRT
   <213> Artificial Sequence
<220>
   <223> Antibody Sequence
<400> 58
   Ala Gln Phe Leu Glu Tyr Pro Pro Thr
   1 5
<210> 59
   <211> 442
   <212> PRT
   <213> Artificial Sequence
<220>
   <223> Antibody Sequence
<400> 59
   Glu Val Gln Leu Val Glu Ser Gly Gly Gly Leu Ile Gln Pro Gly Gly
   1               5                   10                  15
   Ser Leu Arg Leu Ser Cys Ala Ala Ser Gly Phe Thr Phe Ser Thr Ser
               20                  25                  30
   Gly Met His Trp Val Arg Gln Ala Pro Gly Lys Gly Leu Glu Trp Val
           35                  40                  45
   Ser Tyr Ile Ser Ser Ser Ser Gly Phe Val Tyr Ala Asp Ala Val Lys
       50                  55                  60
   Gly Arg Phe Thr Ile Ser Arg Asp Asn Ser Lys Asn Thr Leu Tyr Leu
   65                  70                  75                  80
   Gln Met Asn Ser Leu Arg Ala Glu Asp Thr Ala Val Tyr Tyr Cys Ala
                   85                  90                  95
   Arg Ser Glu Ala Ala Phe Trp Gly Gln Gly Thr Leu Val Thr Val Ser
               100                 105                 110
   Ser Ala Ser Thr Lys Gly Pro Ser Val Phe Pro Leu Ala Pro Ser Ser
           115                 120                 125
   Lys Ser Thr Ser Gly Gly Thr Ala Ala Leu Gly Cys Leu Val Lys Asp
       130                 135                 140
   Tyr Phe Pro Glu Pro Val Thr Val Ser Trp Asn Ser Gly Ala Leu Thr
   145                 150                 155                 160
   Ser Gly Val His Thr Phe Pro Ala Val Leu Gln Ser Ser Gly Leu Tyr
                   165                 170                 175
   Ser Leu Ser Ser Val Val Thr Val Pro Ser Ser Ser Leu Gly Thr Gln
               180                 185                 190
   Thr Tyr Ile Cys Asn Val Asn His Lys Pro Ser Asn Thr Lys Val Asp
           195                 200                 205
   Lys Lys Val Glu Pro Lys Ser Cys Asp Lys Thr His Thr Cys Pro Pro
       210                 215                 220
   Cys Pro Ala Pro Glu Leu Leu Gly Gly Pro Ser Val Phe Leu Phe Pro
   225                 230                 235                 240
   Pro Lys Pro Lys Asp Thr Leu Met Ile Ser Arg Thr Pro Glu Val Thr
                   245                 250                 255
   Cys Val Val Val Asp Val Ser His Glu Asp Pro Glu Val Lys Phe Asn
               260                 265                 270
   Trp Tyr Val Asp Gly Val Glu Val His Asn Ala Lys Thr Lys Pro Arg
           275                 280                 285
   Glu Glu Gln Tyr Asn Ser Thr Tyr Arg Val Val Ser Val Leu Thr Val
       290                 295                 300
   Leu His Gln Asp Trp Leu Asn Gly Lys Glu Tyr Lys Cys Lys Val Ser
   305                 310                 315                 320
   Asn Lys Ala Leu Pro Ala Pro Ile Glu Lys Thr Ile Ser Lys Ala Lys
                   325                 330                 335
   Gly Gln Pro Arg Glu Pro Gln Val Tyr Thr Leu Pro Pro Ser Arg Asp
               340                 345                 350
   Glu Leu Thr Lys Asn Gln Val Ser Leu Thr Cys Leu Val Lys Gly Phe
   355 360 365
   Tyr Pro Ser Asp Ile Ala Val Glu Trp Glu Ser Asn Gly Gln Pro Glu
   370 375 380
   Asn Asn Tyr Lys Thr Thr Pro Pro Val Leu Asp Ser Asp Gly Ser Phe
   385 390 395 400
   Phe Leu Tyr Ser Lys Leu Thr Val Asp Lys Ser Arg Trp Gln Gln Gly
   405 410 415
   Asn Val Phe Ser Cys Ser Val Met His Glu Ala Leu His Asn His Tyr
   420 425 430
   Thr Gln Lys Ser Leu Ser Leu Ser Pro Gly
   435 440
<210> 60
   <211> 219
   <212> PRT
   <213> Artificial Sequence
<220>
   <223> Antibody Sequence
<400> 60
   Asp Ile Val Met Thr Gln Ser Pro Leu Ser Leu Pro Val Thr Pro Gly
   1               5                   10                  15
   Glu Pro Ala Ser Ile Ser Cys Arg Ser Gln Lys Ser Arg Leu Ser Arg
               20                  25                  30
   Met Gly Ile Thr Pro Leu Asn Trp Tyr Leu Gln Lys Pro Gly Gln Ser
           35                  40                  45
   Pro Gln Leu Leu Ile Tyr Arg Met Ser Asn Leu Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ala Gln Phe
                   85                  90                  95
   Leu Glu Tyr Pro Pro Thr Phe Gly Gln Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 61
   <211> 372
   <212> PRT
   <213> Homo sapiens
<400> 61
   Met Asn Tyr Pro Leu Thr Leu Glu Met Asp Leu Glu Asn Leu Glu Asp
   1               5                   10                  15
   Leu Phe Trp Glu Leu Asp Arg Leu Asp Asn Tyr Asn Asp Thr Ser Leu
               20                  25                  30
   Val Glu Asn His Leu Cys Pro Ala Thr Glu Gly Pro Leu Met Ala Ser
           35                  40                  45
   Phe Lys Ala Val Phe Val Pro Val Ala Tyr Ser Leu Ile Phe Leu Leu
       50                  55                  60
   Gly Val Ile Gly Asn Val Leu Val Leu Val Ile Leu Glu Arg His Arg
   65                  70                  75                  80
   Gln Thr Arg Ser Ser Thr Glu Thr Phe Leu Phe His Leu Ala Val Ala
                   85                  90                  95
   Asp Leu Leu Leu Val Phe Ile Leu Pro Phe Ala Val Ala Glu Gly Ser
               100                 105                 110
   Val Gly Trp Val Leu Gly Thr Phe Leu Cys Lys Thr Val Ile Ala Leu
           115                 120                 125
   His Lys Val Asn Phe Tyr Cys Ser Ser Leu Leu Leu Ala Cys Ile Ala
   130 135 140
   Val Asp Arg Tyr Leu Ala Ile Val His Ala Val His Ala Tyr Arg His
   145 150 155 160
   Arg Arg Leu Leu Ser Ile His Ile Thr Cys Gly Thr Ile Trp Leu Val
   165 170 175
   Gly Phe Leu Leu Ala Leu Pro Glu Ile Leu Phe Ala Lys Val Ser Gln
   180 185 190
   Gly His His Asn Asn Ser Leu Pro Arg Cys Thr Phe Ser Gln Glu Asn
   195 200 205
   Gln Ala Glu Thr His Ala Trp Phe Thr Ser Arg Phe Leu Tyr His Val
   210 215 220
   Ala Gly Phe Leu Leu Pro Met Leu Val Met Gly Trp Cys Tyr Val Gly
   225 230 235 240
   Val Val His Arg Leu Arg Gln Ala Gln Arg Arg Pro Gln Arg Gln Lys
   245 250 255
   Ala Val Arg Val Ala Ile Leu Val Thr Ser Ile Phe Phe Leu Cys Trp
   260 265 270
   Ser Pro Tyr His Ile Val Ile Phe Leu Asp Thr Leu Ala Arg Leu Lys
   275 280 285
   Ala Val Asp Asn Thr Cys Lys Leu Asn Gly Ser Leu Pro Val Ala Ile
   290 295 300
   Thr Met Cys Glu Phe Leu Gly Leu Ala His Cys Cys Leu Asn Pro Met
   305 310 315 320
   Leu Tyr Thr Phe Ala Gly Val Lys Phe Arg Ser Asp Leu Ser Arg Leu
   325 330 335
   Leu Thr Lys Leu Gly Cys Thr Gly Pro Ala Ser Leu Cys Gln Leu Phe
   340 345 350
   Pro Ser Trp Arg Arg Ser Ser Leu Ser Glu Ser Glu Asn Ala Thr Ser
   355 360 365
   Leu Thr Thr Phe
   370
<210> 62
   <211> 378
   <212> PRT
   <213> Homo sapiens
<400> 62
   Met Val Leu Leu Trp Leu Thr Leu Leu Leu Ile Ala Leu Pro Cys Leu
   1               5                   10                  15
   Leu Gln Thr Lys Glu Asp Pro Asn Pro Pro Ile Thr Asn Leu Arg Met
               20                  25                  30
   Lys Ala Lys Ala Gln Gln Leu Thr Trp Asp Leu Asn Arg Asn Val Thr
           35                  40                  45
   Asp Ile Glu Cys Val Lys Asp Ala Asp Tyr Ser Met Pro Ala Val Asn
       50                  55                  60
   Asn Ser Tyr Cys Gln Phe Gly Ala Ile Ser Leu Cys Glu Val Thr Asn
   65                  70                  75                  80
   Tyr Thr Val Arg Val Ala Asn Pro Pro Phe Ser Thr Trp Ile Leu Phe
                   85                  90                  95
   Pro Glu Asn Ser Gly Lys Pro Trp Ala Gly Ala Glu Asn Leu Thr Cys
               100                 105                 110
   Trp Ile His Asp Val Asp Phe Leu Ser Cys Ser Trp Ala Val Gly Pro
           115                 120                 125
   Gly Ala Pro Ala Asp Val Gln Tyr Asp Leu Tyr Leu Asn Val Ala Asn
       130                 135                 140
   Arg Arg Gln Gln Tyr Glu Cys Leu His Tyr Lys Thr Asp Ala Gln Gly
   145                 150                 155                 160
   Thr Arg Ile Gly Cys Arg Phe Asp Asp Ile Ser Arg Leu Ser Ser Gly
                   165                 170                 175
   Ser Gln Ser Ser His Ile Leu Val Arg Gly Arg Ser Ala Ala Phe Gly
               180                 185                 190
   Ile Pro Cys Thr Asp Lys Phe Val Val Phe Ser Gln Ile Glu Ile Leu
           195                 200                 205
   Thr Pro Pro Asn Met Thr Ala Lys Cys Asn Lys Thr His Ser Phe Met
   210 215 220
   His Trp Lys Met Arg Ser His Phe Asn Arg Lys Phe Arg Tyr Glu Leu
   225 230 235 240
   Gln Ile Gln Lys Arg Met Gln Pro Val Ile Thr Glu Gln Val Arg Asp
   245 250 255
   Arg Thr Ser Phe Gln Leu Leu Asn Pro Gly Thr Tyr Thr Val Gln Ile
   260 265 270
   Arg Ala Arg Glu Arg Val Tyr Glu Phe Leu Ser Ala Trp Ser Thr Pro
   275 280 285
   Gln Arg Phe Glu Cys Asp Gln Glu Glu Gly Ala Asn Thr Arg Ala Trp
   290 295 300
   Arg Thr Ser Leu Leu Ile Ala Leu Gly Thr Leu Leu Ala Leu Val Cys
   305 310 315 320
   Val Phe Val Ile Cys Arg Arg Tyr Leu Val Met Gln Arg Leu Phe Pro
   325 330 335
   Arg Ile Pro His Met Lys Asp Pro Ile Gly Asp Ser Phe Gln Asn Asp
   340 345 350
   Lys Leu Val Val Trp Glu Ala Gly Lys Ala Gly Leu Glu Glu Cys Leu
   355 360 365
   Val Thr Glu Val Gln Val Val Gln Lys Thr
   370 375

## Beschreibung

### Einleitung und Stand der Technik

Die Erfindung betrifft spezifische Binder-Wirkstoff-Konjugate (ADCs) von Kinesin Spindel Protein-Inhibitoren, wirksame Metabolite dieser ADCs, Verfahren zur Herstellung dieser ADCs, die Verwendung dieser ADCs zur Behandlung und/oder Prävention von Krankheiten sowie die Verwendung dieser ADCs zur Herstellung von Arzneimitteln zur Behandlung und/oder Prävention von Krankheiten, insbesondere von hyperproliferativen und/oder angiogenen Erkrankungen wie beispielsweise Krebserkrankungen. Solche Behandlungen können als Monotherapie oder auch in Kombination mit anderen Arzneimitteln oder weiteren therapeutischen Maßnahmen erfolgen.

Krebserkrankungen sind die Folge unkontrollierten Zellwachstums verschiedenster Gewebe. In vielen Fällen dringen die neuen Zellen in bestehende Gewebe ein (invasives Wachstum), oder sie metastasieren in entfernte Organe. Krebserkrankungen treten in verschiedensten Organen auf und haben oft gewebespezifische Krankheitsverläufe. Daher beschreibt die Bezeichnung Krebserkrankung als Oberbegriff eine große Gruppe definierter Erkrankungen verschiedener Organe, Gewebe und Zelltypen.

Tumore früher Stadien lassen sich gegebenenfalls durch chirurgische und radiotherapeutische Maßnahmen entfernen. Metastasierte Tumore können im Regelfall durch Chemotherapeutika nur palliativ therapiert werden. Ziel hierbei ist, die optimale Kombination aus einer Verbesserung der Lebensqualität und der Verlängerung der Lebenszeit zu erreichen.

Konjugate von Binderproteinen mit einem oder mehreren Wirkstoffmolekülen sind bekannt, insbesondere in Form sogenannter "antibody drug conjugates" (ADCs), in welchen ein internalisierender, gegen ein Tumor-assoziiertes Antigen gerichteter Antikörper auf kovalente Weise über eine Verknüpfungseinheit ("linker") mit einem zytotoxisch wirkenden Agens verbunden ist. Nach Einschleusung des ADCs in die Tumorzelle und anschließender Spaltung des Konjugats wird dann innerhalb der Tumorzelle entweder das zytotoxische Agens selbst oder ein anderer daraus gebildeter zytotoxisch wirksamer Metabolit freigesetzt, und kann dort seine Wirkung direkt und selektiv entfalten. Auf diesem Wege könnte die Schädigung von normalem Gewebe im Vergleich zu einer konventionellen Chemotherapie der Krebserkrankung in signifikant engeren Grenzen gehalten werden [siehe z.B. J. M. Lambert, Curr. Opin. Pharmacol. 5, 543-549 (2005); A. M. Wu und P. D. Senter, Nat. Biotechnol. 23, 1137-1146 (2005); P. D. Senter, Curr. Opin. Chem. Biol. 13, 235-244 (2009); L. Ducry und B. Stump, Bioconjugate Chem. 21, 5-13 (2010)]. So beschreibt WO2012/171020 ADCs, bei denen mehrere Toxophormoleküle über einen polymeren Linker mit einem Antikörper verknüpft ist. Als mögliche Toxophore werden in WO2012/171020 unter anderem die Substanzen SB 743921, SB 715992 (Ispinesib), MK-0371, AZD8477, AZ3146 und ARRY-520 erwähnt.

Die zuletzt genannten Substanzen sind sogenannte Kinesin Spindel Protein-Inhibitoren. Kinesin Spindel Protein (KSP, auch bekannt als Eg5, HsEg5, KNSL1 oder KIF11) ist ein Kinesin-ähnliches Motorprotein, welches für die Funktion der bipolaren mitotischen Spindel essentiell ist. Die Inhibierung von KSP führt zum mitotischen Stillstand und über einen längeren Zeitraum zur Apoptose (Tao et al., Cancer Cell 2005 Jul 8(1), 39-59). Nach dem Auffinden des ersten zellgängigen KSP-Inhibitors, Monastrol, haben sich KSP-Inhibitoren als eine Klasse neuer Chemotherapeutika etabliert (Mayer et al., Science 286: 971-974, 1999) und sind Gegenstand einer Reihe von Patentanmeldungen (z.B. WO2006/044825; WO2006/002236; WO2005/051922; WO2006/060737; WO03/060064; WO03/040979; und WO03/049527). Da KSP jedoch nur in einem kurzen Zeitraum der Mitosephase aktiv ist, müssen KSP-Inhibitoren in ausreichend hoher Konzentration während dieser Phase vorliegen. WO2014/151030 offenbar ADCs mit bestimmten KSP-Inhibitoren.

Die Patentanmeldungen WO2015/096982 und WO2016/096610 offenbaren weitere ADCs mit KSP-Inhibitoren.

### Zusammenfassung der Erfindung

Trotz verschiedener Offenbarungen von Antikörper-Wirkstoff-Konjugaten ist es die Aufgabe der vorliegenden Erfindung, Substanzen bereitzustellen, die nach Verabreichung in relativ geringer Konzentration eine lang-anhaltende apoptotische Wirkung zeigen und damit für die Krebstherapie nützlich sein können. Hierbei spielt das Profil der aus den ADCs intrazellulär freigesetzten Metabolite eine entscheidende Rolle. Häufig sind die aus ADCs gebildeten Metabolite Substrate von Efflux-Pumpen und/oder weisen eine hohe Permeabilität durch Zellmembranen auf. Beide Phänomene können zu einer kurzen Verweildauer und so zu einer suboptimalen apoptotischen Wirkung in der Tumorzelle beitragen.

Gegenstand der vorliegenden Erfindung sind ADCs mit einer spezifischen Toxophor-Linker-Zusammensetzung, die insbesondere sowohl in Verbindung mit einem spezifischen anti-CD123- als auch mit einem anti-CXCR5 Antikörper ein verbessertes Wirkprofil aufweisen. Der Gegenstand der vorliegenden Erfindung wird durch die Ansprüche definiert.

Der Antikörper ist vorzugsweise ein humanisierter oder chimärer monoklonaler anti-CD123-Antikörper oder ein anti-CXCR5-Antikörper. Besonders bevorzugt sind die humanisierten anti-CD123 Antikörper TPP-8987, TPP-8988 und TPP-9476 sowie die humanisierten oder chimärenanti-CXCR5 Antikörper TPP-9024, TPP-9574 und TPP-9580.

Es wurde nun gefunden, dass Antikörper-Wirkstoff-Konjugate (ADCs) der Formel (I) 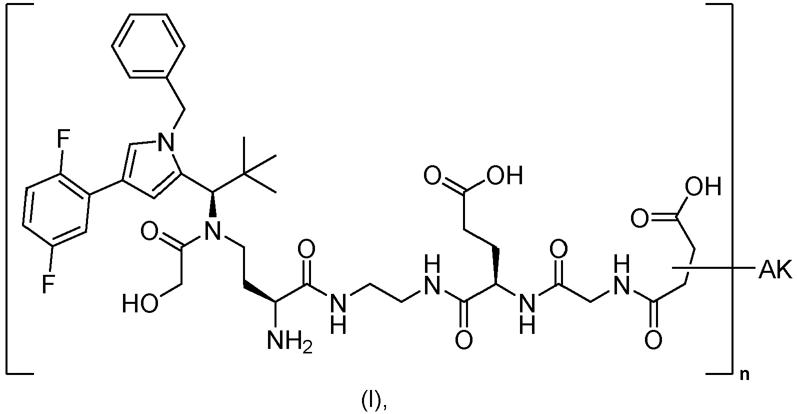 in der
- n: für 1 bis 8 steht,
- AK: für einen anti-CD123 Antikörper, ausgewählt aus der Gruppe bestehend aus TPP-8987, TPP-9476 und TPP-8988 steht, oder
- AK: für einen anti-CXCR5 Antikörper, bevorzugt ausgewählt aus der Gruppe bestehend aus TPP-9574, TPP-9580 und TPP-9024 steht, oder
- AK: für ein Antigen-bindendes Fragment von diesen Antikörpern steht,
wobei der Antikörper oder das Antigen-bindende Fragment über ein Schwefelatom einer Cystein-Seitengruppe gebunden ist,
sowie deren Salze, Solvate und Salze dieser Solvate, überlegene Eigenschaften gegenüber den bekannten Konjugaten aufweisen.

Bevorzugt sind solche Antikörper-Wirkstoff-Konjugate (ADCs) der Formel (I), in der n für 4 bis 8 steht.

Bevorzugt sind solche Antikörper-Wirkstoff-Konjugate (ADCs) der Formel (I), in der AK für einen anti-CD123 Antikörper, ausgewählt aus der Gruppe bestehend aus TPP-8987, TPP-9476 und TPP-8988 sowie für ein Antigen-bindendes Fragment von diesen Antikörpern steht; insbesondere bevorzugt steht AK für TPP-9476, sowie für ein Antigen-bindendes Fragment von diesem Antikörper.

### Beschreibung der Figuren

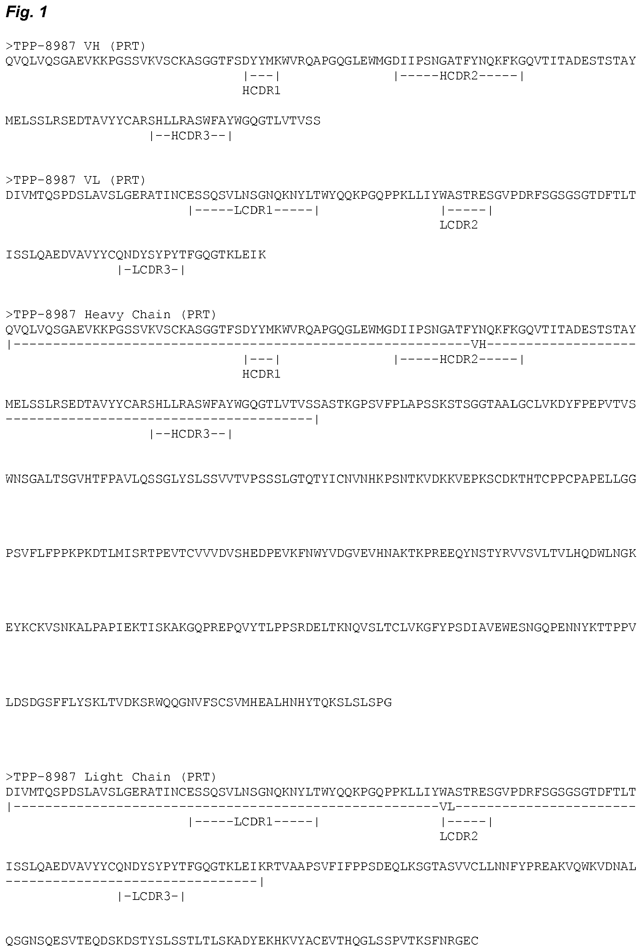

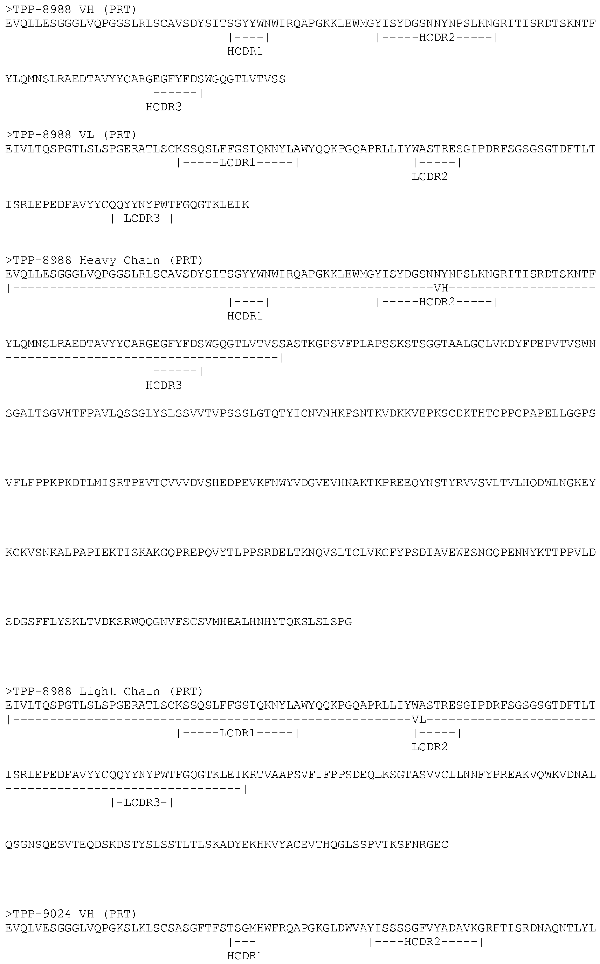

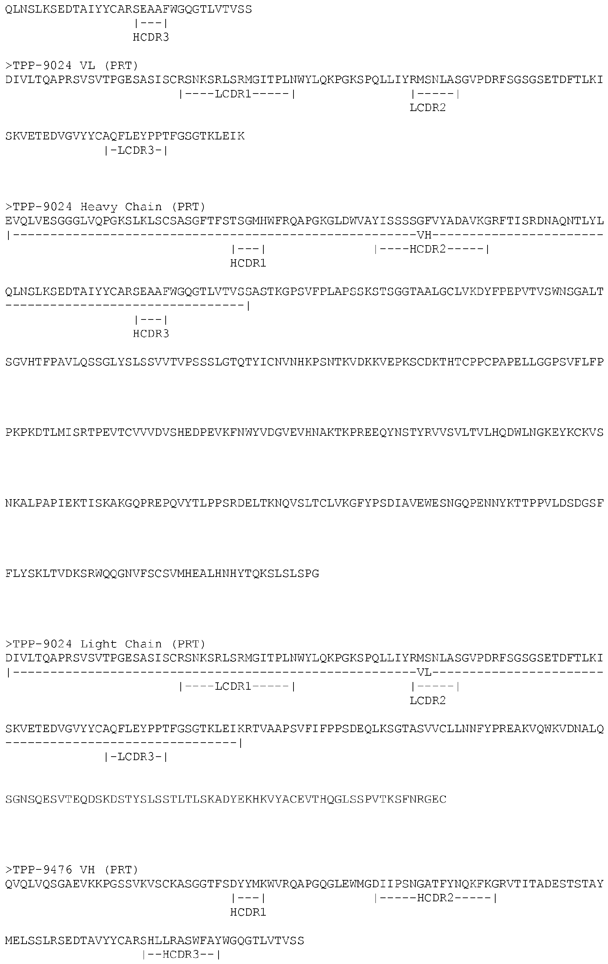

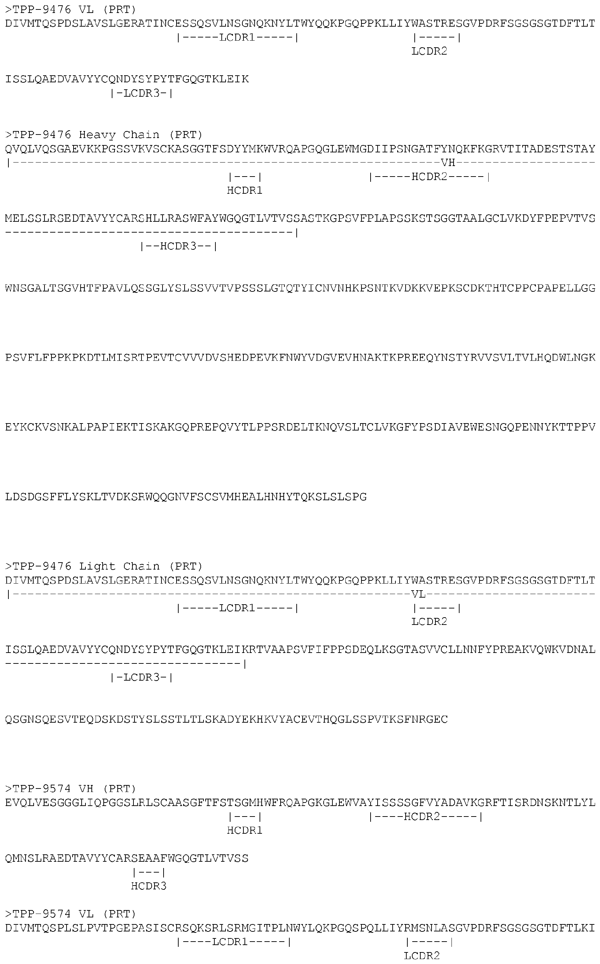

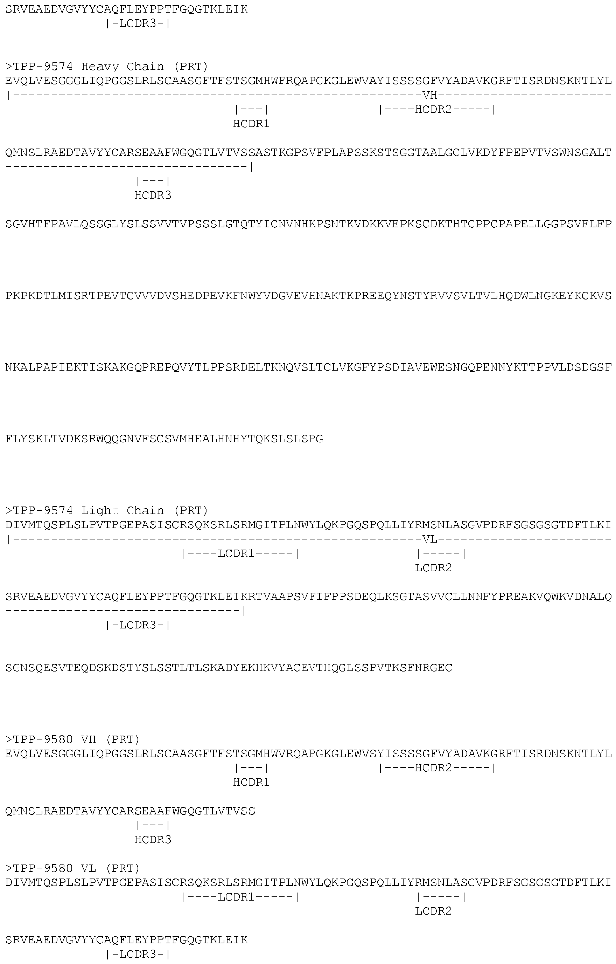

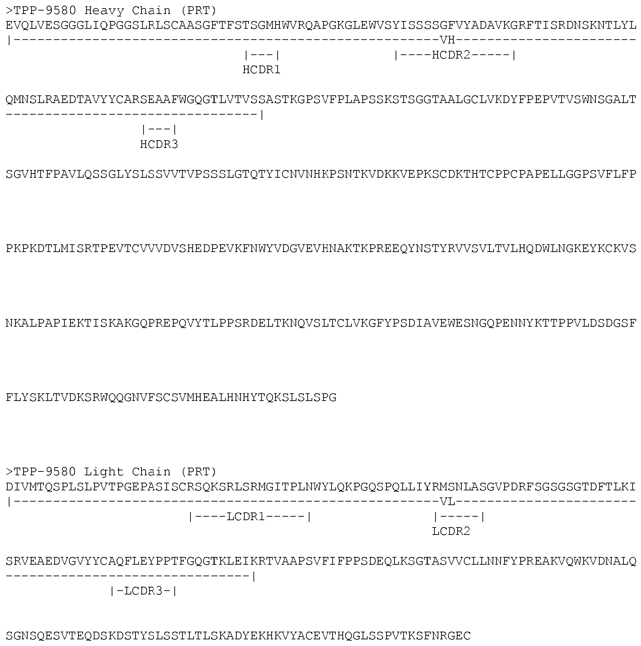

**Fig. 1****:** Annotierte Sequenzen von bevorzugten Antikörpern für Binder-Wirkstoff-Konjugate. Gezeigt sind die Proteinsequenzen der schweren und leichten Ketten der IgGs, sowie die VH und VL Regionen dieser Antikörper. Unterhalb der Sequenzen werden wichtige Regionen annotiert (VH und VL Regionen in IgGs, und die CDR Regionen (H-CDR1, H-CDR2, H-CDR3, L-CDR1, L-CDR2, L-CDR3)).

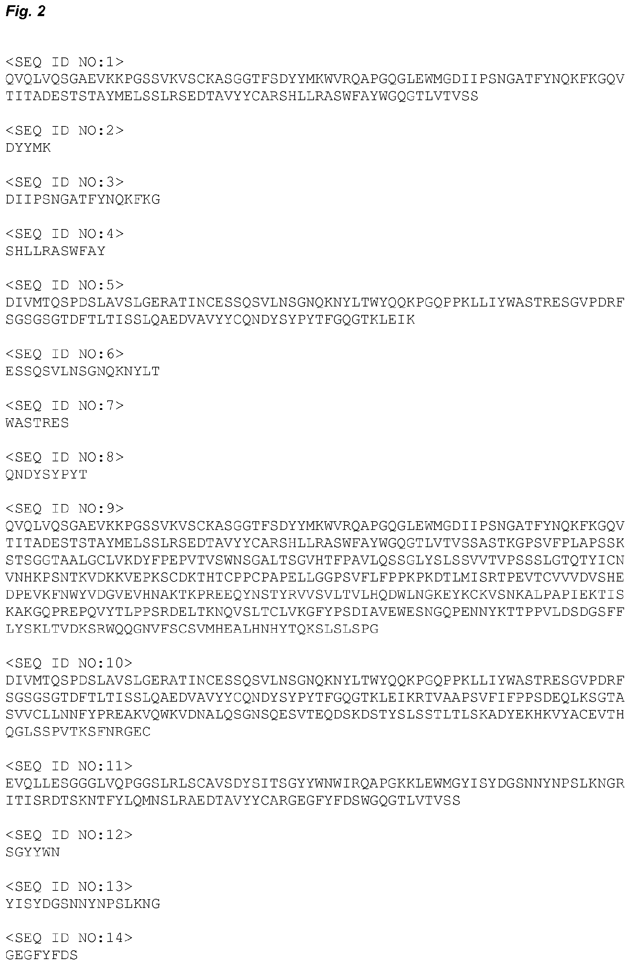

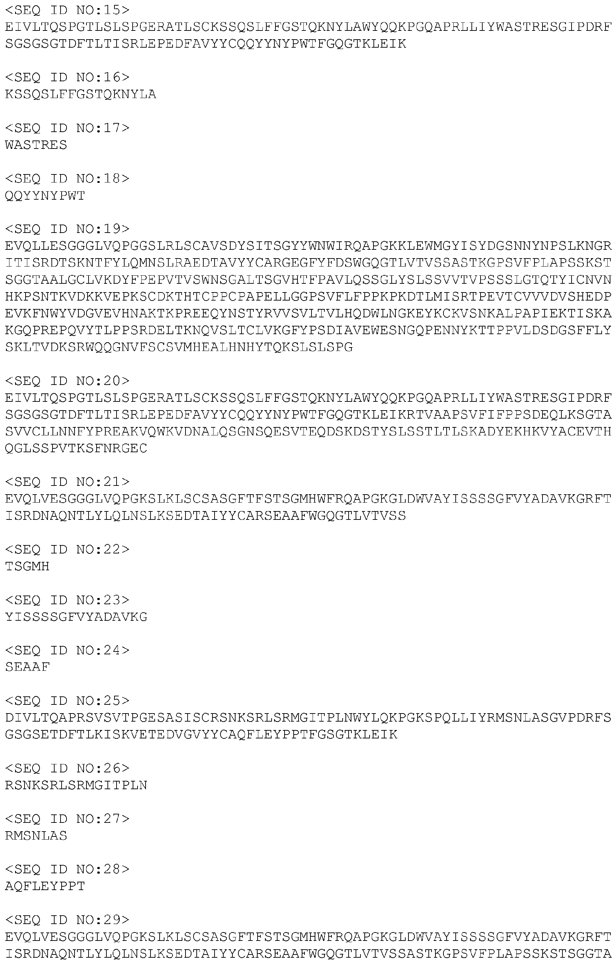

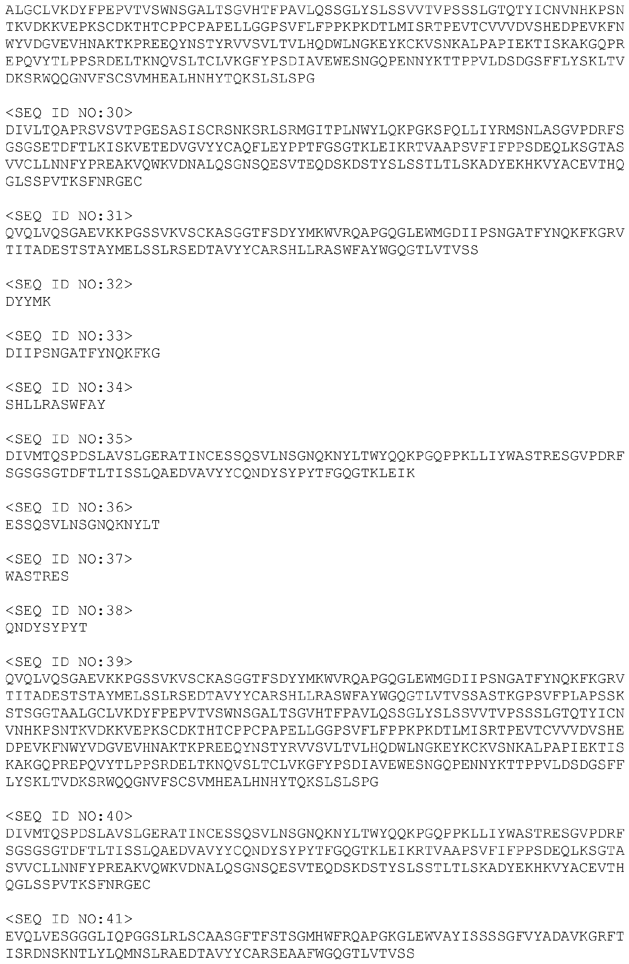

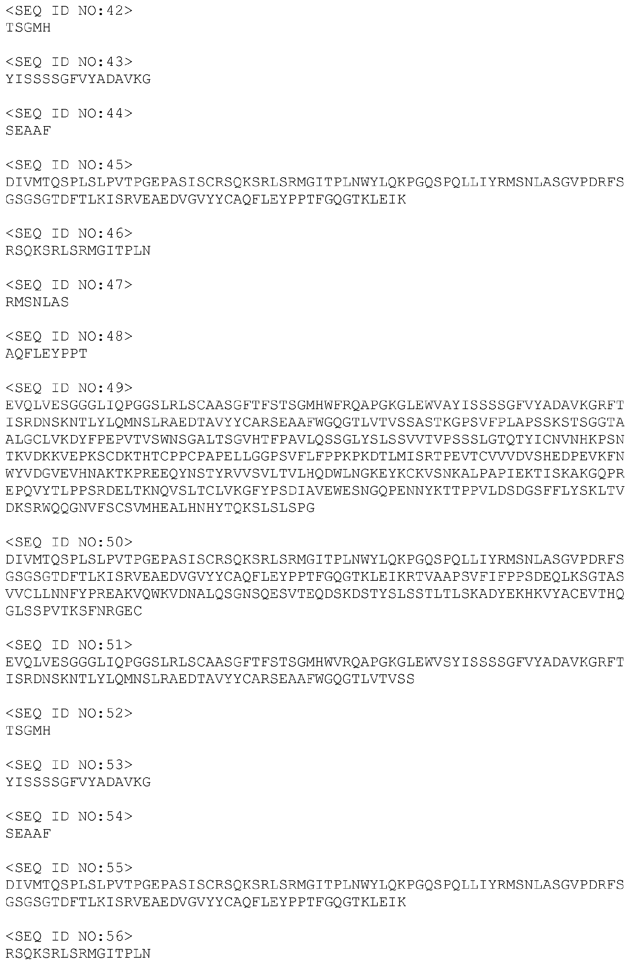

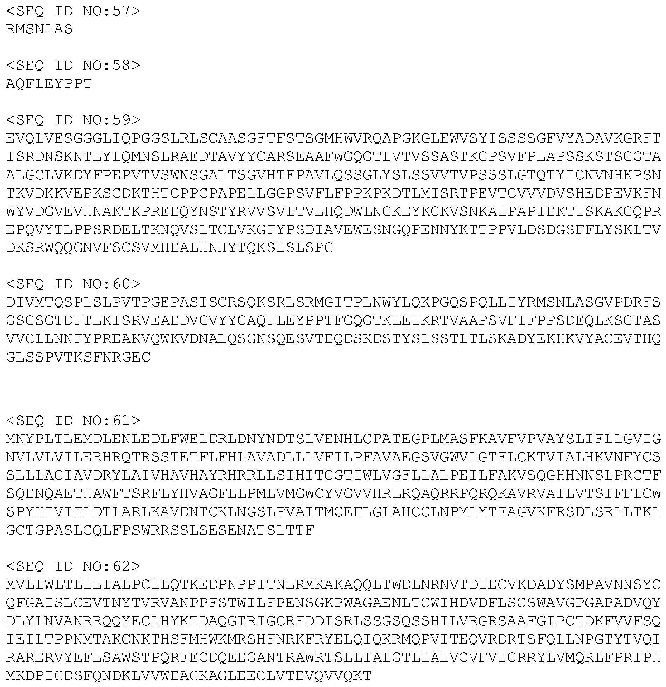

**Fig. 2****:** Sequenzlisting von Sequenzen der bevorzugten Antikörper für Binder-Wirkstoff-Konjugate und von Sequenzen der Zielmoleküle.

### Detaillierte Beschreibung der Erfindung

Die Erfindung stellt Konjugate eines humanisierten anti-CD123 Antikörpers oder eines humanisierten oder chimären monoklonalen anti-CXCR5 Antikörpers bereit, wobei das Wirkstoffmolekül ein Kinesin Spindel Protein-Inhibitor (KSP-Inhibitor) ist, das mit dem Antikörper über einen Linker L verbunden ist. Besonders bevorzugt sind hierbei die humanisierten anti-CD123 Antikörper TPP-8987, TPP-8988 und TPP-9476 sowie die humanisierten oder chimären anti-CXCR5 Antikörper TPP-9024, TPP-9574 und TPP-9580.

### Binder

Der Begriff "Binder" wird im breitesten Sinne als ein Molekül verstanden, welches an ein Zielmolekül, das auf einer bestimmten, mit dem Binder-Wirkstoffkonjugat zu adressierenden Zielzellen-Population vorhanden ist, bindet. Der Begriff Binder ist in seiner breitesten Auslegung zu verstehen und umfasst z.B. auch Lektine, Proteine die bestimmte Zuckerketten binden können, oder Phospholipid-bindende Proteine. Solche Binder umfassen beispielsweise hochmolekulare Proteine (Bindeproteine), Polypeptide oder Peptide (Bindepeptide), nicht-peptidische (z.B. Aptamere (US5,270,163) Übersichtsartikel von Keefe AD., et al., Nat. Rev. Drug Discov. 2010; 9:537-550), oder Vitamine) und alle anderen zellbindenden Moleküle oder Substanzen. Bindeproteine sind z.B. Antikörper und Antikörperfragmente oder Antikörpermimetika wie z.B. Affibodies, Adnectins, Anticalins, DARPins, Avimers, Nanobodies (Übersichtsartikel von Gebauer M. et al., Curr. Opinion in Chem. Biol. 2009; 13:245-255; Nuttall S.D. et al., Curr. Opinion in Pharmacology 2008; 8:608-617). Bindepeptide sind z.B. Liganden eines Liganden-Rezeptorspaares, wie z.B. VEGF des Liganden-Rezeptorpaares VEGF/KDR, wie Transferrin des Liganden-Rezeptorpaares Transferrin/Transferrin-Rezeptor oder Cytokine/Cytokin-Rezeptor, wie TNFalpha des Liganden-Rezeptorpaares TNFalpha/TNFalpha Rezeptor.

Der Binder kann ein Bindeprotein sein. Bevorzugte Ausführungsformen der Binder sind ein Antikörper, ein antigen-bindendes Antikörperfragment, ein multispezifischer Antikörper oder ein Antikörpermimetikum.

Aus der Literatur sind verschiedene Möglichkeiten der kovalenten Kopplung (Konjugation) von organischen Molekülen an Binder und insbesondere Antikörper bekannt. Erfindungsgemäß bevorzugt ist die Konjugation der Toxophore an den Antikörper über ein oder mehrere Schwefelatome von Cystein-Resten des Antikörpers und/oder über ein oder mehrere NH-Gruppen von Lysin-Resten des Antikörpers. Es ist jedoch auch möglich, das Toxophor an den Antikörper über freie Carboxylgruppen oder über Zuckerreste des Antikörpers zu binden.

Ein "Zielmolekül" wird im breitesten Sinne als ein Molekül verstanden, welches in der Zielzellenpopulation vorhanden ist, und kann ein Protein (z.B. ein Rezeptor eines Wachstumsfaktors) oder ein nicht-peptidisches Molekül sein (z.B. ein Zucker oder Phospholipid). Bevorzugt ist es ein Rezeptor oder ein Antigen.

Der Begriff "extrazelluläres" Zielmolekül beschreibt ein an die Zelle gebundenes Zielmolekül, welches sich auf der Außenseite einer Zelle befindet oder den Teil eines Zielmoleküls, welcher sich auf der Außenseite einer Zelle befindet, d.h. ein Binder kann an einer intakten Zelle an sein extrazelluläres Zielmolekül binden. Ein extrazelluläres Zielmolekül kann in der Zellmembran verankert sein oder Bestandteil der Zellmembran sein. Der Fachmann kennt Verfahren, um extrazelluläre Zielmoleküle zu identifizieren. Für Proteine kann dies über eine Bestimmung der Transmembrandomäne(n) und die Orientierung des Proteins in der Membran geschehen. Diese Angaben sind in der Regel in Proteindatenbanken (z.B. SwissProt) hinterlegt.

Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Der Binder kann über eine Bindung mit dem Linker verknüpft werden. Die Verknüpfung des Binders kann mittels eines Heteroatoms des Binders erfolgen. Erfindungsgemäße Heteroatome des Binders, die zur Verknüpfung verwendet werden können, sind Schwefel (in einer Ausführungsform via einer Sulfhydrylgruppe des Binders), Sauerstoff (erfindungsgemäß mittels einer Carboxyl oder Hydroxylgruppe des Binders) und Stickstoff (in einer Ausführungsform via einer primären oder sekundären Amingruppe oder Amidgruppe des Binders). Diese Heteroatome können im natürlichen Binder vorhanden sein oder durch chemische oder molekularbiologische Methoden eingeführt werden. Erfindungsgemäß hat die Verknüpfung des Binders mit dem Toxophor nur einen geringen Einfluss auf die Bindeaktivität des Binders zum Zielmolekül. In einer bevorzugten Ausführungsform hat die Verknüpfung keinen Einfluss auf die Bindeaktivität des Binders zum Zielmolekül.

Der Begriff "Antikörper" wird gemäß der vorliegenden Erfindung in seinem breitesten Sinne verstanden und umfasst Immunglobulinmoleküle, beispielsweise intakte oder modifizierte monoklonale Antikörper, polyklonale Antikörper oder multispezifische Antikörper (z.B. bispezifische Antikörper). Ein Immunglobulinmolekül umfasst bevorzugt ein Molekül mit vier Polypeptidketten, zwei schwere Ketten (H Ketten) und zwei leichte Ketten (L Ketten), welche typischerweise durch Disulfidbrücken verknüpft sind. Jede schwere Kette umfasst eine variable Domäne der schweren Kette (abgekürzt VH) und konstante Domäne der schweren Kette. Die konstante Domäne der schweren Kette kann beispielsweise drei Domänen CH1, CH2 und CH3 umfassen. Jede leichte Kette umfasst eine variable Domäne (abgekürzt VL) und eine konstante Domäne. Die konstante Domäne der leichten Kette umfasst eine Domäne (abgekürzt CL). Die VH und VL Domänen können weiter unterteilt werden in Regionen mit Hypervariabilität, auch Komplementaritäts-bestimmende Regionen genannt ("complementarity determining region", abgekürzt CDR) und Regionen mit geringerer Sequenzvariabilität ("framework region", abgekürzt FR). Jede VH und VL Region setzt sich typischerweise aus drei CDRs und bis zu vier FRs zusammen. Beispielsweise vom Aminoterminus zum Carboxyterminus in der folgenden Reihenfolge FR1, CDR1, FR2, CDR2, FR3, CDR3, FR4. Ein Antikörper kann aus jeder dafür geeigneten Spezies erhalten werden z.B. Kaninchen, Lama, Kamel, Maus, oder Ratte. In einer Ausführungsform ist der Antikörper humanen oder murinen Ursprungs. Ein Antikörper kann z.B. human, humanisiert oder chimär sein.

Der Begriff "monoklonaler" Antikörper bezeichnet Antikörper, die aus einer Population substantiell homogener Antikörper erhalten wurde, d.h. individuelle Antikörper der Population sind bis auf natürlich auftretende Mutationen, welche in geringfügiger Anzahl auftreten können, identisch. Monoklonale Antikörper erkennen mit hoher Spezifität eine einzige antigene Bindestelle. Der Begriff monoklonaler Antikörper bezieht sich nicht auf ein bestimmtes Herstellungsverfahren.

Der Begriff "intakter" Antikörper bezieht sich auf Antikörper, die sowohl eine Antigen-bindende Domäne als auch die konstante Domäne der leichten und schweren Kette umfassen. Die konstante Domäne kann eine natürlich vorkommende Domäne sein, oder eine Variante davon, bei der mehrere Aminosäurepositionen verändert wurden, und kann auch aglykosyliert sein.

Der Begriff "modifizierter intakter" Antikörper bezieht sich auf intakte Antikörper, die mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert wurden. Des Weiteren können Antikörper dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug;26(8):925-32).

Mit "Aminosäuremodifikation" oder "Mutation" ist hier eine Aminosäuresubstitution, -insertion, und/oder eine -deletion in einer Polypeptidsequenz gemeint. Die bevorzugte Aminosäuremodifikation ist hier eine Substitution. Mit "Aminosäuresubstitution" oder "Substitution" ist hier ein Austausch einer Aminosäure an einer gegebenen Position in einer Proteinsequenz durch eine andere Aminosäure gemeint. Zum Beispiel beschreibt die Substitution Y50W eine Variante eines parentalen Polypeptids, in welcher das Tyrosin an Position 50 durch ein Tryptophan ausgetauscht ist. Eine "Variante" von einem Polypeptid beschreibt ein Polypeptid, welches eine Aminosäuresequenz hat, die substanziell identisch zu einem Referenzpolypeptid ist, typischerweise einem nativen oder "parentalen" Polypeptid. Die Polypeptidvariante kann eine oder mehrere Aminosäureaustauche, -deletionen, und/oderinsertionen an bestimmten Positionen in der nativen Aminosäuresequenz aufweisen.

Der Begriff "humaner" Antikörper bezeichnet Antikörper, die aus einem Menschen erhalten werden können oder synthetische humane Antikörper sind. Ein "synthetischer" humaner Antikörper ist ein Antikörper, der in Teilen oder schweren als Ganzes von synthetischen Sequenzen in silico erhältlich ist, die auf der Analyse humaner Antikörpersequenzen basieren. Ein humaner Antikörper kann z.B. durch eine Nukleinsäure kodiert sein, die aus einer Bibliothek von Antikörpersequenzen, die humanen Ursprungs sind, isoliert wurde. Ein Beispiel solcher Antikörper ist in Söderlind et al., Nature Biotech. 2000, 18:853-856 zu finden. Solche "humanen" und "synthetischen" Antikörper beinhalten auch aglykosylierte Varianten, die entweder durch Deglykosylierung durch PNGaseF oder durch Mutation von N297 (kabat Nummerierung) der schweren Kette zu einer beliebigen anderen Aminosäure hergestellt wurden.

Der Begriff "humanisierter" oder "chimärer" Antikörper beschreibt Antikörper, die aus einem nicht-humanen und einem humanen Sequenzanteil bestehen. Bei diesen Antikörpern wird ein Teil der Sequenzen des humanen Immunoglobulins (Rezipient) durch Sequenzanteile eines nicht-humanen Immunoglobulins (Donor) ersetzt. Der Donor ist in vielen Fällen ein murines Immunoglobulin. Bei humanisierten Antikörpern werden Aminosäuren der CDR des Rezipienten durch Aminosäuren des Donors ersetzt. Manchmal werden auch noch Aminosäuren des Frameworks durch korrespondierende Aminosäuren des Donors ersetzt. In manchen Fällen enthält der humanisierte Antikörper Aminosäuren, die weder im Rezipient noch im Donor enthalten waren und die während der Optimierung des Antikörpers eingefügt wurden. Bei chimären Antikörpern werden die variablen Domänen des Donor-Immunoglobulins mit den konstanten Regionen eines humanen Antikörpers fusioniert. Solche "humanisierten" und "chimären" Antikörper beinhalten auch aglykosylierte Varianten, die entweder durch Deglykosylierung durch PNGaseF oder durch Mutation von N297 (Kabat Nummerierung) der schweren Kette zu einer beliebigen anderen Aminosäure hergestellt wurden.

Der Begriff Komplementaritäts-bestimmende Region (CDR) wie er hier verwendet wird, bezieht sich auf diejenigen Aminosäuren einer variablen Antikörperdomäne, die für die Bindung an das Antigen notwendig sind. Jede variable Region hat typischerweise drei CDR Regionen, welche als CDR1, CDR2 und CDR3 bezeichnet werden. Jede CDR Region kann Aminosäuren nach der Definition von Kabat und/oder Aminosäuren eines Hypervariablen Loops definiert nach Chotia umfassen. Die Definition nach Kabat umfasst zum Beispiel die Region von ungefähr Aminosäureposition 24 - 34 (CDR1), 50 - 56 (CDR2) und 89 - 97 (CDR3) der variablen leichten Kette / Domäne (VL) und 31 - 35 (CDR1), 50 - 65 (CDR2) und 95 - 102 (CDR3) der variablen schweren Kette / Domäne (VH) (Kabat et al., Sequences of Proteins of Immunological Interest, 5th Ed. Public Health Service, National Institutes of Health, Bethesda, MD. (1991)). Die Definition nach Chotia umfasst zum Beispiel die Region von ungefähr Aminosäureposition 26 - 32 (CDR1), 50 - 52 (CDR2) und 91 -96 (CDR3) der variablen leichetn Kette (VL) und 26 - 32 (CDR1), 53 - 55 (CDR2) und 96 - 101 (CDR3) der variablen schweren Kette (VH) Chothia and Lesk; J Mol Biol 196: 901-917 (1987)). In manchen Fällen kann eine CDR Aminosäuren aus einer CDR Region definiert nach Kabat und Chotia umfassen.

Abhängig von der Aminosäure-Sequenz der konstanten Domäne der schweren Kette können Antikörper in verschiedene Klassen eingeteilt werden. Es gibt fünf Hauptklassen von intakten Antikörpern: IgA, IgD, IgE, IgG und IgM, wobei mehrere davon in weitere Unterklassen aufgegliedert werden können. (Isotypen), z.B. IgG1, IgG2, IgG3, IgG4, IgA1 und IgA2. Die konstante Domäne der schweren Kette, die zu den unterschiedlichen Klassen korrespondieren, werden als [alpha/α], [delta/δ], [epsilon/ε], [gamma/γ] und [my/µ] bezeichnet. Sowohl die dreidimensionale Struktur als auch die Untereinheitenstruktur von Antikörpern sind bekannt.

Der Begriff "funktionales Fragment" oder "antigen-bindendes Antikörperfragment" eines Antikörpers/Immunoglobulins ist definiert als ein Fragment eines Antikörpers/Immunoglobulins (z.B. die variable Domänen eines IgG), welches die Antigen-Bindedomänen des Antikörpers/Immunoglobulins noch umfasst. Die "Antigen-Bindedomäne" eines Antikörpers umfasst typischerweise eine oder mehrere Hypervariable Regionen eines Antikörpers, z.B. die CDR, CDR2 und/oder CDR3 Region. Allerdings kann auch die "Framework" oder die "Gerüst" Region eines Antikörpers zur Bindung des Antikörpers an das Antigen eine Rolle spielen. Die Framework Region bildet das Gerüst für die CDRs. Vorzugsweise umfasst die Antigen-Bindedomäne mindestens die Aminosäuren 4 bis 103 der variablen leichten Kette und Aminosäure 5 bis 109 der variablen schweren Kette, bevorzugter die Aminosäure 3 bis 107 der variablen leichten Kette und 4 bis 111 der variablen schweren Kette, besonders bevorzugt sind die kompletten variablen leichten und schweren Ketten , also Aminosäure 1 - 109 der VL und 1 bis 113 der VH (Nummerierung nach WO97/08320).

"Funktionale Fragmente" oder "antigen-bindende Antikörperfragmente" der Erfindung umfassen nicht abschließend Fab, Fab', F(ab')₂ und Fv Fragmente, Diabodies, Single Domain Antibodies (DAbs), lineare Antikörper, Einzelketten Antikörper (single-chain Fv, abgekürzt scFv); und multispezifische, wie z.B. bi- und tri-spezifische, Antikörper, gebildet aus Antikörperfragmenten C. A. K Borrebaeck, editor (1995) Antibody Engineering (Breakthroughs in Molecular Biology), Oxford University Press; R. Kontermann & S. Duebel, editors (2001) Antibody Engineering (Springer Laboratory Manual), Springer Verlag). Andere Antikörper als "multi-spezifische" oder "multi-funktionale" sind solche mit identischen Bindestellen. Multispezifische Antikörper können spezifisch für unterschiedliche Epitope eines Antigens sein oder spezifisch für Epitope von mehr als einem Antigen sein (siehe z.B. WO 93/17715; WO 92/08802; WO 91/00360; WO 92/05793; Tutt, et al., 1991, J. Immunol. 147:60 69; U. S. Pat. Nos. 4,474,893; 4,7 14,68 1 ; 4,925,648; 5,573,920; 5,601,8 19; oder Kostelny et al., 1992, J. Immunol. 148: 1547 1553). Ein F(ab')₂ oder Fab Molekül kann so konstruiert werden, dass die Zahl der intermolekularen Disulfidinteraktionen, die zwischen den Ch1 und den CL Domänen stattfindet, reduziert oder komplett verhindert werden kann.

"Epitope" bezeichnen Proteindeterminanten, die eine spezifische Bindung mit einem Immunglobulin oder T-Zell-Rezeptoren eingehen können. Epitopische Determinanten bestehen normalerweise aus chemisch aktiven Oberflächengruppen von Molekülen wie Aminosäuren oder Zuckerseitenketten, oder Kombinationen hiervon, und weisen normalerweise spezifische 3-dimensionale Struktureigenschaften wie auch spezifische Ladungseigenschaften auf.

"Funktionale Fragmente" oder "antigen-bindende Antikörperfragmente" können mit einem weiteren Polypeptid oder Protein, welche nicht von einem Antikörper stammen, über ihren Aminoterminus oder Carboxyterminus mittels einer kovalenten Bindung (z.B. einer Peptidverknüpfung) fusioniert werden. Des Weiteren können Antikörper und antigen-bindende Fragmente dahingehend modifiziert werden, dass an definierten Stellen reaktive Cysteine eingeführt werden, um die Kopplung an ein Toxophor zu erleichtern (siehe Junutula et al. Nat Biotechnol. 2008 Aug; 26(8):925-32).

Polyklonale Antikörper können durch für den Durchschnittsfachmann bekannte Methoden hergestellt werden. Monoklonale Antiköper können durch für den Durchschnittsfachmann bekannte Methoden hergestellt werden (Köhler und Milstein, Nature, 256, 495-497, 1975). Humane bzw. humanisierte monoklonale Antiköper können durch für den Durchschnittsfachmann bekannte Methoden hergestellt werden (Olsson et al., Meth Enzymol. 92, 3-16 bzw. Cabilly et al US 4,816,567 oder Boss et al US 4,816,397).

Der Durchschnittsfachmann kennt vielfältige Methoden, um humane Antikörper und deren Fragmente herzustellen, wie beispielsweise mittels transgener Mäuse (N Lonberg und D Huszar, Int Rev Immunol. 1995; 13(1):65-93) oder Phage Display Technologien (Clackson et al., Nature. 1991 Aug 15;352(6336):624-8). Antikörper der Erfindung können aus rekombinanten Antikörperbibliotheken erhalten werden, die z.B. auf den Aminosäuresequenzen einer Vielzahl von Antikörpern besteht, die aus einer großen Anzahl gesunder Freiwilliger erstellt wurden. Antikörper können auch mittels bekannter rekombinanter DNS-Technologien hergestellt werden. Die Nukleinsäuresequenz eines Antikörpers kann durch Routinesequenzierung erhalten werden, oder ist aus öffentlich zugänglichen Datenbanken erhältlich.

Ein "isolierter" Antikörper oder Binder wurde von anderen Bestandteilen der Zelle gereinigt. Kontaminierende Bestandteile einer Zelle, welche mit einer diagnostischen oder therapeutischen Verwendung interferieren können, sind z.B. Enzyme, Hormone, oder andere peptidische- oder nicht-peptidische Bestandteile einer Zelle. Bevorzugt ist ein Antikörper oder Binder, der zu mehr als 95 Gew-% bezogen auf den Antikörper bzw. Binder aufgereinigt wurde (bestimmt durch z.B. Lowry Verfahren, UV-Vis Spektroskopie oder durch SDS-Kapillargelelektrophorese). Außerdem ein Antikörper, der soweit aufgereinigt wurde, dass mindestens 15 Aminosäuren des Aminoterminus oder einer internen Aminosäuresequenz bestimmt werden können, oder zur Homogenität aufgereinigt wurde, wobei die Homogenität bestimmt wird durch SDS-PAGE unter reduzierenden oder nicht-reduzierenden Bedingungen (die Detektion kann mittels Coomassie Blau Anfärbung oder bevorzugt durch Silberfärbung bestimmt werden). Jedoch wird ein Antikörper normalerweise durch einen oder mehrere Reinigungsschritte hergestellt.

Der Begriff "spezifische Bindung" oder "bindet spezifisch" bezieht sich auf einen Antikörper oder Binder, der an ein vorbestimmtes Antigen/Zielmolekül bindet. Spezifische Bindung eines Antikörpers oder Binders beschreibt typischerweise einen Antikörper bzw. Binder mit einer Affinität von mindestens 10⁻⁷ M_(als Kd-Wert; also vorzugsweise solche mit kleineren Kd-Werten als 10⁻⁷ M), wobei der Antikörper bzw. Binder eine mindestens zweifach höhere Affinität zum vorbestimmten Antigen/Zielmolekül als zu einem nicht-spezifischen Antigen/Zielmolekül hat (z.B. Rinder Serumalbumin, oder Casein), welches nicht das vorbestimmte Antigen/Zielmolekül oder ein eng verwandtes Antigen/Zielmolekül ist.

Spezifische Bindung eines Antikörpers oder Binders schließt nicht aus, dass der Antikörpers oder Binder an mehrere Antigene/Zielmoleküle bindet (z.B. Orthologe aus verschiedenen Spezies). Die Antikörper weisen vorzugsweise eine Affinität von mindestens 10⁻⁷ M (als Kd-Wert; also vorzugsweise solche mit kleineren Kd-Werten als 10⁻⁷ M), vorzugsweise von mindestens 10⁻⁸ M, besonders bevorzugt in dem Bereich von 10⁻⁹ M bis 10⁻¹¹ M auf. Die Kd-Werte können durch z.B. Oberflächenplasmonresonanzspektroskopie bestimmt werden.

Die erfindungsgemäßen Antikörper-Wirkstoff-Konjugate weisen ebenfalls Affinitäten in diesen Bereichen auf. Durch die Konjugation der Wirkstoffe wird die Affinität vorzugsweise nicht wesentlich beeinflusst (in der Regel wird die Affinität weniger als eine Größenordnung verringert, also z.B. maximal von 10⁻⁸ M auf 10⁻⁷ M).

Die erfindungsgemäßen verwendeten Antikörper zeichnen sich weiterhin vorzugsweise durch eine hohe Selektivität aus. Eine hohe Selektivität liegt vor, wenn der erfindungsgemäße Antikörper eine mindestens um den Faktor 2, bevorzugt Faktor 5 oder insbesondere bevorzugt Faktor 10 bessere Affinität am Zielprotein aufweist als an einem unabhängigen anderen Antigen, z.B. humanem Serumalbumin (die Affinität kann z.B. durch Oberflächenplasmonenresonanzspektroskopie bestimmt werden).

Zudem sind die erfindungsgemäßen verwendeten Antikörper vorzugsweise kreuzreaktiv. Um präklinische Studien, z.B. toxikologische oder Wirksamkeitsstudien (z.B. in Xenograft-Mäusen), zu erleichtern und besser interpretieren zu können, ist es von Vorteil, wenn der erfindungsgemäß verwendete Antikörper nicht nur das humane Zielprotein bindet, sondern auch in der für die Studien verwendeten Spezies das Spezies-Zielprotein bindet. In einer Ausführungsform ist der erfindungsgemäß verwendete Antikörper zusätzlich zum humanen Zielprotein kreuzreaktiv zum Zielprotein mindestens einer weiteren Spezies. Für toxikologische und Wirksamkeitsstudien werden bevorzugt Spezien der Familien Nager, Hunde und nicht-humane Primaten, verwendet. Bevorzugte Nager Spezien sind Maus und Ratte. Bevorzugte nicht-humane Primaten sind Rhesusaffen, Schimpansen und Langschwanzmakaken.

In einer Ausführungsform ist der erfindungsgemäß verwendete Antikörper zusätzlich zum humanen Zielprotein kreuzreaktiv zum Zielprotein mindestens einer weiteren Spezies ausgewählt aus der Gruppe von Spezien bestehend aus Maus, Ratte und Langschwanzmakak (Macaca fascicularis). Insbesondere bevorzugt sind erfindungsgemäß verwendete Antikörper, die zusätzlich zum humanen Zielprotein mindestens kreuzreaktiv zum Maus-Zielprotein sind. Bevorzugt sind kreuzreaktive Antikörper, deren Affinität zum Zielprotein der weiteren nicht-humanen Spezies sich nicht um mehr als den Faktor 50, insbesondere nicht mehr als den Faktor zehn von der Affinität zum humanen Zielprotein unterscheidet.

### Gegen ein Krebs-Zielmolekül gerichtete Antikörper

Bevorzugt ist das Zielmolekül, gegen das der Binder, z.B. ein Antikörper oder ein Antigen bindendes Fragment davon, gerichtet ist, ein Krebs-Zielmolekül. Der Begriff "Krebs-Zielmolekül" beschreibt ein Zielmolekül, welches auf einer oder mehreren Krebszellarten im Vergleich zu nicht-Krebszellen des gleichen Gewebetypus vermehrt vorhanden ist. Bevorzugt ist das Krebs-Zielmolekül auf einer oder mehreren Krebszellarten im Vergleich zu nicht-Krebszellen des gleichen Gewebetypus selektiv vorhanden, wobei selektiv eine mindestens zweifache Anreicherung auf Krebszellen im Vergleich zu nicht-Krebszellen des gleichen Gewebetypus beschreibt (ein "selektives Krebs-Zielmolekül"). Die Verwendung von Krebs-Zielmolekülen erlaubt die selektive Therapie von Krebszellen mit den erfindungsgemäßen Konjugaten.

Antikörper, welche spezifisch gegen ein Antigen, wie z.B. ein Krebszell-Antigen, gerichtet sind, können vom Durchschnittsfachmann mittels ihm bekannter Verfahren hergestellt werden (wie z.B. rekombinante Expression) oder kommerziell erworben werden (wie z.B. von Merck KGaA, Deustchland). Beispiele bekannter kommerziell erhältlicher Antikörper in der Krebstherapie sind Erbitux® (Cetuximab, Merck KGaA), Avastin® (Bevacizumab, Roche) und Herceptin® (Trastuzumab, Genentech). Trastuzumab ist ein rekombinanter humanisierter monokloaler Antikörper vom lgG1 kappa Typ, welcher mit hoher Affinität in einem Zell-basierten Assay (Kd = 5 nM) die extrazelluläre Domäne des humanen epidermalen Wachstumsrezeptors bindet. Der Antikörper wird rekombinant in CHO-Zellen hergestellt. Alle diese Antikörper können auch als aglykosylierte Varianten dieser Antikörper hergestellt werden, entweder durch Deglycosylierung durch PNGase F oder durch Mutation von N297 (Kabat Nummerierung) der schweren Kette zu einer beliebigen Aminosäure.

In der vorliegenden Erfindung sind die Krebs-Zielmoleküle
(1) das Rezeptorprotein CXCR5 (CD185; SwissProt: P32302; NCBI-Gene ID 643, NCBI-Referenzsequenz: NP_001707.1; SEQ ID NO: 61)
(2) der Oberflächenrezeptor CD123 (IL3RA; NCBI-Gene ID: 3563; NCBI-Referenzsequenz: NP_002174.1; Swiss-Prot: P26951; SEQ ID NO: 62)

In einem besonders bevorzugten Gegenstand der Erfindung bindet der Binder spezifisch an ein extrazelluläres Krebs-Zielmolekül, welches ausgewählt wird aus der Gruppe bestehend aus aus den Krebs-Zielmolekülen CXCR5 und CD123. In einer bevorzugten Ausführungsform wird der Binder nach Bindung an sein extrazelluläres Zielmolekül auf der Zielzelle durch die Bindung von der Zielzelle internalisiert. Dies bewirkt, dass das Binder-Wirkstoffkonjugat, welches ein Immunokonjugat oder ein ADC sein kann, von der Zielzelle aufgenommen wird. Anschließend wird der Binder vorzugsweise intrazellulär, bevorzugt lysosomal, prozessiert.

In einer Ausführungsform ist der Binder ein Bindeprotein. In einer bevorzugten Ausführungsform ist der Binder ein Antikörper, ein antigen-bindendes Antikörperfragment, ein multispezifischer Antikörper oder ein Antikörpermimetikum.

Bevorzugte Antikörpermimetika sind Affibodies, Adnectins, Anticalins, DARPins, Avimers, oder Nanobodies. Bevorzugte multispezifischer Antikörper sind bispezifische und trispezifische Antikörper.

In einer bevorzugten Ausführungsform ist der Binder ein Antikörper oder ein antigen-bindendes Antikörperfragment, weiter bevorzugt ist ein isolierter Antikörper oder ein isoliertes antigen-bindendes Antikörperfragment.

Bevorzugte antigen-bindende Antikörperfragmente sind Fab, Fab', F(ab')₂ und Fv Fragmente, Diabodies, DAbs, lineare Antikörper und scFv. Besonders bevorzugt sind Fab, Diabodies und scFv.

In einer besonders bevorzugten Ausführungsform ist der Binder ein Antikörper. Besonders bevorzugt sind monoklonale Antikörper oder antigen-bindende Antikörperfragmente davon. Weiter besonders bevorzugt sind humane, humanisierte oder chimäre Antikörper oder antigen-bindende Antikörperfragmente davon.

Antikörper oder antigen-bindende Antikörperfragmente, die Krebs-Zielmoleküle binden, können vom Durchschnittsfachmann mit bekannten Verfahren hergestellt werden, wie z.B. chemische Synthese oder rekombinante Expression. Binder für Krebs-Zielmoleküle können kommerziell erworben werden oder können durch den Durchschnittsfachmann mit bekannten Verfahren hergestellt werden, wie z.B. chemische Synthese oder rekombinante Expression. Weitere Verfahren zur Herstellung von Antikörpern oder antigen-bindenden Antikörperfragmenten sind in WO 2007/070538 beschrieben (siehe Seite 22 "Antibodies"). Der Fachmann kennt Verfahren wie sogenannte Phage-Display Bibliotheken (z.B. Morphosys HuCAL Gold) erstellt und zur Auffindung von Antikörpern oder antigen-bindenden Antikörperfragmenten verwendet werden können (siehe WO 2007/070538, Seite 24 ff und AK-Beispiel 1 auf Seite 70, AK-Beispiel 2 auf Seite 72). Weitere Verfahren zur Herstellung von Antikörper, die DNA Bibliotheken aus B-Zellen verwenden, sind zum Beispiel auf Seite 26 (WO 2007/070538) beschrieben. Verfahren zur Humanisierung von Antikörpern sind auf Seite 30-32 von WO2007070538 und im Detail in Queen, et al.,.Pros. Natl. Acad. Sci. USA 86:10029-10033,1989 oder in WO 90/0786 beschrieben. Des Weiteren sind dem Fachmann Verfahren zur rekombinanten Expression von Proteinen im allgemeinen und im speziellen von Antikörpern bekannt (siehe z.B. in Berger and Kimrnel (Guide to Molecular Cloning Techniques, Methods in Enzymology, Vo1. 152, Academic Press, Inc.); Sambrook, et al., (Molecular Cloning: A Laboratory Manual, (Second Edition, Cold Spring Harbor Laboratory Press; Cold Spring Harbor, N.Y.; 1989) Vol. 1-3); Current Protocols in Molecular Biolony, (F. M. Ausabel et al. [Eds.], Current Protocols, Green Publishing Associates, Inc. / John Wiley & Sons, Inc.); Harlow et al., (Monoclonal Antibodies: A Laboratory Manual, Cold Spring Harbor Laboratory Press (19881, Paul [Ed.]); Fundamental Immunology, (Lippincott Williams & Wilkins (1998)); and Harlow, et al., (Using Antibodies: A Laboratory Manual, Cold Spring Harbor Laboratory Press (1998)). Der Fachmann kennt die entsprechenden Vektoren, Promotoren und Signalpeptide die zur Expression eines Proteins/Antikörpers notwendig sind. Gebräuchliche Verfahren sind auch in WO 2007/070538 auf den Seiten 41 - 45 beschrieben. Verfahren zur Herstellung eines IgG1-Antikörpers sind z.B. in WO 2007/070538 in Beispiel 6 auf Seite 74 ff beschrieben. Verfahren, mit denen die Internalisierung eines Antikörpers nach Bindung an sein Antigen bestimmt werden kann, sind dem Fachmann bekannt und sind z.B. in WO 2007/070538 auf Seite 80 beschrieben. Der Fachmann kann die in WO 2007/070538 beschriebenen Verfahren, die zur Herstellung von Carboanhydrase IX (Mn)-Antikörpern verwendet wurden, anlog zur Herstellung für Antikörper mit anderer Zielmolekülspezifität verwenden.

### Bakterielle Expression

Dem Fachmann ist bekannt, auf welche Weise Antikörper, antigenbindenden Fragmente von diesen, oder Varianten von diesen mit Hilfe bakterieller Expression hergestellt werden können.

Geeignete Expressionsvektoren zur bakteriellen Expression gewünschter Proteine werden durch Insertion einer DNA Sequenz, welche das gewünschte Protein kodiert, im funktionellen Leserahmen zusammen mit geeigneten Translationsinitiations- und Translationsterminationssignalen und mit einem funktionellen Promotor konstruiert. Der Vektor umfasst ein oder mehrere phänotypisch selektierbare Marker und einen Replikationsursprung, um die Erhaltung des Vektors und, falls erwünscht, die Amplifikation desselben innerhalb des Wirtes zu ermöglichen. Geeignete prokaryotische Wirte zur Transformation umfassen, sind aber nicht limitiert auf, *E. coli, Bacillus subtilis, Salmonella typhimurium* und verschiedene Spezies aus dem Genus *Pseudomonas, Streptomyces,* und *Staphylococcus.* Bakterielle Vektoren können zum Beispiel basieren auf Bakteriophagen, Plasmiden, oder Phagemiden. Diese Vektoren können selektierbare Marker und einen bakteriellen Replikationsursprung enthalten, welche aus kommerziell erhältlichen Plasmiden abgeleitet sind. Viele kommerziell erhältlichen Plasmide enthalten typischerweise Elemente des gut bekannten Klonierungsvektors pBR322 (ATCC 37017). In bakteriellen Systemen können eine Anzahl von vorteilhaften Expressionsvektoren auf Basis der beabsichtigten Verwendung des zu exprimierenden Proteins ausgewählt werden.

Nach Transformation eines geeigneten Wirtsstammes und Wachstum des Wirtsstammes zu einer angemessenen Zelldichte wird der ausgewählte Promotor durch geeignete Mittel (z. B. Temperaturveränderung oder chemische Induktion) de-reprimiert / induziert, und die Zellen werden für eine zusätzliche Periode kultiviert. Die Zellen werden üblicherweise durch Zentrifugation geerntet, falls nötig auf physikalische Weise oder mit chemischen Mitteln aufgeschlossen, und der resultierende Rohextrakt wird zur weiteren Reinigung zurückgehalten.

Daher ist eine weitere Ausführungsform der vorliegenden Erfindung ein Expressionsvektor, welcher eine Nukleinsäure umfasst, welche einen neuen Antikörper der vorliegenden Erfindung kodiert.

Antikörper der vorliegenden Erfindung oder antigenbindende Fragmente von diesen beinhalten natürlich gereinigte Produkte, Produkte, die aus chemischen Synthesen stammen, und Produkte, die durch rekombinante Technologien in prokaryotischen Wirten, wie zum Beispiel *E. coli, Bacillus subtilis, Salmonella typhimurium* und verschiedene Spezies aus dem Genus *Pseudomonas, Streptomyces,* und *Staphylococcus,* bevorzugt *E. coli,* produziert werden.

### Säuqerzellexpression

Dem Fachmann ist bekannt, auf welche Weise Antikörper, antigenbindenden Fragmente von diesen, oder Varianten von diesen mit Hilfe von Säugerzellexpression hergestellt werden können.

Bevorzugte regulatorische Sequenzen zur Expression in Säugerzellwirten umfassen virale Elemente, die zu einer hohen Expression in Säugerzellen führen, wie Promotoren und/oder Expressionsverstärker, welche vom Cytomegalovirus (CMV) (wie dem CMV Promotor/Enhancer), Simian Virus 40 (SV40) (wie dem SV40 Promotor/Enhancer), vom Adenovirus, (z.B. der Adenovirus major late promoter (AdMLP)) und vom Polyoma abgeleitet sind. Die Expression der Antikörper kann konstitutiv oder reguliert erfolgen (z.B. induziert durch Zugabe oder Entfernen von Kleinmolekül Induktoren wie Tetracyclin in Kombination mit dem Tet-System).

Zur weiteren Beschreibung viraler regulatorischer Elemente und Sequenzen von diesen sei verwiesen auf z.B. U.S. 5,168,062 von Stinski, U.S. 4,510,245 von Bell et al. und U.S. 4,968,615 von Schaffner et al.. Die rekombinanten Expressionsvektoren können ebenfalls einen Replikationsursprung und selektierbare Marker beinhalten (siehe z.B. U.S. 4,399,216, 4,634,665 und U.S. 5,179,017). Geeignete selektierbare Marker umfassen Gene, die Resistenz gegenüber Substanzen wie G418, Puromycin, Hygromycin, Blasticidin, Zeocin/Bleomycin, oder Methotrexate verleihen, oder selektierbare Marker, welche zur Auxotrophie einer Wirtszelle führen, wie Glutamine Synthetase (Bebbington et al., Biotechnology (N Y). 1992 Feb;10(2):169-75), wenn der Vektor in die Zelle eingebracht wurde.

Zum Beispiel vermittelt das Dihydrofolate-Reductase (DHFR) Gen Resistenz gegenüber Methotrexate, das neo Gen vermittelt Resistenz gegenüber G418, das bsd Gen aus *Aspergillus terreus* vermittelt Resistenz gegenüber Blasticidin, Puromycin N-acetyl-transferase vermittelt Resistenz gegenüber Puromycin, das Sh ble Genprodukt vermittelt Resistenz gegenüber Zeocin, und Resistenz gegenüber Hygromycin wird vermittelt durch das *E. coli* Hygromycin-Resistenzgen (hyg or hph). Selektierbare Marker wie DHFR oder Glutamine-Synthetase sind auch hilfreich für Amplifikationstechniken in Verbindung mit MTX und MSX.

Die Transfektion eines Expressionsvektors in eine Wirtszelle kann mit Hilfe von Standardtechniken ausgeführt werden, unter anderem mit Elektroporation, Nucleofection, Calcium-Phosphate-Präzipitation, Lipofection, Polykation-basierter Transfektion wie Polyethlylenimine (PEI)-basierter Transfektion und DEAE-Dextran Transfection.

Geeignete Säugerwirtszellen für die Expression von Antikörpern, antigenbindenden Fragmenten von diesen, oder Varianten von diesen umfassen Chinese Hamster Ovary (CHO Zellen) wie CHO-K1, CHO-S, CHO-K1SV [inbegriffen DHFR-CHO Zellen, beschrieben in Urlaub and Chasin, (1980) Proc. Natl. Acad. Sci. USA 77:4216-4220 und Urlaub et al., Cell. 1983 Jun;33(2):405-12, verwendet mit einem DHFR selektierbaren Marker, wie beschrieben in R. J. Kaufman and P. A. Sharp (1982) Mol. Biol. 159:601-621, sowie andere Knockout-Zellen, wie ausgeführt in Fan et al., Biotechnol Bioeng. 2012 Apr;109(4):1007-15), NS0 myeloma Zellen, COS Zellen, HEK293 Zellen, HKB11 Zellen, BHK21 Zellen, CAP Zellen, EB66 Zellen, und SP2 Zellen.

Die Expression von Antikörpern, antigenbindenden Fragmenten von diesen, oder Varianten von diesen kann auch transient oder semi-stabil in Expressionssystemen erfolgen, wie HEK293, HEK293T, HEK293-EBNA, HEK293E, HEK293-6E, HEK293-Freestyle, HKB11, Expi293F, 293EBNALT75, CHO Freestyle, CHO-S, CHO-K1, CHO-K1SV, CHOEBNALT85, CHOS-XE, CHO-3E7 oder CAP-T Zellen (beispielsweise wie Durocher et al., Nucleic Acids Res. 2002 Jan 15;30(2):E9)

In einigen Ausführungsformen ist der Expressionsvektor in jener Weise konstruiert, dass das zu exprimierende Protein in das Zellkulturmedium, in welchem die Wirtszellen wachsen, sekretiert wird. Die Antikörper, die antigenbindenden Fragmente von diesen, oder die Varianten von diesen können aus dem Zellkulturmedium mit Hilfe dem Fachmann bekannten Proteinreinigungsmethoden gewonnen werden.

### Reinigung

Die Antikörper, die antigenbindenden Fragmente von diesen, oder die Varianten von diesen können aus rekombinanten Zellkulturen mit Hilfe gut bekannter Methoden gewonnen und gereinigt werden, umfassend beispielsweise Ammoniumsulfat- oder Ethanol- Präzipitation, Säureextraktion, Protein A Chromatographie, Protein G Chromatographie, Anion- oder Kationenaustauschchromatographie, Phospho-Cellulose Chromatographie, hydrophobe Interaktionschromatographie (HIC), Affinitätschromatographie, Hydroxylapatite Chromatographie and Lectin Chromatographie. High Performance Flüssigchromatographie ("HPLC") kann ebenfalls zur Reinigung angewendet werden. Siehe, beispielsweise, Colligan, Current Protocols in Immunology, or Current Protocols in Protein Science, John Wiley & Sons, NY, N.Y., (1997-2001), e.g., Chapters 1, 4, 6, 8, 9, 10.

Antikörper der vorliegenden Erfindung oder antigenbindenden Fragmente von diesen, oder die Varianten von diesen umfassen natürlich gereinigte Produkte, Produkte aus chemischen Syntheseverfahren und Produkte, welche mit Hilfe rekombinanter Techniken in prokaryotischen oder eukaryotischen Wirtszellen hergestellt werden. Eukaryotische Wirte umfassen beispielsweise Hefezellen, höhere Pflanzenzellen, Insektenzellen und Säugerzellen. Abhängig von der für die rekombinanten Expression gewählten Wirtszelle kann das exprimierte Protein glykosyliert oder nicht-glykosyliert vorliegen.

In einer bevorzugen Ausführungsform wird der Antikörper gereinigt (1) zu mehr als 95 Gew.-% gemessen beispielsweise mit der Lowry-Methode, mit UV-Vis Spektroskopie oder mit der SDS-Kapillargelelektrophorese (zum Beispiel mit einem Caliper LabChip GXII, GX 90 oder Biorad Bioanalyzer Gerät), und in mehr bevorzugten Ausführungsformen mehr als 99 Gew.-%, (2) zu einem Grade geeignet zur Bestimmung von mindestens 15 Resten der N-terminalen oder internen Aminosäuresequenz, oder (3) zur Homogenität bestimmt durch SDS-PAGE unter reduzierenden oder nicht-reduzierenden Bedingungen mit Hilfe von Coomassie-Blau oder bevorzugt Silber-Färbung.

Gewöhnlich wird ein isolierter Antikörper mit Hilfe wenigstens eines Proteinreinigungsschrittes gewonnen.

### anti-CD 123 Antikörper

Erfindungsgemäß können anti-CD123 Antikörper verwendet werden.

Der Begriff "anti-CD123 Antikörper" oder "ein Antikörper, der spezifisch an CD123 bindet" bezieht sich auf einen Antikörper, der das Krebs Zielmolekül CD123 (NCBI -Referenzsequenz: NP_002174.1; SEQ ID NO: 62) bindet, bevorzugt mit einer für diagnostischen und/oder therapeutischen Anwendung genügenden Affinität. In bestimmten Ausführungsformen bindet der Antikörper CD123 mit einer Dissotiationskonstante (K_{D}) von ≤ 1µM, ≤ 100 nM, ≤ 10 nM, ≤ 1 nM, ≤ 0.1 nM, ≤ 0.01 nM, oder ≤ 0.001 nM.

Die Generierung und Eigenschaften des monoklonalen Antikörpers 7G3, welcher die N-terminale Domäne von IL-3Rα, CD123, bindet, werden von Sun et al. beschrieben (Sun et al., 1996, Blood 87(1):83-92). US Patent Nummer 6,177,078 (Lopez) bezieht sich auf den anti-CD123-Antikörper 7G3. Eine chimäre Variante dieses Antikörpers (CSL360) ist in WO 2009/070844 sowie eine humanisierte Version (CSL362) in WO 2012/021934 beschrieben. Die Sequenz des 7G3 Antikörpers ist in EP2426148 offenbart worden. Diese Sequenz stellt den Ausgangspunkt der humanisierten Antikörper dar, die durch CDR-Umsetzung ("CDR grafting") erhalten wurden

Einen Antikörper, der besonders gut nach Zelloberflächenantigenbindung internalisiert, ist der anti-CD123 Antikörper 12F1, der von Kuo et al. offenbart wurde (Kuo et a., 2009, Bioconjug Chem. 20(10):1975-82). Der Antikörper 12F1 bindet mit einer höheren Affinität an CD123 als der Antikörper 7G3 und internalisiert nach Zelloberflächenantigenbindung deutlich schneller als 7G3. Bispezifische scFv Immunofusionsproteine basierend auf 12F1 werden in WO 2013/173820 offenbart. Antikörper TPP-6013 ist eine chimäre Variante des 12F1.

Die Erfindung betrifft insbesondere Konjugate mit Antikörpern oder antigenbindende Antikörperfragmente davon oder Varianten davon, die auf den aus der Maus stammenden Antikörper 7G3 (Sun et al., 1996, Blood 87(1):83-92) und 12F1 (Kuo et a., 2009, Bioconjug Chem. 20(10):1975-82) zurückgehen, oder Konjugate mit Antikörpern oder antigenbindende Antikörperfragmente davon oder Varianten davon, die auf den aus der Maus stammenden Antikörper 12F1 (Kuo et a., 2009, Bioconjug Chem. 20(10):1975-82) zurückgehen.

Besonders bevorzugt sind im Rahmen dieser Erfindung die anti-CD123 Antikörper TPP-9476, TPP-8988, und TPP-8987.

### anti-CXCR5 Antikörper

Erfindungsgemäß können anti-CXCR5 Antikörper verwendet werden.

Der Begriff "anti-CXCR5 Antikörper" oder "ein Antikörper, der spezifisch an CXCR5 bindet" bezieht sich auf einen Antikörper, der das Krebs Zielmolekül CXCR5 (NCBI - Referenzsequenz: NP_001707.1; SEQ ID NO: 61) bindet, bevorzugt mit einer für diagnostischen und/oder therapeutischen Anwendung genügenden Affinität. In bestimmten Ausführungsformen bindet der Antikörper CXCR5 mit einer Dissotiationskonstante (K_{D}) von ≤ 1µM, ≤ 100 nM, ≤ 10 nM, ≤ 1 nM, ≤ 0.1 nM, ≤ 0.01 nM, oder ≤ 0.001 nM.

Beispiele für Antikörper,und antigen-bindene Fragmente die an CXCR5 binden, sind dem Fachmann bekannt und z.B in EP2195023 beschrieben.

Die Hybridomzellen für den Rattenantikörpers RF8B2 (ACC2153) wurden von DSMZ bezogen und die Sequenz des Antikörpers nach Standardmethoden identifiziert. TPP-9024 eine chimäre Variante dieses Antikörpers mit einer Punktmutation an Position 67 (S67F) wurde hergestellt. Weiterhin stellte die Rattenantikörper-Sequenz den Ausgangspunkt der humanisierten Antikörper dar, die durch CDR-Umsetzung ("CDR grafting") in humanes framework erhalten wurden.

Diese Antikörper und antigenbindende Fragmente können im Rahmen dieser Erfindung verwendet werden.

Besonders bevorzugt sind im Rahmen dieser Erfindung die humanisierten anti-CXCR5 Antikörper TPP-9574, TPP-9580 und der chimäre Antikörper TPP-9024.

### Bevorzugte Antikörper und Antigen-bindende Antikörperfragmente für erfindungsgemäße Binder-Wirkstoff-Konjugate

In dieser Anmeldung wird bei den Binder-Wirkstoff-Konjugaten auf die folgenden bevorzugten Antikörper Bezug genommen, wie in der nachstehenden Tabelle A gezeigt: anti-CD123 Antikörper TPP-8987, TPP-8988 und TPP-9476 sowie die anti-CXCR5 Antikörper TPP-9024, TPP-9574 und TPP-9580.

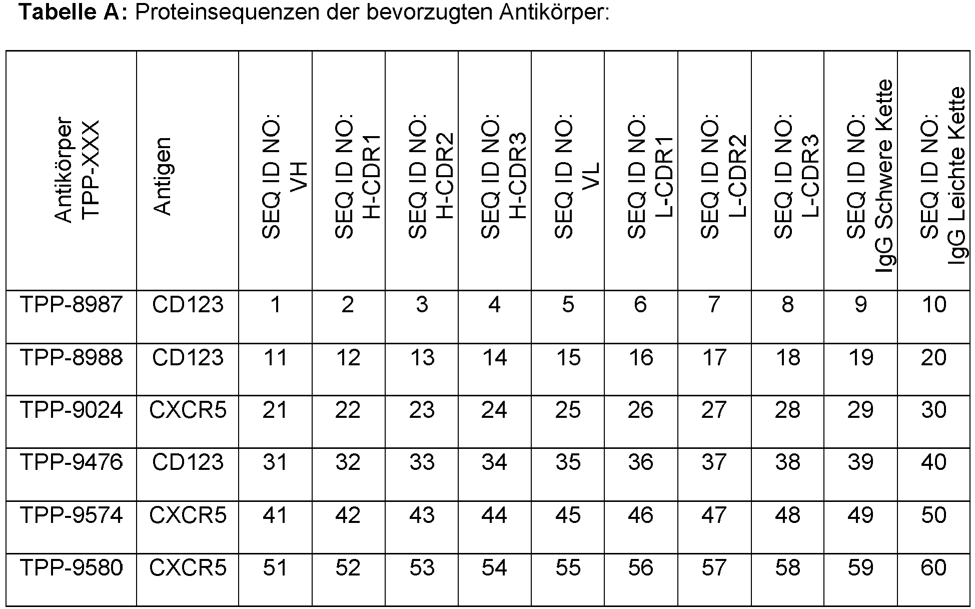

TPP-8987, TPP-8988, TPP-9476, TPP-9024, TPP-9574 und TPP-9580 sind Antikörper umfassend eine oder mehrere der in obiger Tabelle angegebenen CDR-Sequenzen (H-CDR1, H-CDR2, H-CDR3, L-CDR1, L-CDR2, L-CDR3) der variable Region der schwere Kette (VH) oder der variable Region der leichte Kette (VL). Vorzugsweise umfassen die Antikörper die angegebene variable Region der schwere Kette (VH) und/oder die variable Region der leichte Kette (VL). Vorzugsweise umfassen die Antikörper die angegebene Region der schweren Kette (IgG Schwere Kette) und/oder die angegebene Region der leichten Kette (IgG Leichte Kette).

TPP-8987 ist ein anti-CD123 Antikörper umfassend eine variable Region der schwere Kette (VH) umfassend die variable CDR1-Sequenz der schweren Kette (H-CDR1), wie dargestellt durch SEQ ID NO: 2, die variable CDR2-Sequenz der schweren Kette (H-CDR2), wie dargestellt durch SEQ ID NO: 3 und die variable CDR3-Sequenz der schweren Kette (H-CDR3), wie dargestellt durch SEQ ID NO: 4, sowie eine variable Region der leichte Kette (VL) umfassend die variable CDR1-Sequenz der leichten Kette (L-CDR1), wie dargestellt durch SEQ ID NO: 6, die variable CDR2-Sequenz der leichten Kette (L-CDR2), wie dargestellt durch SEQ ID NO: 7 und die variable CDR3-Sequenz der leichten Kette (L-CDR3), wie dargestellt durch SEQ ID NO: 8.

TPP-8988 ist ein anti-CD123 Antikörper umfassend eine variable Region der schwere Kette (VH) umfassend die variable CDR1-Sequenz der schweren Kette (H-CDR1), wie dargestellt durch SEQ ID NO: 12, die variable CDR2-Sequenz der schweren Kette (H-CDR2), wie dargestellt durch SEQ ID NO: 13 und die variable CDR3-Sequenz der schweren Kette (H-CDR3), wie dargestellt durch SEQ ID NO: 14, sowie eine variable Region der leichte Kette (VL) umfassend die variable CDR1-Sequenz der leichten Kette (L-CDR1), wie dargestellt durch SEQ ID NO: 16, die variable CDR2-Sequenz der leichten Kette (L-CDR2), wie dargestellt durch SEQ ID NO: 17 und die variable CDR3-Sequenz der leichten Kette (L-CDR3), wie dargestellt durch SEQ ID NO: 18.

TPP-9024 ist ein anti-CXCR5 Antikörper umfassend eine variable Region der schwere Kette (VH) umfassend die variable CDR1-Sequenz der schweren Kette (H-CDR1), wie dargestellt durch SEQ ID NO: 22, die variable CDR2-Sequenz der schweren Kette (H-CDR2), wie dargestellt durch SEQ ID NO: 23 und die variable CDR3-Sequenz der schweren Kette (H-CDR3), wie dargestellt durch SEQ ID NO: 24, sowie eine variable Region der leichte Kette (VL) umfassend die variable CDR1-Sequenz der leichten Kette (L-CDR1), wie dargestellt durch SEQ ID NO: 26, die variable CDR2-Sequenz der leichten Kette (L-CDR2), wie dargestellt durch SEQ ID NO: 27 und die variable CDR3-Sequenz der leichten Kette (L-CDR3), wie dargestellt durch SEQ ID NO: 28.

TPP-9476 ist ein anti-CD123 Antikörper umfassend eine variable Region der schwere Kette (VH) umfassend die variable CDR1-Sequenz der schweren Kette (H-CDR1), wie dargestellt durch SEQ ID NO: 32, die variable CDR2-Sequenz der schweren Kette (H-CDR2), wie dargestellt durch SEQ ID NO: 33 und die variable CDR3-Sequenz der schweren Kette (H-CDR3), wie dargestellt durch SEQ ID NO: 34, sowie eine variable Region der leichte Kette (VL) umfassend die variable CDR1-Sequenz der leichten Kette (L-CDR1), wie dargestellt durch SEQ ID NO: 36, die variable CDR2-Sequenz der leichten Kette (L-CDR2), wie dargestellt durch SEQ ID NO: 37 und die variable CDR3-Sequenz der leichten Kette (L-CDR3), wie dargestellt durch SEQ ID NO: 38.

TPP-9574 ist ein anti-CXCR5 Antikörper umfassend eine variable Region der schwere Kette (VH) umfassend die variable CDR1-Sequenz der schweren Kette (H-CDR1), wie dargestellt durch SEQ ID NO: 42, die variable CDR2-Sequenz der schweren Kette (H-CDR2), wie dargestellt durch SEQ ID NO: 43 und die variable CDR3-Sequenz der schweren Kette (H-CDR3), wie dargestellt durch SEQ ID NO: 44, sowie eine variable Region der leichte Kette (VL) umfassend die variable CDR1-Sequenz der leichten Kette (L-CDR1), wie dargestellt durch SEQ ID NO: 46, die variable CDR2-Sequenz der leichten Kette (L-CDR2), wie dargestellt durch SEQ ID NO: 47 und die variable CDR3-Sequenz der leichten Kette (L-CDR3), wie dargestellt durch SEQ ID NO: 48.

TPP-9580 ist ein anti-CXCR5 Antikörper umfassend eine variable Region der schwere Kette (VH) umfassend die variable CDR1-Sequenz der schweren Kette (H-CDR1), wie dargestellt durch SEQ ID NO: 52, die variable CDR2-Sequenz der schweren Kette (H-CDR2), wie dargestellt durch SEQ ID NO: 53 und die variable CDR3-Sequenz der schweren Kette (H-CDR3), wie dargestellt durch SEQ ID NO: 54, sowie eine variable Region der leichte Kette (VL) umfassend die variable CDR1-Sequenz der leichten Kette (L-CDR1), wie dargestellt durch SEQ ID NO: 56, die variable CDR2-Sequenz der leichten Kette (L-CDR2), wie dargestellt durch SEQ ID NO: 57 und die variable CDR3-Sequenz der leichten Kette (L-CDR3), wie dargestellt durch SEQ ID NO: 58.

TPP-8987 ist ein anti-CD123 Antikörper umfassend vorzugsweise eine variable Region der schweren Kette (VH) wie dargestellt durch SEQ ID NO: 1 sowie eine variable Region der leichten Kette (VL) wie dargestellt durch SEQ ID NO: 5.

TPP-8988 ist ein anti-CD123 Antikörper umfassend vorzugsweise eine variable Region der schweren Kette (VH) wie dargestellt durch SEQ ID NO: 11 sowie eine variable Region der leichten Kette (VL) wie dargestellt durch SEQ ID NO: 15.

TPP-9024 ist ein anti-CXCR5 Antikörper umfassend vorzugsweise eine variable Region der schweren Kette (VH) wie dargestellt durch SEQ ID NO: 21 sowie eine variable Region der leichten Kette (VL) wie dargestellt durch SEQ ID NO: 25.

TPP-9476 ist ein anti-CD123 Antikörper umfassend vorzugsweise eine variable Region der schweren Kette (VH) wie dargestellt durch SEQ ID NO: 31 sowie eine variable Region der leichten Kette (VL) wie dargestellt durch SEQ ID NO: 35.

TPP-9574 ist ein anti-CXCR5 Antikörper umfassend vorzugsweise eine variable Region der schweren Kette (VH) wie dargestellt durch SEQ ID NO: 41 sowie eine variable Region der leichten Kette (VL) wie dargestellt durch SEQ ID NO: 45.

TPP-9580 ist ein anti-CXCR5 Antikörper umfassend vorzugsweise eine variable Region der schweren Kette (VH) wie dargestellt durch SEQ ID NO: 51 sowie eine variable Region der leichten Kette (VL) wie dargestellt durch SEQ ID NO: 55.

TPP-8987 ist ein anti-CD123 Antikörper umfassend vorzugsweise eine Region der schweren Kette wie dargestellt durch SEQ ID NO: 9 sowie eine Region der leichten Kette wie dargestellt durch SEQ ID NO: 10.

TPP-8988 ist ein anti-CD123 Antikörper umfassend vorzugsweise eine Region der schweren Kette wie dargestellt durch SEQ ID NO: 19 sowie eine Region der leichten Kette wie dargestellt durch SEQ ID NO: 20.

TPP-9024 ist ein anti-CXCR5 Antikörper umfassend vorzugsweise eine Region der schweren Kette wie dargestellt durch SEQ ID NO: 29 sowie eine Region der leichten Kette wie dargestellt durch SEQ ID NO: 30.

TPP-9476 ist ein anti-CD123 Antikörper umfassend vorzugsweise eine Region der schweren Kette wie dargestellt durch SEQ ID NO: 39 sowie eine Region der leichten Kette wie dargestellt durch SEQ ID NO: 40.

TPP-9574 ist ein anti-CXCR5 Antikörper umfassend vorzugsweise eine Region der schweren Kette wie dargestellt durch SEQ ID NO: 49 sowie eine Region der leichten Kette wie dargestellt durch SEQ ID NO: 50.

TPP-9580 ist ein anti-CXCR5 Antikörper umfassend vorzugsweise eine Region der schweren Kette wie dargestellt durch SEQ ID NO: 59 sowie eine Region der leichten Kette wie dargestellt durch SEQ ID NO: 60.

### Therapeutische Verwendung

Zu den hyperproliferativen Erkrankungen, zu deren Behandlung die erfindungsgemäßen Verbindungen eingesetzt werden können, zählt insbesondere die Gruppe der Krebs- und Tumorerkrankungen. Hierunter werden im Rahmen der vorliegenden Erfindung insbesondere die folgenden Erkrankungen verstanden, ohne jedoch auf sie beschränkt zu sein: Brustkarzinome und Brusttumore (Mammakarzinome einschließlich ductaler und lobulärer Formen, auch *in situ),* Atemwegstumore (kleinzelliges und nicht-kleinzelliges Karzinom, Bronchialkarzinome), Hirntumore (z.B. des Hirnstamms und des Hypothalamus, Astrocytoma, Ependymoma, Glioblastoma, Gliome, Medulloblastoma, Meningiome sowie neuro-ektodermale und pineale Tumore), Tumore der Verdauungsorgane (Speiseröhren-, Magen-, Gallenblasen-, Dünndarm-, Dickdarm-, Rektum- und Analkarzinome), Lebertumore (u.a. hepatozelluläres Karzinom, Cholangiokarzinom und gemischt-hepatozelluläres Cholangiokarzinom), Tumore des Kopf- und Halsbereiches (Larynx-, Hypopharynx-, Nasopharynx-, Oropharynx-, Lippen- und Mundhöhlenkarzinome, orale Melanome), Hauttumore (Basaliome, Spinaliome, Plattenepithelkarzinome, Kaposi-Sarkom, maligne Melanome, nicht-melanomartiger Hautkrebs, Merkelzell-Hautkrebs, Mastzelltumore), Tumore des Stütz- und Bindegewebes (u.a. Weichteilsarkome, Osteosarkome, maligne fibröse Histiozytome, Chondrosarkome, Fibrosarkome, Hämangiosarkome, Leiomyosarkome, Liposarkome, Lymphosarkome und Rhabdomyosarkome), Tumore der Augen (u.a. intraokuläres Melanom und Retinoblastom), Tumore der endokrinen und exokrinen Drüsen (z.B. der thyroiden und parathyroiden Drüsen, Bauchspeicheldrüsen- und Speicheldrüsenkarzinome, Adenokarzinome), Tumore des Harntrakts (Blasen-, Penis-, Nieren-, Nierenbecken- und Harnleitertumore) sowie Tumore der reproduktiven Organe (Endometrium-, Zervix-, Ovarial-, Vaginal-, Vulva- und Uteruskarzinome der Frau sowie Prostata- und Hodenkarzinome des Mannes). Dazu gehören auch proliferative Erkrankungen des Blutes, des Lymphsystems und des Rückenmarks, in solider Form und als zirkulierende Zellen, wie Leukämien, Lymphome und myeloproliferative Erkrankungen, z.B. akute myeloide, akute lymphoblastische, chronisch-lymphozytische, chronisch-myelogene und Haarzell-Leukämie, sowie AIDS-korrelierte Lymphome, Hodgkin-Lymphome, Non-Hodgkin-Lymphome, kutane T-Zell-Lymphome, Burkitt-Lymphome und Lymphome im zentralen Nervensystem.

Diese gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Die hier beschriebenen gegen CD123 gerichteten Antikörper-Wirkstoff-Konjugate (ADCs) können verwendet werden, um CD123 exprimierende Störungen, wie CD123 exprimierende Krebserkrankungen, zu behandeln. Typischerweise zeigen derartige Krebszellen messbare Mengen von CD123 gemessen auf Protein- (z.B. mittels Immunoassay) oder RNA-Ebene. Einige dieser Krebsgewebe zeigen einen erhöhten Spiegel an CD123 verglichen mit nichtkanzerogenem Gewebe des gleichen Typs, bevorzugt gemessen am gleichen Patienten. Optional wird der Gehalt an CD123 gemessen, bevor die Krebsbehandlung mit einem erfindungsgemäßen Antikörper-Wirkstoff-Konjugat (ADCs) begonnen wird (Patienten-Stratifizierung). Die CD123 gerichteten Antikörper-Wirkstoff-Konjugate (ADCs) können verwendet werden, um CD123 exprimierende Störungen, wie CD123 exprimierende Krebserkrankungen zu behandeln, wie Tumore des hämatopoetischen und lymphatischen Gewebes oder hämatopoetische und lymphatische bösartige Tumore. Beispiele für Krebserkrankungen, die mit einer CD123-Expression verbunden sind, beinhalten myeloische Krankheiten, wie akute myeloische Leukämie (AML) und myelodysplastisches Syndrom (MDS). Weitere Krebsarten beinhalten B-Zell akute lymphoblastische Leukämie (B-ALL), Haarzellleukämie, Blastic Plasmacytoid Dendritic Cell Neoplasm (BPDCN), Hodgkin-Lymphom, immature T-Zell akute lymphoblastische Leukämie (Immature T-ALL), Burkitt-Lymphom, follikuläres Lymphom, chronische lymphatische Leukämie (CLL), Mantelzelllymphom (MCL). Methoden der beschriebenen Erfindung umfassen die Behandlung von Patienten mit einem CD123 exprimierenden Krebs, wobei die Methode die Gabe eines erfinderischen Antikörper-Wirkstoff-Konjugates (ADCs)umfasst.

Die Behandlung der zuvor genannten Krebserkrankungen mittels der erfindungsgemäßen Verbindungen umfasst sowohl eine Behandlung der soliden Tumore als auch eine Behandlung metastasierter oder zirkulierender Formen hiervon.

Der Begriff "Behandlung" oder "behandeln" wird im Rahmen dieser Erfindung konventionell verwendet und bedeutet die Versorgung, Pflege und Betreuung eines Patienten mit dem Ziel, eine Krankheit oder gesundheitliche Abweichung zu bekämpfen, zu verringern, abzuschwächen oder zu erleichtern und die Lebensbedingungen zu verbessern, die durch diese Krankheit beeinträchtigt werden, wie beispielsweise bei einer Krebserkrankung.

Weiterer Gegenstand der vorliegenden Erfindung ist somit die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Beispielsweise können die Verbindungen der vorliegenden Erfindung mit bekannten antihyperproliferativen, zytostatischen, zytotoxischen oder immuntherapeutischen Substanzen zur Behandlung von Krebserkrankungen kombiniert werden. Als geeignete Kombinationswirkstoffe seien beispielhaft genannt:
1311-chTNT, Abarelix, Abirateron, Aclarubicin, Adalimumab, Ado-Trastuzumab Emtansin, Afatinib, Aflibercept, Aldesleukin, Alemtuzumab, Alendronsäure, Alitretinoin, Altretamin, Amifostine, Aminoglutethimid, Hexyl-5-Aminolevulinat, Amrubicin, Amsacrin, Anastrozol, Ancestim, Anethole dithiolethione, Anetumab Ravtansine, Angiotensin II, Antithrombin III, Aprepitant, Arcitumomab, Arglabin, Arsentrioxid, Asparaginase, Atezolizumab, Avelumab, Axitinib, Azacitidin, Belotecan, Bendamustin, Besilesomab, Belinostat, Bevacizumab, Bexaroten, Bicalutamid, Bisantren, Bleomycin, Blinatumomab, Bortezomib, Buserelin, Bosutinib, Brentuximab Vedotin, Busulfan, Cabazitaxel, Cabozantinib, Calcitonin, Calciumfolinat, Calciumlevofolinat, Capecitabin, Capromab, Carbamazepine, Carboplatin, Carboquon, Carfilzomib, Carmofur, Carmustin, Catumaxomab, Celecoxib, Celmoleukin, Ceritinib, Cetuximab, Chlorambucil, Chlormadinon, Chlormethin, Cidofovir, Cinacalcet, Cisplatin, Cladribin, Clodronsäure, Clofarabin, Cobimetinib, Copanlisib, Crisantaspase, Crizotinib, Cyclophosphamid, Cyproteron, Cytarabin, Dacarbazin, Dactinomycin, Daratumumab, Dabrafenib, Darolutamide, Dasatinib, Daunorubicin, Decitabin, Degarelix, Denileukin-Diftitox, Denosumab, Depreotid, Deslorelin, Dexrazoxane, Dibrospidiumchlorid, Dianhydrogalactitol, Diclofenac, Docetaxel, Dolasetron, Doxifluridin, Doxorubicin, Doxorubicin + Estron, Dronabinol, Durvalumab, Edrecolomab, Elliptiniumacetat, Endostatin, Enocitabin, Enzalutamid, Epacadostat, Epirubicin, Epitiostanol, Epoetin-alfa, Epoetin-beta, Epoetin-zeta, Eptaplatin, Eribulin, Erlotinib, Esomeprazol, Estramustin, Etoposid, Ethinylestradiol, Everolimus, Exemestan, Fadrozol, Fentanyl, Fluoxymesteron, Floxuridin, Fludarabin, Fluoruracil, Flutamid, Folinsäure, Formestan, Fosaprepitant, Fotemustin, Fulvestrant, Gadobutrol, Gadoteridol, Gadotersäure-Megluminsalz, Gadoversetamid, Gadoxetsäure Dinatriumsalz (Gd-EOB-DTPA Dinatriumsalz), Galliumnitrat, Ganirelix, Gefitinib, Gemcitabin, Gemtuzumab, Glucarpidase, Glutoxim, Goserelin, Granisetron, Granulocyten Kolonie stimulierender Factor (G-CSF), Granulocyten Macrophagen Kolonie stimulierender Factor (GM-CSF), Histamindihydrochlorid, Histrelin, Hydroxycarbamid, I-125-Seeds, Ibandronsäure, Ibritumomab-Tiuxetan, Ibrutinib, Idarubicin, Ifosfamid, Imatinib, Imiquimod, Improsulfan, Indisetron, Incadronic acid, Ingenolmebutat, Interferon-alfa, Interferon-beta, Interferon-gamma, lobitridol, lobenguane (123l), lomeprol, Ipilimumab, Irinotecan, Itraconazole, Ixabepilon, Ixazomib,Lanreotid, Lansoprazole, Lansoprazol, Lapatinib, Lasocholine, Lenalidomid, Lenvatinib, Lenograstim, Lentinan, Letrozol, Leuprorelin, Levamisol, Levonorgestrel, Levothyroxin-Natrium, Lipegfilgrastim, Lisurid, Lobaplatin, Lomustin, Lonidamin, Masoprocol, Medroxyprogesteron, Megestrol, Melarsoprol, Melphalan, Mepitiostan, Mercaptopurin, Mesna, Methadon, Methotrexat, Methoxsalen, Methylaminolevulinat, Methylprednisolon, Methyltestosteron, Metirosin, Mifamurtid, Miltefosin, Miriplatin, Mitobronitol, Mitoguazon, Mitolactol, Mitomycin, Mitotan, Mitoxantron, Mogamulizumab, Molgramostim, Mopidamol, Morphinhydrochlorid, Morphinsulfat, Nabilon, Nabiximols, Nafarelin, Naloxon + Pentazocin, Naltrexon, Nartograstim, Necitumumab, Nedaplatin, Nelarabin, Neridronsäure, Netupitant/palonosetron, Nivolumab, Nivolumabpentetreotid, Nilotinib, Nilutamid, Nimorazol, Nimotuzumab, Nimustin, Nintedanib, Nitracrin, Nivolumab, Obinutuzumab, Octreotid, Ofatumumab, Olaparib, Olaratumab, Omacetaxin-Mepesuccinat, Omeprazol, Ondansetron, Orgotein, Orilotimod, Osimertinib, Oxaliplatin, Oxycodon, Oxymetholon, Ozogamicin, p53-Gentherapie, Paclitaxel, Palbociclib, Palifermin, Palladium-103-Seed, Palonosetron, Pamidronsäure, Panitumumab, Panobinostat, Pantoprazol, Pazopanib, Pegaspargase, Pembrolizumab, Peg-interferon-alfa-2b, Pembrolizumab, Pemetrexed, Pentostatin, Peplomycin, Perflubutane, Perfosfamid, Pertuzumab, Picibanil, Pilocarpin, Pirarubicin, Pixantron, Plerixafor, Plicamycin, Poliglusam, Polyestradiolphosphat, Polyvinylpyrrolidone + Natriumhyaluronat, Polysaccharid-K, Pomalidomid, Ponatinib, Porfimer-Natrium, Pralatrexat, Prednimustin, Prednison, Procarbazin, Procodazole, Propranolol, Quinagolid, Rabeprazol, Racotumomab, Radium-223-chlorid, Radotinib, Raloxifen, Raltitrexed, Ramosetron, Ramucirumab, Ranimustin, Rasburicase, Razoxan, Refametinib, Regorafenib, Risedronsäure, Rhenium-186 Etidronat, Rituximab, Rogaratinib, Rolapitant, Romidepsin, Romurtid, Roniciclib , Samarium (153Sm) lexidronam, Satumomab, Secretin, Siltuximab, Sipuleucel-T, Sizofiran, Sobuzoxan, Natriumglycididazol, Sonidegib, Sorafenib, Stanozolol, Streptozocin, Sunitinib, Talaporfin, Talimogen Laherparepvec, Tamibaroten, Tamoxifen, Tapentadol, Tasonermin, Teceleukin, Technetium (99mTc) Nofetumomab Merpentan, 99mTc-HYNIC-[Tyr3]-octreotid, Tegafur, Tegafur + Gimeracil + Oteracil, Temoporfin, Temozolomid, Temsirolimus, Teniposid, Testosteron, Tetrofosmin, Thalidomid, Thiotepa, Thymalfasin, Thyrotropin alfa, Tioguanin, Tocilizumab, Topotecan, Toremifen, Tositumomab, Trabectedin, Trametinib, Tramadol, Trastuzumab, Treosulfan, Tretinoin, Trifluridine + Tipiracil, Trametinib, Trilostan, Triptorelin, Trofosfamid, Thrombopoietin, Ubenimex, Valrubicin, Vandetanib, Vapreotid, Valatinib , Vemurafenib, Vinblastin, Vincristin, Vindesin, Vinflunin, Vinorelbin, Vismodegib, Vorinostat, Yttrium-90-Glasmikrokugeln, Zinostatin, Zinostatin-Stimalamer, Zoledronsäure, Zorubicin. Darüber hinaus können die Verbindungen der vorliegenden Erfindung beispielsweise mit Bindern (z.B. Antikörpern) kombiniert werden, die beispielhaft an die folgenden Targets binden können: OX-40, CD137/4-1BB, DR3, IDO1/IDO2, LAG-3, CD40.

Da ein nicht Zell-permeabler Toxophormetabolit eines Antikörper-Wirkstoff-Konjugates (ADCs) keine schädigende Wirkung auf die Zellen des adaptiven Immunsystems haben sollte, ist die Kombination eines erfindungsgemäßen Antikörper-Wirkstoff-Konjugates (ADCs) mit einer Krebsimmuntherapie zur Verwendung in der Behandlung von Krebs oder Tumoren ein weiterer Gegenstand dieser Erfindung. Der intrinsische Wirkmechanismus von zytotoxischen Antikörper-Wirkstoffkonjugaten beinhaltet die direkte Auslösung von Zelltod der Tumorzellen und somit die Freisetzung von Tumorantigenen, die eine Immunantwort stimulieren können. Zudem gibt es Hinweise, dass die KSP-Inhibitor-Toxophorklasse Marker des sogenannten immunogenen Zelltod [immunogenic cell death (ICD)] *in vitro* induziert. Somit stellt die Kombination der Antikörper-Wirkstoff-Konjugate (ADCs) der vorliegenden Erfindung mit einem oder mehreren therapeutischer Ansätzen zur Krebs-Immuntherapie oder mit einem oder mehreren Wirkstoffen, bevorzugt Antikörpern, gerichtet gegen ein molekulares Target aus der Krebs-Immuntherapie eine bevorzugte Methode zur Behandlung von Krebs oder Tumoren da. i) Beispiele therapeutischer Ansätze zur Krebs-Immuntherapie umfassen Immun-modulatorische monoklonale Antikörper und niedermolekulare Substanzen gerichtet gegen Targets aus der Krebs Immuntherapie, Vakzine, CAR T Zellen, bi-spezifische T Zellrekrutierende Antikörper, onkolytische Viren, zellbasierte Vakzinierungsansätze. ii) Beispiele ausgewählter Targets aus der Krebs Immuntherapie geeignet für Immun-modulatorische monoklonale Antikörper umfassen CTLA-4, PD-1/PDL-1, OX-40, CD137, DR3, IDO1, IDO2,TDO2, LAG-3, TIM-3 CD40.,ICOS / ICOSL,TIGIT; GITR/GITRL, VISTA, CD70, CD27, HVEM/BTLA, CEACAM1, CEACAM6, ILDR2, CD73, CD47, B7H3, TLR's. Die Kombination eines erfindungsgemäßen Antikörper-Wirkstoff-Konjugates (ADCs) mit eine Krebsimmuntherapie könnte daher zum einen Tumoren mit schwach immunogenen Eigenschaften immunogener machen und die Wirksamkeit eine Krebsimmuntherapie verstärken und außerdem eine langanhaltende therapeutische Wirkung entfalten.

Darüber hinaus können die erfindungsgemäßen Verbindungen auch in Kombination mit einer Strahlentherapie und/oder einer chirurgischen Intervention eingesetzt werden.

Generell können mit der Kombination von Verbindungen der vorliegenden Erfindung mit anderen, zytostatisch, zytotoxisch oder immuntherapeutisch wirksamen Agenzien folgende Ziele verfolgt werden:
- eine verbesserte Wirksamkeit bei der Verlangsamung des Wachstums eines Tumors, bei der Reduktion seiner Größe oder sogar bei seiner völligen Eliminierung im Vergleich zu einer Behandlung mit einem einzelnen Wirkstoff;
- die Möglichkeit, die verwendeten Chemotherapeutika in geringerer Dosierung als bei der Monotherapie einzusetzen;
- die Möglichkeit einer verträglicheren Therapie mit weniger Nebeneffekten im Vergleich zur Einzelgabe;
- die Möglichkeit zur Behandlung eines breiteren Spektrums von Tumorerkrankungen;
- das Erreichen einer höheren Ansprechrate auf die Therapie;
- eine längere Überlebenszeit der Patienten im Vergleich zur heutigen Standardtherapie.

Darüber hinaus können die erfindungsgemäßen Verbindungen auch in Kombination mit einer Strahlentherapie und/oder einer chirurgischen Intervention eingesetzt werden.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie beispielsweise, parenteral, möglicherweise inhalativ oder als Implantat bzw. Stent.

Für diese Applikationswege können die erfindungsgemäßen Verbindungen in geeigneten Applikationsformen verabreicht werden.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen oder Lyophilisaten. Bevorzugt ist die parenterale Applikation, insbesondere die intravenöse Applikation.

Im Allgemeinen hat es sich als vorteilhaft erwiesen, bei parenteraler Applikation Mengen von etwa 0.1 bis 20 mg/kg, vorzugsweise etwa 0.3 bis 7 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

### Beispiele

Die nachfolgenden Beispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Wenn bei Versuchsbeschreibungen keine Angaben bzgl. einer Temperatur gemacht werden, bei der die Reaktion durchgeführt wird, ist von Raumtemperatur auszugehen.

### Synthesewege:

Exemplarisch für die Ausführungsbeispiele sind in folgenden Schemata beispielhafte Synthesewege zu den Ausführungsbeispielen dargestellt. Sowohl die Synthesesequenz als auch die Schutzgruppenstrategie kann auf dem Weg zu den Zielverbindungen variiert werden.

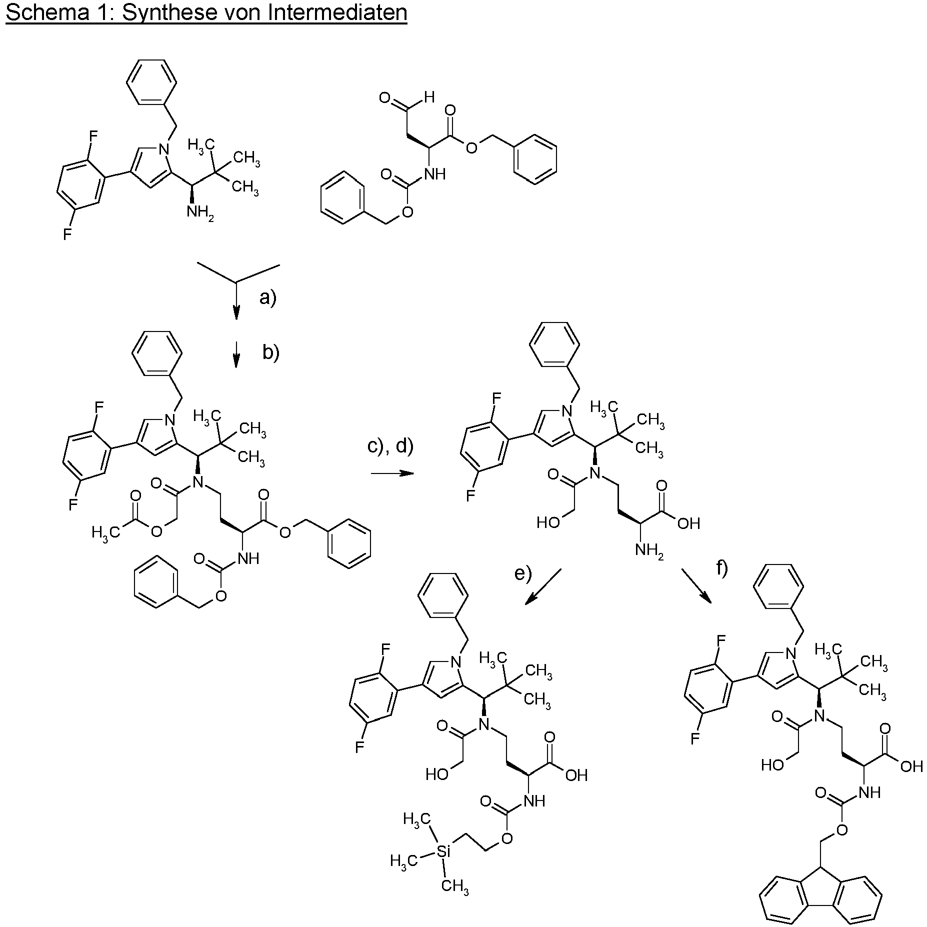

[a): beispielsweise Natriumtriacetoxyborhydrid, Essigsäure, DCM, RT; b) beispielsweise Acetoxyacetylchlorid, NEt3, DCM, RT; c) beispielsweise LiOH, THF/Wasser, RT; d) beispielsweise H₂, Pd-C, EtOH, RT; e) beispielsweise Teoc-OSu, NEt3, Dioxan, RT; f) beispielsweise Fmoc-CI, Diisopropylethylamin, Dioxan/Wasser 2:1, RT] 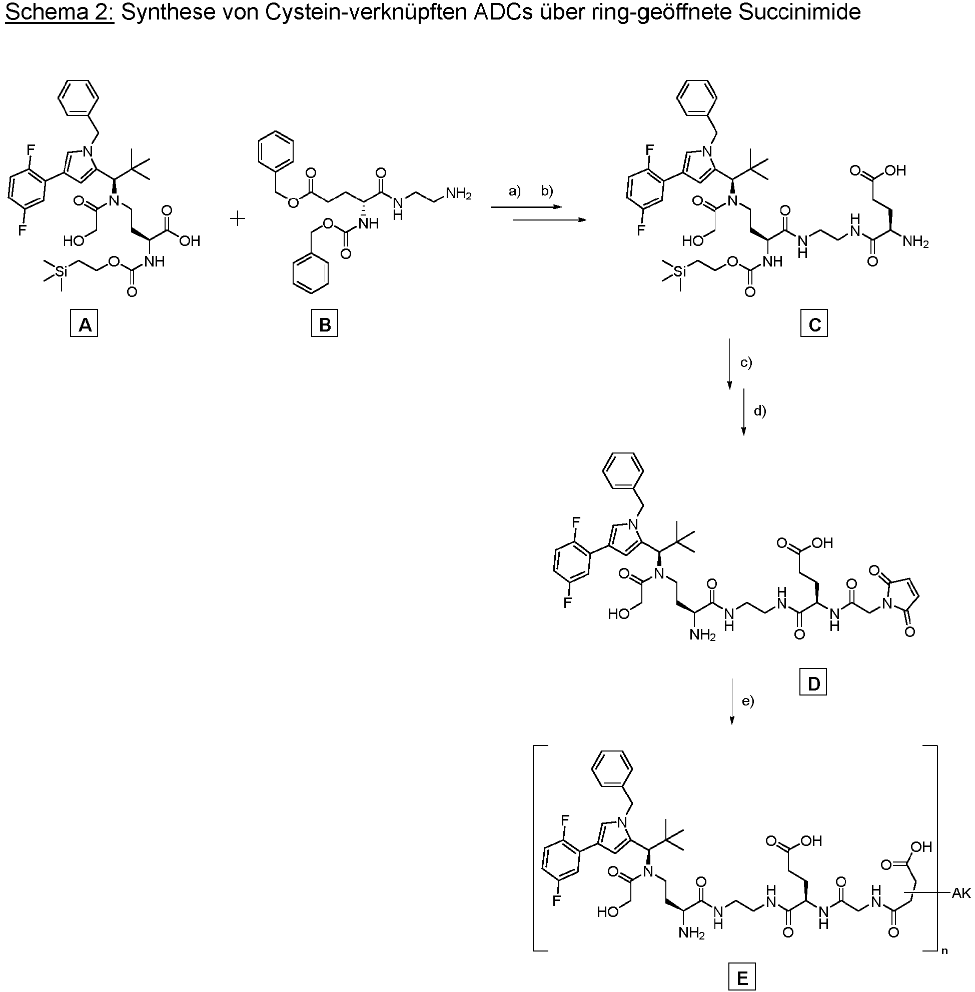 [a): HATU, DMF, N,N-Diisopropylethylamin, RT; b): H₂, 10% Pd-C, Methanol, RT c): 1,1'-[(1,5-Dioxopyrrolidin-1-yl)oxy]-2-oxoethyl}-1H-pyrrol-2,5-dion, N,N-Diisopropylethylamin, DMF, Rühren bei RT; d) 2,2,2-Trifluorethanol, 4-8 Equivalente Zinkchlorid, 2-6 h bei 50°C; e) AK in PBS lösen, unter Argon 3-4 Äquivalente TCEP in PBS-Puffer zusetzen und ca. 30 min Rühren bei RT rühren, dann 5-10 Equivalente von Verbindung D gelöst in DMSO zusetzten, ca. 90 min bei RT Rühren, dann Umpuffern auf pH 8 über mit PBS Puffer (pH 8) equillibrierten PD 10-Säulen (Sephadex® G-25, GE Healthcare), dann über Nacht bei RT Rühren; dann ggf. Reinigung über mit PBS Puffer (pH 7.2) equillibrierte PD 10-Säulen (Sephadex® G-25, GE Healthcare) und anschließendes Aufkonzentrieren mittels Ultrazentrifugation und Einstellen der angestrebten Konzentration mit PBS Puffer (pH 7.2)]. Ggf. schließt sich bei in vivo Batches noch eine Sterilfiltration an.

### A. Beispiele

### Abkürzungen und Akronyme:

- ABCB1: ATP-binding cassette sub-family B member 1 (Synonym für P-gp und MDR1)
- abs.: Absolut
- Ac: Acetyl
- ACN: Acetonitril
- aq.: wässrig, wässrige Lösung
- ATP: Adenosintriphosphat
- BCRP: Brustkrebs-Resistenz-Protein, ein Efflux-Transporter
- BEP: 2-Brom-1-ethylpyridinium-Tetrafluoroborat
- Boc: *tert*.-Butoxycarbonyl
- br.: breit (bei NMR)
- Bsp.: Beispiel
- C: Konzentration
- *ca.*: *circa,* ungefähr
- Cl: chemische Ionisation (bei MS)
- D: Dublett (bei NMR)
- D: Tag(e)
- DAR: drug-to-antibody ratio
- DC: Dünnschichtchromatographie
- DCI: direkte chemische Ionisation (bei MS)
- DCM: Dichlormethan
- Dd: Dublett von Dublett (bei NMR)
- DMAP: 4-*N*,*N*-Dimethylaminopyridin
- DME: 1,2-Dimethoxyethan
- DMEM: Dulbecco's Modified Eagle Medium (standardisiertes Nährmedium für die Zellkultur)
- DMF: *N*,*N*-Dimethylformamid
- DMSO: Dimethylsulfoxid
- D/P: Dye (Fluoreszenzfarbstoff)/Protein Verhältnis
- DPBS, D-PBS,: Dulbecco's Phosphat-gepufferte Salz-Lösung
- DSMZ: Deutsche Sammlung von Mikroorganismen und Zellkulturen
- PBS: PBS = DPBS = D-PBS, pH7,4, Fa. Sigma, No D8537 Zusammensetzung: 0,2 g KCl 0,2 g KH₂PO₄ (anhyd) 8,0 g NaCl 1,15 g Na₂HPO₄ (anhyd) ad 1 l mit H₂O auffüllen
- Dt: Dublett von Triplett (bei NMR)
- DTT: DL-Dithiothreitol
- d. Th.: der Theorie (bei chemischer Ausbeute)
- EDC: *N*'-(3-Dimethylaminopropyl)-*N*-ethylcarbodiimid-Hydrochlorid
- EGFR: Epidermal growth factor receptor = Epidermaler Wachstumsfaktor Rezeptor
- El: Elektronenstoß-Ionisation (bei MS)
- ELISA: Enzyme-linked Immunosorbent Assay
- eq.: Äquivalent(e)
- ESI: Elektrospray-Ionisation (bei MS)
- ESI-MicroTofq: ESI- MicroTofq (Name des Massenspektrometer mit Tof = Time Of Flight und q = Quadrupol)
- FCS: fötales Kälberserum
- Fmoc: (9*H*-Fluoren-9-ylmethoxy)carbonyl
- ges.: Gesättigt
- GTP: Guanosin-5'-triphosphat
- H: Stunde(n)
- HATU: *O*-(7-Azabenzotriazol-1-yl)-*N*,*N*,*N*',*N*'-tetramethyluroniumhexafluorophosphat
- HEPES: 4-(2-Hydroxyethyl)piperazin-1-ethansulfonsäure
- HOAc: Essigsäure
- HOAt: 1-Hydroxy-7-azabenzotriazol
- HOBt: 1-Hydroxy-1*H*-benzotriazol-Hydrat
- HOSu: *N*-Hydroxysuccinimid
- HPLC: Hochdruck-, Hochleistungsflüssigchromatographie
- IC₅₀: halbmaximale Inhibitionskonzentration
- i.m.: intramuskulär, Applikation in den Muskel
- i.v.: intravenös, Applikation in die Vene
- KLP-4: humane Tumorzelllinie
- konz.: Konzentriert
- LC-MS: Flüssigchromatographie-gekoppelte Massenspektrometrie
- LLC-PK1-Zellen: Lewis lung carcinoma pork kidney cell line
- L-MDR: human MDR1 transfizierte LLC-PK1 Zellen
- M: Multiplett (bei NMR)
- MDR1: Multidrug resistence protein 1
- MeCN: Acetonitril
- Me: Methyl
- Min: Minute(n)
- MOLM-13: humane Tumorzelllinie
- MS: Massenspektrometrie
- MTT: 3-(4,5-Dimethylthiazol-2-yl)-2,5-diphenyl-2H-tetrazoliumbromid
- MV-4-11: humane Tumorzelllinie
- NCI-H292: humane Tumorzelllinie
- NMM: *N*-Methylmorpholin
- NMP: *N*-Methyl-2-pyrrolidinon
- NMR: Kernresonanzspektrometrie
- NMRI: Mausstamm, Herkunft aus dem Naval Medical Research Institute (NMRI)
- Nude Mäuse: Nacktmäuse (Versuchstiere)
- NSCLC: Non small cell lung cancer (Nicht-kleinzelliges Bronchialkarzinom)
- PBS: Phosphat-gepufferte Salz-Lösung
- Pd/C: Palladium auf Aktivkohle
- P-gp: P-Glycoprotein, ein Transporterprotein
- PNGaseF: Enzym zur Zuckerabspaltung
- quant.: quantitativ (bei Ausbeute)
- quart: Quartett (bei NMR)
- quint: Quintett (bei NMR)
- Rec-1: humane Tumorzelllinie
- R_{f}: Retentionsindex (bei DC)
- RT: Raumtemperatur
- Rₜ: Retentionszeit (bei HPLC)
- S: Singulett (bei NMR)
- s.c.: subcutan, Applikation unter die Haut
- SCID Mäuse: Versuchsmäuse mit einem schweren kombinierten Immundefekt (severe combined immunodeficiency)
- SK-HEP-1: humane Tumorzelllinie
- t: Triplett (bei NMR)
- TBAF: Tetra-n-butylammoniumfluorid
- TEMPO: (2,2,6,6-Tetramethyl-piperidin-1-yl)oxyl
- Teoc: Trimethylsilylethoxycarbonyl
- Teoc-OSu: 1-({[2-(Trimethylsilyl)ethoxy]carbonyl}oxy)pyrrolidin-2,5-dion
- *tert.*: Tertiär
- TFA: Trifluoressigsäure
- THF: Tetrahydrofuran
- T3P^{®}: 2,4,6-Tripropyl-1,3,5,2,4,6-trioxatriphosphinan-2,4,6-trioxid
- UV: Ultraviolett-Spektrometrie
- v/v: Volumen zu Volumen-Verhältnis (einer Lösung)
- Z: Benzyloxycarbonyl

### HPLC- und LC-MS-Methoden:

### Methode 1 (LC-MS):

Instrument: Waters ACQUITY SQD UPLC System; Säule: Waters Acquity UPLC HSS T3 1.8 µ 50 × 1 mm; Eluent A: 1 l Wasser + 0.25 mL 99%ige Ameisensäure, Eluent B: 1 l Acetonitril + 0.25 mL 99%ige Ameisensäure; Gradient: 0.0 min 90% A → 1.2 min 5% A → 2.0 min 5% A Ofen: 50°C; Fluss: 0.40 mL/min; UV-Detektion: 208-400 nm.

### Methode 2 (LC-MS):

Gerätetyp MS: Waters Synapt G2S; Gerätetyp UPLC: Waters Acquity I-CLASS; Säule: Waters, BEH300, 2.1 × 150 mm, C18 1.7 µm; Eluent A: 1 l Wasser + 0.01% Ameisensäure; Eluent B: 1 l Acetonitril + 0.01 % Ameisensäure; Gradient: 0.0 min 2% B → 1.5 min 2% B → 8.5 min 95% B → 10.0 min 95% B; Ofen: 50°C; Fluss: 0.50 mL/min; UV-Detektion: 220 nm

### Methode 3 (LC-MS):

Instrument MS: Waters (Micromass) QM; Instrument HPLC: Agilent 1100 Serie; Säule : Agient ZORBAX Extend-C18 3.0x50mm 3.5-Micron; Eluent A: 1 l Wasser + 0.01 mol

Ammoniumcarbonat, Eluent B: 1 l Acetonitril; Gradient: 0.0 min 98% A → 0.2min 98% A → 3.0 min 5% A→ 4.5 min 5% A ; Ofen: 40°C; Fluss: 1.75 mL/min; UV-Detektion: 210 nm

### Methode 4 (LC-MS):

Gerätetyp MS: Waters Synapt G2S; Gerätetyp UPLC: Waters Acquity I-CLASS; Säule: Waters, HSST3, 2.1 × 50mm, C18 1.8 µm; Eluent A: 1 l Wasser + 0.01% Ameisensäure; Eluent B: 1 l Acetonitril + 0.01% Ameisensäure; Gradient: 0.0 min 10% B → 0.3 min 10% B → 1.7 min 95% B → 2.5 min 95% B; Ofen: 50°C; Fluss: 1.20 mL/min; UV-Detektion: 210 nm

### Methode 5 (LC-MS):

Instrument: Waters ACQUITY SQD UPLC System; Säule: Waters Acquity UPLC HSS T3 1.8 µ 50 × 1 mm; Eluent A: 1 l Wasser + 0.25 mL 99%ige Ameisensäure , Eluent B: 1 l Acetonitril + 0.25 mL 99%ige Ameisensäure; Gradient: 0.0 min 95% A → 6.0 min 5% A → 7.5 min 5% A Ofen: 50°C; Fluss: 0.35 mL/min; UV-Detektion: 210 - 400 nm.

### Methode 6 (LC-MS):

Instrument: Micromass Quattro Premier mit Waters UPLC Acquity; Säule: Thermo Hypersil GOLD 1.9 µ 50 × 1 mm; Eluent A: 1 l Wasser + 0.5 mL 50%ige Ameisensäure, Eluent B: 1 l Acetonitril + 0.5 mL 50%ige Ameisensäure; Gradient: 0.0 min 97% A → 0.5 min 97% A → 3.2 min 5% A → 4.0 min 5% A Ofen: 50°C; Fluss: 0.3 mL/min; UV-Detektion: 210 nm.

### Methode 7 (LC-MS):

Instrument: Agilent MS Quad 6150;HPLC: Agilent 1290; Säule: Waters Acquity UPLC HSS T3 1.8 µ 50 × 2.1 mm; Eluent A: 1 l Wasser + 0.25 mL 99%ige Ameisensäure , Eluent B: 1 l Acetonitril + 0.25 mL 99%ige Ameisensäure; Gradient: 0.0 min 90% A → 0.3 min 90% A → 1.7 min 5% A → 3.0 min 5% A Ofen: 50°C; Fluss: 1,20 mL/min; UV-Detektion: 205 - 305 nm.

### Methode 8 (LC-MS):

Gerätetyp MS: Waters Synapt G2S; Gerätetyp UPLC: Waters Acquity I-CLASS; Säule: Waters, HSST3, 2.1 × 50 mm, C18 1.8 µm; Eluent A: 1 l Wasser + 0.01% Ameisensäure; Eluent B: 1 l Acetonitril + 0.01% Ameisensäure; Gradient: 0.0 min 2% B → 2.0 min 2% B → 13.0 min 90% B → 15.0 min 90% B; Ofen: 50°C; Fluss: 1.20 ml/min; UV-Detektion: 210 nm Methode 9: (LC-MS-Prep Reinigungsmethode)

Instrument MS: Waters, Instrument HPLC: Waters (Säule Waters X-Bridge C18, 19 mm × 50 mm, 5 µm, Eluent A: Wasser + 0.05% Ammoniak, Eluent B: Acetonitril (ULC) mit Gradient; Fluss: 40 ml/min; UV-Detektion: DAD; 210-400 nm).

### bzw.

Instrument MS: Waters, Instrument HPLC: Waters (Säule Phenomenex Luna 5µ C18(2) 100A, AXIA Tech. 50 × 21.2 mm, Eluent A: Wasser + 0.05% Ameisensäure, Eluent B: Acetonitril (ULC) mit Gradient; Fluss: 40 ml/min; UV-Detektion: DAD; 210 ― 400 nm).

### Methode 10: (LC-MS-Analytik-Methode)

Instrument MS: Waters SQD; Instrument HPLC: Waters UPLC; Säule: Zorbax SB-Aq (Agilent), 50 mm × 2.1 mm, 1.8 µm; Eluent A: Wasser + 0.025% Ameisensäure, Eluent B: Acetonitril (ULC) + 0.025% Ameisensäure; Gradient: 0.0 min 98%A - 0.9 min 25%A - 1.0 min 5%A - 1.4 min 5%A - 1.41 min 98%A - 1.5 min 98%A; Ofen: 40°C; Fluss: 0.600 ml/min; UV-Detektion: DAD; 210 nm.

### Methode 11 (HPLC):

Gerät: HP1100 Serie, Säule: Merck Chromolith SpeedROD RP-18e, 50-4,6mm, Best.-Nr. 1.51450.0001, Vorsäule Chromolith Guard Cartridge Kit, RP-18e, 5-4,6mm, Best.-Nr. 1.51470.0001; Gradient: Flow 5mL/ Min;lnjection Volume 5µl; Solvent A: HCLO4 (70%ig) in Wasser (4mL/ l), Solvent B: Acetonitril Start 20% B, 0.50 Min 20% B, 3.00 Min 90% B, 3.50 Min 90% B, 3.51 Min 20% B, 4.00 Min 20% B, Säulentemperatur: 40°C, Wellenlänge: 210nm

### Methode 12 (LC-MS):

Gerätetyp MS: Thermo Scientific FT-MS; Gerätetyp UHPLC+: Thermo Scientific UltiMate 3000; Säule: Waters, HSST3, 2.1 × 75 mm, C18 1.8 µm; Eluent A: 1 l Wasser + 0.01% Ameisensäure; Eluent B: 1 l Acetonitril + 0.01% Ameisensäure; Gradient: 0.0 min 10% B → 2.5 min 95% B → 3.5 min 95% B; Ofen: 50°C; Fluss: 0.90 ml/min; UV-Detektion: 210 nm/ Optimum Integration Path 210-300 nm

### Methode 13: (LC-MS):

Instrument MS: Waters (Micromass) Quattro Micro; Instrument Waters UPLC Acquity; Säule : Waters BEH C18 1.7 µ 50 × 2.1 mm; Eluent A: 1 l Wasser + 0.01 mol Ammoniumformiat, Eluent B: 1 l Acetonitril; Gradient: 0.0 min 95% A → 0.1 min 95% A → 2.0 min 15% A → 2.5 min 15% A→ 2.51 min 10% A → 3.0 min 10% A; Ofen: 40°C; Fluss: 0.5 ml/min; UV-Detektion: 210 nm

### Methode 14: (LC-MS):

Gerätetyp MS: ThermoFisherScientific LTQ-Orbitrap-XL; Gerätetyp HPLC: Agilent 1200SL; Säule: Agilent, POROSHELL 120, 3 × 150 mm, SB - C18 2.7 µm; Eluent A: 1 l Wasser + 0.1% Trifluoressigsäure; Eluent B: 1 l Acetonitril + 0.1% Trifluoressigsäure; Gradient: 0.0 min 2% B → 0.3 min 2% B → 5.0 min 95% B → 10.0 min 95% B; Ofen: 40°C; Fluss: 0.75 ml/min; UV-Detektion: 210 nm

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist und die nicht kommerziell erhältlich waren oder von Quellen bezogen wurden, die nicht allgemein zugänglich sind, ist ein Verweis auf die veröffentlichte Literatur angegeben, in der ihre Herstellung beschrieben ist.

### Ausgangsverbindungen und Intermediate:

### Intermediat C52

### (1R)-1-[1-Benzyl-4-(2,5-difluorphenyl)-1H-pyrrol-2-yl]-2,2-dimethylpropan-1-amin

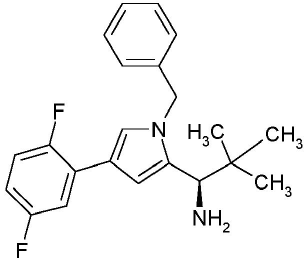

10.00 g (49.01 mmol) Methyl-4-brom-1H-pyrrol-2-carboxylat wurden in 100.0 mL DMF vorgelegt und mit 20.76 g (63.72 mmol) Cäsiumcarbonat und 9.22 g (53.91 mmol) Benzylbromid versetzt. Die Reaktionsmischung rührte über Nacht bei RT. Die Reaktionsmischung wurde zwischen Wasser und Ethylacetat verteilt und die wässrige Phase mit Ethylacetat extrahiert. Die vereinigten organischen Phasen wurden über Magnesiumsulfat getrocknet und das Lösemittel im Vakuum verdampft. Der Ansatz wurde mit 90.0 g Methyl-4-brom-1H-pyrrol-2-carboxylat wiederholt.

Die Reinigung der vereinigten beiden Ansätze erfolgte mittles präp. RP-HPLC (Säule: Daiso 300x100; 10µ, Fluss: 250 mL/min, MeCN/Wasser). Die Lösemittel wurden im Vakuum verdampft und der Rückstand im Hochvakuum getrocknet. Man erhielt 125.15 g (87 % d. Th.) der Verbindung Methyl-1-benzyl-4-brom-1H-pyrrol-2-carboxylat.

LC-MS (Methode 1): Rₜ = 1.18 min; MS (ESlpos): m/z = 295 [M+H]⁺.

Unter Argon wurden 4.80 g (16.32 mmol) Methyl-1-benzyl-4-brom-1H-pyrrol-2-carboxylat in DMF vorgelegt und mit 3.61 g (22.85 mmol) (2,5-Difluorphenyl)boronsäure und 19.20 mL ges. Natriumcarbonat-Lösung und 1.33 g (1.63 mmol) [1,1'-Bis-(diphenylphosphino)-ferrocen]-dichlorpalladium(II):Dichlormethan versetzt. Die Reaktionsmischung wurde über Nacht bei 85 °C gerührt. Die Reaktionsmischung wurde über Celite filtriert und der Filterkuchen mit Ethylacetat gewaschen. Die organische Phase wurde mit Wasser extahiert und dann mit ges. NaCI-Lösung gewaschen. Die organische Phase wurde über Magnesiumsulfat getrocknet und das Lösemittel im Vakuum verdampft. Der Rückstand wurde mittels Kieselgel (Laufmittel: Cyclohexan/Ethylacetat 100:3) gereinigt. Die Lösemittel wurden im Vakuum verdampft und der Rückstand im Hochvakuum getrocknet. Man erhielt 3.60 g (67 % d. Th.) der Verbindung Methyl-1-benzyl-4-(2,5-difluorphenyl)-1H-pyrrol-2-carboxylat.

LC-MS (Methode 7): Rₜ = 1.59 min; MS (ESlpos): m/z = 328 [M+H]⁺.

3.60 g (11.00 mmol) Methyl-1-benzyl-4-(2,5-difluorphenyl)-1H-pyrrol-2-carboxylat wurden in 90.0 mL THF vorgelegt und bei 0 °C mit 1.04 g (27.50 mmol) Lithiumaluminiumhydrid (2.4 M in THF) versetzt. Die Reaktionsmischung wurde 30 Minuten bei 0 °C gerührt. Es wurde bei 0 °C ges. Kaliumnatriumtartrat-Lösung zugegeben und die Reaktionsmischung mit Ethylacetat versetzt. Die organische Phase wurde dreimal mit ges. Kaliumnatriumtartrat-Lösung extrahiert. Die organische Phase wurde einmal mit ges. NaCI-Lösung gewaschen und über Magnesiumsulfat getrocknet. Das Lösemittel wurde im Vakuum verdampft und der Rückstand in 30.0 mL Dichlormethan gelöst. Es wurden 3.38 g (32.99 mmol) Mangan(IV)oxid zugegeben und 48 h bei RT gerührt. Es wurden nochmals 2.20 g (21.47 mmol) Mangan(IV)oxid zugegeben und über Nacht bei RT gerührt. Die Reaktionsmischung wurde über Celite filtriert und der Filterkuchen mit Dichlormethan gewaschen. Das Lösemittel wurde im Vakuum verdampft und der Rückstand 2.80 g (1-Benzyl-4-(2,5-difluorphenyl)-1H-pyrrol-2-carbaldehyd) wurde ohne weitere Reinigung in der nächsten Synthesestufe eingesetzt.

LC-MS (Methode 7): Rₜ = 1.48 min; MS (ESlpos): m/z = 298 [M+H]⁺.

28.21 g (94.88 mmol) 1-Benzyl-4-(2,5-difluorphenyl)-1H-pyrrol-2-carbaldehyd wurden zusammen mit 23.00 g (189.77 mmol) (R)-2-Methylpropan-2-sulfinamid in 403.0 mL absol. THF vorgelegt und mit 67.42 g (237.21 mmol) Titan(IV)isopropylat versetzt und über Nacht bei RT gerührt. Es wurden 500.0 mL ges. NaCI-Lösung und 1000.0 mL Ethylacetat zugegeben und 1 h bei RT gerührt. Es wurde über Kieselgur filtriert und das Filtrat zweimal mit ges. NaCl-Lösung gewaschen. Die org. Phase wurde über Magnesiumsulfat getrocknet und das Lösemittel wurde im Vakuum verdampft und der Rückstand wurde mittels Biotage Isolera gereinigt (Kieselgel, Säule 1500+340 g SNAP, Fluss 200 mL/min, Ethylacetat/Cyclohexan 1:10).

LC-MS (Methode 7): Rₜ = 1.63 min; MS (ESlpos): m/z = 401 [M+H]⁺.

25.00 g (62.42 mmol) (R)-N-{(E/Z)-[1-Benzyl-4-(2,5-difluorphenyl)-1H-pyrrol-2-yl]methylen}-2-methylpropan-2-sulfinamid wurden in absol. THF unter Argon vorgelegt und auf -78 °C abgekühlt. Dann wurden 12.00 g (187.27 mmol) tert.-Butyllithium (1.7 M Lösung in Pentan) bei -78 °C zugegeben und 3 h bei dieser Temperatur gerührt. Dann wurden bei -78 °C 71.4 mL Methanol und 214.3 mL ges. Ammoniumchlorid-Lösung nacheinander zugegeben und die Reaktionsmischung wurde auf RT kommen lassen und 1 h bei RT gerührt. Es wurde mit Ethylacetat verdünnt und mit Wasser gewaschen. Die organische Phase wurde über Magnesiumsulfat getrocknet und das Lösemittel wurde im Vakuum verdampft. Der Rückstand (R)-N-{(1R)-1-[1-Benzyl-4-(2,5-difluorphenyl)-1H-pyrrol-2-yl]-2,2-dimethylpropyl}-2-methylpropan-2-sulfinamid wurde ohne weitere Reinigung in der nächsten Synthesestufe eingesetzt.

LC-MS (Methode 6): Rₜ = 2.97 min; MS (ESlpos): m/z = 459 [M+H]⁺.

28.00 g (61.05 mmol) (R)-N-{(1R)-1-[1-Benzyl-4-(2,5-difluorphenyl)-1H-pyrrol-2-yl]-2,2-dimethylpropyl}-2-methylpropan-2-sulfinamid wurden in 186.7 mL 1,4-Dioxan vorgelegt und dann mit 45.8 mL HCl in 1,4-Dioxan-Lösung (4.0 M) versetzt. Die Reaktionsmischung wurde 2 h bei RT gerührt und das Lösemittel wurde im Vakuum verdampft. Der Rückstand wurde mittles präp. RP-HPLC gereinigt (Säule: Kinetix 100x30; Fluss: 60 mL/min, MeCN/Wasser). Das Acetonitril wurde im Vakuum verdampft und der wässrige Rückstand wurde mit Dichlormethan versetzt. Die organische Phase wurde mit Natriumhydrogencarbonat-Lösung gewaschen und über Magnesiumsulfat getrocknet. Das Lösemittel wurde im Vakuum verdampft und der Rückstand wurde im Hochvakuum getrocknet. Man erhielt 16.2 g (75 % d. Th.) der Titelverbindung.

LC-MS (Methode 6): Rₜ = 2.10 min; MS (ESlpos): m/z = 338 [M-NH_{2]}⁺, 709 [2M+H]+.

¹H-NMR (400 MHz, DMSO-d₆): δ [ppm] = 0.87 (s, 9H), 1.53 (s, 2H), 3.59 (s, 1H), 5.24 (d, 2H), 6.56 (s, 1H), 6.94 (m, 1H), 7.10 (d, 2H), 7.20 (m, 1H), 7.26 (m, 2H), 7.34 (m, 2H), 7.46 (m, 1H).

### Intermediat C58

(2S)-4-[{(1R)-1-[1-Benzyl-4-(2,5-difluorphenyl)-1H-pyrrol-2-yl]-2,2-dimethylpropyl}(glycoloyl) amino]-2-({[2-(trimethylsilyl)ethoxy]carbonyl}amino)butansäure

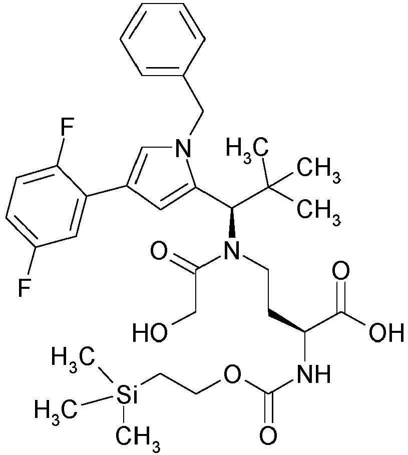

4.3 g (12.2 mmol) von Intermediat C52 wurden in 525 mL DCM gelöst und mit 3.63 g (17.12 mmol) Natriumtriacetoxyborhydrid sowie mit 8.4 mL Essigsäure versetzt. Nach 5 min Rühren bei RT wurden 8.99 g (24.5 mmol) von Intermediat L57 gelöst in 175 mL DCM hinzugegeben und der Ansatz weitere 45 min bei RT gerührt. Der Ansatz wurde dann mit 300 mL DCM verdünnt und zweimal mit 100 mL Natriumhydrogencarbonat-Lösung und einmal mit ges. NaCI-Lösung gewaschen. Die organische Phase wurde über Magnesiumsulfat getrocknet, das Lösemittel im Vakuum verdampft und der Rückstand im Hochvakuum getrocknet. Der Rückstand wurde anschließend mittels präparativer RP-HPLC (Säule: Chromatorex C18) gereinigt. Nach Vereinigung der entsprechenden Fraktionen wurde das Lösemittel im Vakuum verdampft und der Rückstand im Hochvakuum getrocknet. So wurden 4.6 g (61 % d. Th.) Methyl-(2S)-4-({(1R)-1-[1-benzyl-4-(2,5-difluorphenyl)-1H-pyrrol-2-yl]-2,2-dimethylpropyl}amino)-2-({[2-(trimethylsilyl)ethoxy]carbonyl}amino)butanoat erhalten.

LC-MS (Methode 12): Rₜ = 1.97 min; MS (ESlpos): m/z = 614 (M+H)⁺.

2.06 g (3.36 mmol) von diesem Intermediat wurden in 76 mL DCM vorgelegt und mit 0.81 mL (7.17 mmol) 2-Chlor-2-oxoethylacetat in Gegenwart von 2.1 ml Triethylamin acyliert. Nach 20 h Rühren bei RT wurden weitere 0.36 mL 2-Chlor-2-oxoethylacetat und 0.94 ml Triethylamin zugegeben und der Ansatz weitere 15 min bei RT gerührt. Anschließend wurde mit 500 mL Ethylacetat verdünnt und nacheinander zweimal mit 300 mL 5%-iger Zitronensäure, zweimal mit 300 mL gesättigter Natriumhydrogencarbonatlösung und einmal mit 100 mL gesättigter Natriumchloridlösung ausgeschüttelt, dann über Magnesiumsulfat getrocknet und eingeengt. Nach Trocknung im Hochvakuum wurden 2.17 g (79% d. Th.) des geschützten Intermediats erhalten.

LC-MS (Methode 1): Rₜ = 1.48 min; MS (ESlpos): m/z = 714 (M+H)⁺.

2.17 g (2.64 mmol) dieses Intermediats wurden in 54 mL THF und 27 mL Wasser gelöst und mit 26 mL einer 2 molaren Lithiumhydroxidlösung versetzt. Der Ansatz wurde 30 min bei RT gerührt und dann mit 1.4 mL TFA auf einen pH-Wert zwischen 3 und 4 eingestellt. Der Ansatz wurde im Vakuum aufkonzentriert. Nachdem THF weitgehend abdestilliert war wurde die wäßrige Lösung zweimal mit DCM extrahiert und anschließend im Vakuum bis zur Trockne eingeengt. Der Rückstand wurde durch präparative HPLC (Säule: Chromatorex C18) gereinigt. Nach Vereinigung der entsprechenden Fraktionen wurde das Lösemittel im Vakuum verdampft und der Rückstand und aus Acetonitril/Wasser lyophilisiert. So wurden 1.1 g (63% d. Th.) der Titelverbindung erhalten.

LC-MS (Methode 1): Rₜ = 1.34 min; MS (ESlpos): m/z = 656 (M-H)⁻.

¹H-NMR (400 MHz, DMSO-d₆): δ [ppm] = 0.03 (s, 9H), 0.58 (m, 1H), 0.74-0.92 (m, 11H), 1.40 (m, 1H), 3.3 (m, 2H), 3.7 (m, 1H), 3.8-4.0 (m, 2H), 4.15 (q, 2H), 4.9 und 5.2 (2d, 2H), 5.61 (s, 1H), 6.94 (m, 2H), 7.13-7.38 (m, 7H), 7.48 (s, 1H), 7.60 (m, 1H), 12.35 (s, 1H).

### Intermediat C66

2-(Trimethylsilyl)ethyl-[(2S)-4-[{(1R)-1-[1-benzyl-4-(2,5-difluorphenyl)-1H-pyrrol-2-yl]-2,2-dimethylpropyl}(glycoloyl)amino]-1-{[2-(glycylamino)ethyl]amino}-1-oxobutan-2-yl]carbamat

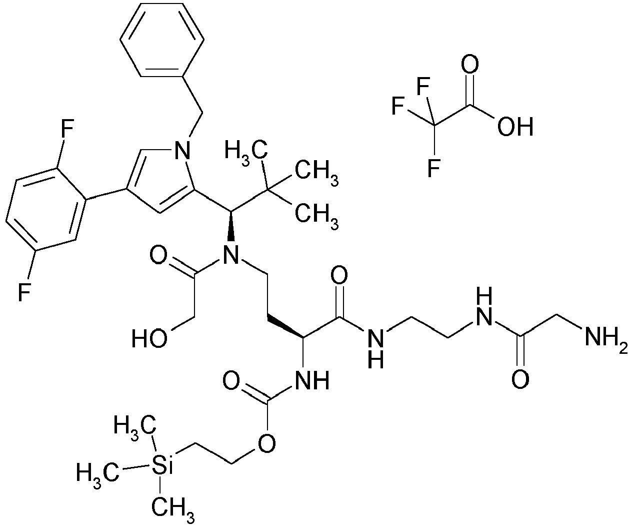

Zuächst wurde aus N-[(benzyloxy)carbonyl]glycin und tert-Butyl (2-aminoethyl)carbamat nach klassischen Methoden der Peptidchemie (HATU-Kupplung und Boc-Spaltung) Trifluoressigsäure -benzyl-{2-[(2-aminoethyl)amino]-2-oxoethyl}carbamat hergestellt.

13 mg (0.036 mmol) von diesem Intermediat sowie 25 mg (0.033 mmol) von Intermediat C58 wurden in 3 mL DMF aufgenommen und mit 19 mg (0.05 mmol) HATU und 17 µl N,N-Diisopropylethylamin versetzt. Nach 10 min Rühren bei RT wurde eingeengt und der Rückstand durch präparative HPLC gereinigt. Es wurden 17.8 mg (60% d. Th.) der Zwischenstufe erhalten.

LC-MS (Methode 1): Rₜ = 1.36 min; MS (ESlpos): m/z = 891 (M+H)⁺.

17 mg (0.019 mmol) dieser Zwischenstufe wurden in 10 ml Ethanol gelöst, mit Palladium auf Kohle (10%ig) versetzt und bei RT mit Wasserstoff unter Normaldruck 2 h hydriert. Der Katalysator wurde abfiltriert, die Lösemittel im Vakuum verdampft und der Rückstand im Hochvakuum getrocknet. Es wurden 9 mg (62% d. Th.) der Titelverbindung erhalten.

LC-MS (Methode 1): Rₜ = 1.03 min; MS (ESlpos): m/z = 757 (M+H)⁺.

### Intermediat C118

tert-Butyl-N-[(8S)-8-{2-[{(1R)-1-[1-benzyl-4-(2,5-difluorphenyl)-1H-pyrrol-2-yl]-2,2-dimethylpropyl}(glycoloyl)amino]ethyl}-2,2-dimethyl-6,9-dioxo-5-oxa-7,10-diaza-2-siladodecan-12-yl]-D-alpha-glutaminat

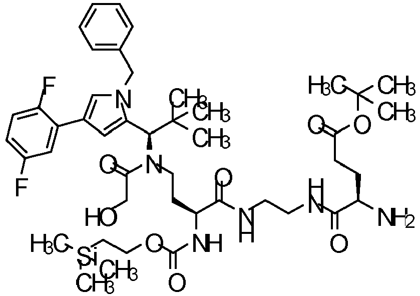

Die Titelverbindung wurde nach klassischen Methoden der Peptidchemie durch Kupplung von Intermediat L119 und Intermediat C58 in Gegenwart von HATU und anschließender hydrogenolytischer Abspaltung der Z-Schutzgruppe hergestellt.

LC-MS (Methode 1): Rₜ = 1.05 min; MS (ESlpos): m/z = 885 (M+H)⁺.

### Intermediat C119

tert-Butyl-glycyl-N-[(8S)-8-{2-[{(1R)-1-[1-benzyl-4-(2,5-difluorphenyl)-1H-pyrrol-2-yl]-2,2-dimethylpropyl}(glycoloyl)amino]ethyl}-2,2-dimethyl-6,9-dioxo-5-oxa-7,10-diaza-2-siladodecan-12-yl]-D-alpha-glutaminat

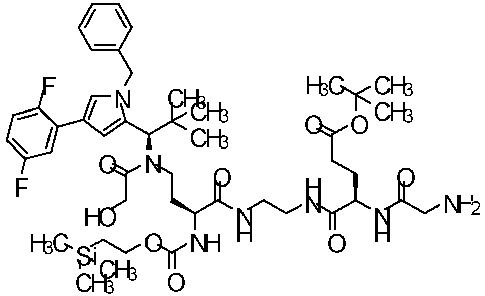

Intermediat C119 wurde nach klassischen Methoden der Peptidchemie durch Kupplung von 2,5-Dioxopyrrolidin-1-yl-N-[(benzyloxy)carbonyl]glycinat und Intermediat C118 in Gegenwart von HATU und anschließender Abspaltung der Z-Schutzgruppe Hydrierung über 10%-igem Palladium auf Aktivkohle in Methanol/Dichlormethan bei RT unter Wasserstoff-Normaldruck hergestellt.

LC-MS (Methode 1): Rₜ = 1.03 min; MS (ESlpos): m/z = 942 (M+H)⁺.

### Intermediat L1

Trifluoressigsäure--N-(2-aminoethyl)-2-(2,5-dioxo-2,5-dihydro-1H-pyrrol-1-yl)acetamid

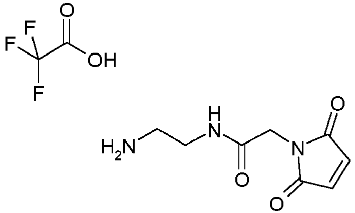

Die Titelverbindung wurde nach klassischen Methoden der Peptidchemie aus kommerziell erhältlicher (2,5-Dioxo-2,5-dihydro-1H-pyrrol-1-yl)essigsäure und tert-Butyl-(2-aminoethyl) carbamat hergestellt.

LC-MS (Methode 1): Rₜ = 0.17 min; MS (ESlpos): m/z = 198 (M+H)⁺.

### Intermediat L57

Methyl-(2S)-4-oxo-2-({[2-(trimethylsilyl)ethoxy]carbonyl}amino)butanoat

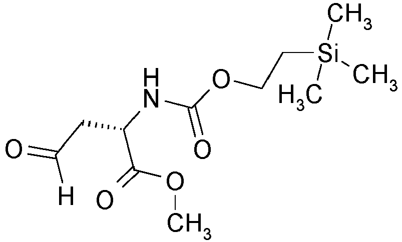

500.0 mg (2.72 mmol) L-Asparaginsäuremethylester Hydrochlorid und 706.3 mg (2.72 mmol) 2-(Trimethylsilyl)ethyl-2,5-dioxopyrrolidin-1-carboxylat wurden in 5.0 mL 1,4-Dioxan vorgelegt und mit 826.8 mg (8.17 mmol) Triethylamin versetzt. Die Reaktionsmischung wurde über Nacht bei RT gerührt. Die Reaktionsmischung wurde direkt mittles präp. RP-HPLC gereinigt (Säule: Reprosil 250x40; 10µ, Fluss: 50 mL/min, MeCN/Wasser, 0.1 % TFA). Die Lösemittel wurden dann im Vakuum verdampft und der Rückstand im Hochvakuum getrocknet. Man erhielt 583.9 mg (74 % d. Th.) der Verbindung (3S)-4-Methoxy-4-oxo-3-({[2-(trimethylsilyl)ethoxy]carbonyl}amino) butansäure.

LC-MS (Methode 1): Rₜ = 0.89 min; MS (ESlneg): m/z = 290 (M-H)⁻.

592.9 mg (3S)-4-Methoxy-4-oxo-3-({[2-(trimethylsilyl)ethoxy]carbonyl}amino)butansäure wurden in 10.0 mL 1,2-Dimethoxyethan vorgelegt und auf-15 °C abgekühlt und mit 205.8 mg (2.04 mmol) 4-Methylmorpholin und 277.9 mg (2.04 mmol) Isobutylchlorformiat versetzt. Der Niederschlag wird nach 15 min abgesaugt und zweimal mit je 10.0 mL 1,2-Dimethoxyethan gewaschen. Das Filtrat wird auf-10 °C abgekühlt und mit 115.5 mg (3.05 mmol) Natriumborhydrid gelöst in 10 mL Wasser unter starkem Rühren versetzt. Die Phasen werden getrennt und die organische Phase je einmal mit ges. Natriumhydrogencarbonat-Lösung und ges. NaCI-Lösung gewaschen. Die organische Phase wurde über Magnesiumsulfat getrocknet und das Lösemittel im Vakuum verdampft und der Rückstand im Hochvakuum getrocknet. Man erhielt 515.9 mg (91 % d. Th.) der Verbindung Methyl-N-{[2-(trimethylsilyl)ethoxy]carbonyl}-L-homoserinat.

LC-MS (Methode 1): Rₜ = 0.87 min; MS (ESlpos): m/z = 278 (M+H)⁺.

554.9 mg (2.00 mmol) Methyl-N-{[2-(trimethylsilyl)ethoxy]carbonyl}-L-homoserinat wurden in 30.0 mL Dichlormethan vorgelegt und mit 1.27 g (3.0 mmol) Dess-Martin periodinan und 474.7 mg (6.00 mmol) Pyridin versetzt. Man rührte über Nacht bei RT. Nach 4 h wurde der Ansatz mit Dichlormethan verdünnt und die organische Phase je dreimal mit 10%iger Na₂S₂O₃-Lösung, 10%iger Citronensäure-Lösung und ges. Natriumhydrogencarbonat-Lösung gewaschen. Die organische Phase wurde über Magnesiumsulfat getrocknet und das Lösemittel im Vakuum verdampft. Man erhielt 565.7 mg (97 % d. Th.) der Titelverbindung.

¹H-NMR (400 MHz, DMSO-d₆): δ [ppm] = 0.03 (s, 9H), 0.91 (m, 2H), 2.70-2.79 (m, 1H), 2.88 (dd, 1H), 3.63 (s, 3H), 4.04 (m, 2H), 4.55 (m, 1H), 7.54 (d, 1H), 9.60 (t, 1H).

### Intermediat L119

Trifluoressigsäure --tert-butyl-N-(2-aminoethyl)-N²-[(benzyloxy)carbonyl]-D-alpha-glutaminat Salz

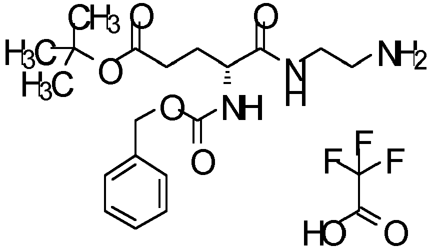

Intermediat L119 wurde nach klassischen Methoden der Peptidchemie durch Kupplung von kommerziell erhältlichen (2R)-2-{[(Benzyloxy)carbonyl]amino}-5-tert-butoxy-5-oxopentansäure (1.00 g, 2.96 mmol) und tert-Butyl-(2-aminoethyl)carbamat (560 µl, 3.6 mmol) in Gegenwart von HATU und anschließender saurer Abspaltung der Boc-Schutzgruppe mit 10%-iger TFA in Dichlormethan unter weitgehendem Erhalt der t-Butylester-Schutzgruppe hergestellt. Nach Reinigung durch präparative HPLC wurde die Titelverbindung erhalten.

LC-MS (Methode 1): Rₜ = 0.62 min; MS (ESI-pos): m/z = 380 (M+H)⁺.

### Intermediat L120

Benzyl-N-(2-aminoethyl)-N2-[(benzyloxy)carbonyl]-D-alpha-glutaminat

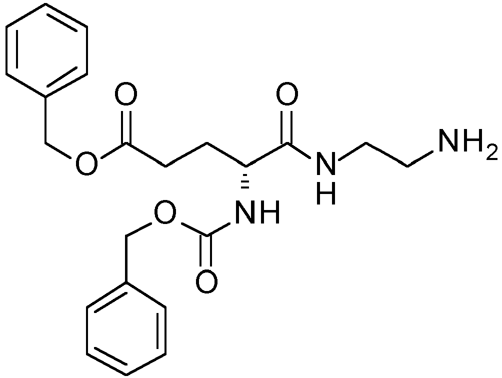

Intermediat L120 wurde nach klassischen Methoden der Peptidchemie durch Kupplung von kommerziell erhältlichen (2R)-5-(Benzyloxy)-2-{[(benzyloxy)carbonyl]amino}-5-oxopentansäure (830 mg, 2.23 mmol) und tert-Butyl-(2-aminoethyl)carbamat (420 µl, 2.7 mmol) in Gegenwart von HATU und anschließender saurer Abspaltung der Boc-Schutzgruppe mit TFA in Dichlormethan hergestellt.

### Intermediat F104

Trifluoressigsäure--(2S)-2-amino-4-[{(1R)-1-[1-benzyl-4-(2,5-difluorphenyl)-1H-pyrrol-2-yl]-2,2-dimethylpropyl}(glycoloyl)amino]-N-(2-{[(2,5-dioxo-2,5-dihydro-1H-pyrrol-1-yl)acetyl]amino}ethyl)butanamid Salz

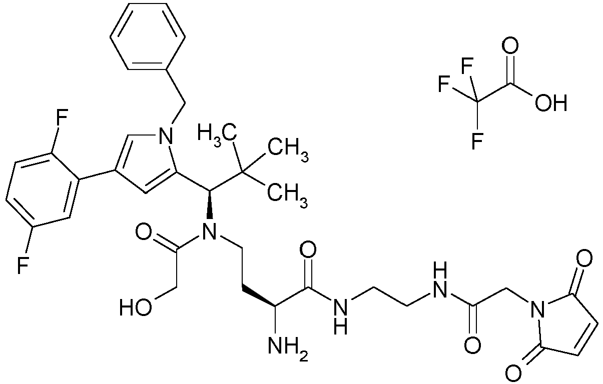

300 mg (0.456 mmol) von Intermediat C58 wurden in 38 mL DMF gelöst und mit 142 mg (0.456 mmol) von Intermediat L1 sowie mit 260 mg (0.684 mmol) HATU und 318 µl N,N-Diisopropylethylamin versetzt. Der Ansatz wurde 60 min bei RT gerührt und anschließend eingeengt. Der Rückstand wurde durch präparative HPLC gereinigt und nach Lyophilisation wurden 338 mg (87% d. Th.) der geschützten Zwischstufe erhalten.

LC-MS (Methode 1): Rₜ = 1.30 min; MS (ESlpos): m/z = 837 (M+H)⁺.

Im zweiten Schritt wurden 338 mg (0.404 mmol) dieses Intermediat in 40 mL 2,2,2-Trifluorethanol gelöst. Es wurden 330.2 mg (2.42 mmol) Zinkchlorid zugegeben und der Ansatz 3 h bei 50°C gerührt. Anschließend wurden 708 mg (2.42 mmol) Ethylendiamin-N,N,N',N'-tetraessigsäure sowie 4 mL einer 0.1%-igen wässrigen Trifluoressigsäurelösung zugesetzt. Der Ansatz wurde mittels präparativer HPLC gereinigt. Nach Einengen der entsprechenden Fraktionen und Lyophilisation des Rückstands aus Acetonitril/Wasser wurden 265 mg (81% d.Th.) der Titelverbindung erhalten.

LC-MS (Methode 1): Rₜ = 0.82 min; MS (ESlpos): m/z = 693 (M+H)⁺.

### Intermediat F325

N-[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-N²-[(2,5-dioxo-2,5-dihydro-1H-pyrrol-1-yl)acetyl]-D-alpha-glutamin -trifluoressigsäure Salz

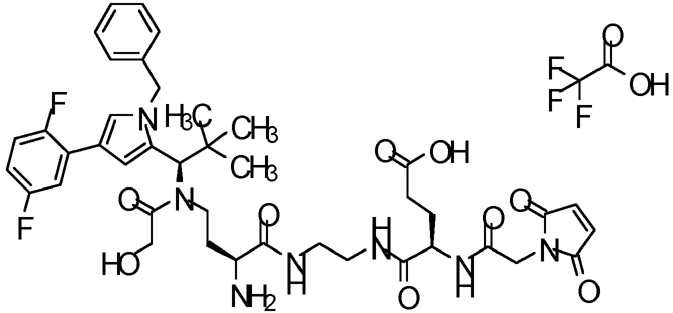

30 mg (0.046 mmol) von Intermediat C58 wurden mit 29 mg (0.055 mmol) Trifluoressigsäure - benzyl-N-(2-aminoethyl)-N2-[(benzyloxy)carbonyl]-D-alpha-glutaminat Salz (Intermediat L120) in Gegenwart von 1.5 Equiv. HATU und 3 Equiv. *N*,*N*-Diisopropylethylamin gekuppelt. Nach Reinigung mittels präparativer HPLC wurden 39.5 mg (82% d.Th.) des geschützten Intermediats erhalten. Von diesem wurden zunächst hydrogenolytisch die Benzylestergruppen abgespalten. Anschließend erfolgte die Kupplung mit 1-{2-[(2,5-Dioxopyrrolidin-1-yl)oxy]-2-oxoethyl}-1H-pyrrol-2,5-dion in DMF in Gegenwart von 3 Equiv. *N*,*N*-Diisopropylethylamin. Im letzten Schritt wurden 13.5 mg (0.012 mmol) dieses Intermediats in 5 mL 2,2,2-Trifluorethanol gelöst. Es wurden 13 mg (0.096 mmol) Zinkchlorid zugegeben und der Ansatz 3 h bei 50°C gerührt. Anschließend wurden 28 mg (0.096 mmol) Ethylendiamin-N,N,N',N'-tetraessigsäure zugesetzt. Der Ansatz wurde mittels präparativer HPLC gereinigt. Nach Einengen der entsprechenden Fraktionen und Lyophilisation des Rückstands aus Acetonitril/Wasser wurden 9 mg (81% d.Th.) der Titelverbindung erhalten.

LC-MS (Methode 12): Rₜ = 1.44 min; MS (ESlpos): m/z = 822 (M+H)⁺.

### B: Herstellung von Antikörper-Wirkstoff-Konjugaten (ADC)

### B-1. Allgemeines Verfahren zur Generierung von anti-CD123 und anti-CXCR5 Antikörpern sowie chimären und humanisierten Varianten von anti-CD123 und anti-CXCR5 Antikörpern

Die Proteinsequenz (Aminosäuresequenz) der verwendeten Antikörper, zum Beispiel die anti-CD123 Antikörper TPP-8987, TPP-8988 und TPP-9476 sowie die anti-CXCR5 Antikörper TPP-9024, TPP-9574 und TPP-9580, wurde in eine für das jeweilige Protein kodierende DNA Sequenz nach einem dem Fachmann bekannten Verfahren überführt und in einen für die transiente Säugerzellkultur geeigneten Expressionsvektor inseriert (wie von Tom et al., Kapitel 12 in Methods Express: Expression Systems herausgegeben von Micheal R. Dyson and Yves Durocher, Scion Publishing Ltd, 2007 beschrieben).

### B-2. Allgemeines Verfahren zur Expression von Antikörpern in Säugerzellen

Die Antikörper, zum Beispiel die anti-CD123 Antikörper TPP-8987, TPP-8988 und TPP-9476 sowie die anti-CXCR5 Antikörper TPP-9024, TPP-9574 und TPP-9580, wurden in transienten Säugerzellkulturen produziert, wie von Tom et al., Kapitel 12 in Methods Express: Expression Systems herausgegeben von Micheal R. Dyson and Yves Durocher, Scion Publishing Ltd, 2007 beschrieben.

### B-3. Allgemeines Verfahren zur Aufreinigung von Antikörpern aus Zellüberständen.

Die Antikörper, zum Beispiel die anti-CD123 Antikörper TPP-8987, TPP-8988 und TPP-9476 sowie die anti-CXCR5 Antikörper TPP-9024, TPP-9574 und TPP-9580, wurden aus den Zellkulturüberständen gewonnen. Die Zellüberstände wurden durch Zentrifugation von Zellen geklärt. Anschließend wurde der Zellüberstand durch Affinitätschromatographie auf einer MabSelect Sure (GE Healthcare) Chromatographiesäule gereinigt. Dazu wurde die Säule in DPBS pH 7.4 (Sigma/Aldrich) equillibriert, der Zellüberstand aufgetragen und die Säule mit ca 10 Säulenvolumina DPBS pH 7.4 + 500 mM Natriumchlorid gewaschen. Die Antikörper wurden in 50 mM Natriumacetat pH 3.5 + 500 mM Natriumchlorid eluiert und anschließend durch Gelfiltrationschromatographie auf einer Superdex 200 Säule (GE Healthcare) in DPBS pH 7.4 weiter gereinigt.

Kommerziell erhältliche Antikörper wurden mit Standard- Chromatographiemethoden aus den Handelsprodukten aufgereinigt (Protein A Chromatopgraphie, präparative Gelfiltrationschomatographie (SEC - size exclusion chromatographie)).

### B-4. Allgemeines Verfahren zur Kupplung an Cystein-Seitenketten

In die Kupplungsreaktionen wurden folgende Antikörper eingesetzt:
anti-CD123 AK TPP-8987
anti-CD123 AK TPP-8988
anti-CD123 AK TPP-9476
anti-CXCR5 AK TPP-9024
anti-CXCR5 AK TPP-9574
anti-CXCR5 AK TPP-9580

### Small Scale Kupplung:

Zu einer Lösung von 2-5 mg des entsprechenden Antikörpers in PBS-Puffer im Konzentrationsbereich zwischen 1 mg/mL und 20 mg/mL, bevorzugt im Bereich von ungefähr 5 mg/ml bis 15 mg/ml wurden zwischen 2 und 5 Äquivalenten Tris(2-carboxyethyl)phosphin-Hydrochlorid (TCEP), gelöst in PBS-Puffer, gegeben und 30 min bis 1h bei RT gerührt. Anschließend wurden, je nach angestrebter Beladung, zwischen 2 und 12 Äquivalenten, bevorzugt etwa 5-10 Äquivalente der zu kuppelnden Maleinimid-Vorläufer-Verbindung als Lösung in DMSO hinzugefügt. Dabei sollte die Menge an DMSO 10% des Gesamtvolumens nicht überschreiten. Der Ansatz wurde 60-240 min bei RT gerührt und anschließend mit PBS-Puffer, der zuvor auf pH 8 eingestellt wurde, auf ein Volumen von 2.5-7.5 ml verdünnt und dann über eine mit PBS-Puffer pH8 equillibrierte PD 10-Säule (Sephadex^{®} G-25, GE Healthcare) gegeben und mit PBS-Puffer pH8 eluiert. Das Eluat wurde über Nacht bei RT unter Argon gerührt. Anschließend wurde die Lösung durch Ultrazentrifugation aufkonzentriert und rückverdünnt mit PBS-Puffer (pH 7.2).

### Medium scale Kupplung:

20-200 mg des betreffenden Antikörpers in PBS-Puffer (c~5-15 mg/mL) wurden unter Argon mit einer Lösung aus 2-5 Equivalenten, bevorzugt 3 Equivalenten TCEP in PBS-Puffer (c~0.2-0.8 mg/ml bevorzugt 0.5 mg/ml) versetzt. Der Ansatz wurde 30min bei RT gerührt und anschließend wurden 2-12, bevorzugt 5-10 Equivalente einer Maleinimid-Vorläuferverbindung gelöst in DMSO zugegeben. Nach weiteren 1.5h-2h Rühren bei RT wurde der Ansatz mit PBS-Puffer, der zuvor auf pH 8 eingestellt wurde, verdünnt.

Diese Lösung wurde dann über mit PBS-Puffer pH8 equillibrierte PD 10-Säulen (Sephadex^{®} G-25, GE Healthcare) gegeben und mit PBS-Puffer pH8 eluiert. Das Eluat wurde mit PBS-Puffer pH8 auf eine Konzentration von 1-5 mg/mL verdünnt. Diese Lösung wurde über Nacht bei RT unter Argon gerührt. Dann erfolgte ggf. wieder ein Umpuffern auf pH 7.2. Die ADC-Lösung wurde durch Ultrazentrifugation aufkonzentriert, rückverdünnt mit PBS-Puffer (pH7.2) und dann ggf. erneut bis auf eine Konzentration von etwa 10mg/mL aufkonzentriert.

In den dargestellten Strukturformeln kann dabei AK die Bedeutung haben, die aus den Tabellen nach den Ausführungsbeispielen hervorgeht:
anti-CD123 AK TPP-8987 (partiell reduziert)- S§¹
anti-CD123 AK TPP-8988 (partiell reduziert)- S§¹
anti-CD123 AK TPP-9476 (partiell reduziert)- S§¹
anti-CXCR5 AK TPP-9024 (partiell reduziert)- S§¹
anti-CXCR5 AK TPP-9574 (partiell reduziert)- S§¹
anti-CXCR5 AK TPP-9580 (partiell reduziert)- S§¹
wobei
- §¹: die Verknüpfung mit der Succinimid-Gruppe oder mit ggf. daraus entstandenen isomeren hydrolysierten offenkettigen Bernsteinsäureamiden bzw. des Alkylenrestes bedeutet, und
- S: für das Schwefelatom eines Cystein-Restes des partiell reduzierten Antikörpers steht.

### Weitere Aufreinigung und Charakterisierung der erfindungsgemäßen Konjugate

Nach erfolgter Umsetzung wurde in einigen Fällen das Reaktionsgemisch beispielsweise durch Ultrafiltration aufkonzentriert und anschließend mittels Chromatographie, beispielsweise mittels einer Sephadex® G-25, entsalzt und gereinigt. Die Elution erfolgte beispielsweise mit Phosphat-gepufferter Salzlösung (PBS). Anschließend wurde die Lösung sterilfiltriert und eingefroren. Alternativ kann das Konjugat lyophylisiert werden.

### B-7. Bestimmung des Antikörpers, der Toxophorbeladung und des Anteils geöffneter Cystein-Addukte

Zur Protein-Identifizierung wurde neben der Molekulargewichtsbestimmung nach Deglykosilierung und/oder Denaturierung ein tryptischer Verdau durchgeführt, der nach Denaturierung, Reduktion und Derivatisierung die Identität des Proteins anhand der nachgewiesenen tryptischen Peptide bestätigt.

Von den erhaltenen Lösungen der in den Ausführungsbeispielen beschriebenen Konjugate in PBS Puffer wurde die Toxophorbeladung (in den Tabellen bezeichnet als DAR, drug-to-antibody ratio)wie folgt bestimmt:
Die Bestimmung der Toxophor Beladung von Lysin-verknüpften ADCs erfolgte nach massenspektrometrischer Bestimmung der Molekulargewichte der einzelnen Konjugatspezies. Hierbei wurden vorab die Antikörperkonjugate mittels PNGaseF deglycosyliert, die Probe angesäuert und nach HPLC-Trennung/Entsalzung massenspektrometrisch mittels ESI-MicroTof_{Q} (Bruker Daltonik) analysiert. Alle Spektren über das Signal im TIC (Total Ion Chromatogramm) wurden addiert und das Molekulargewicht der verschiedenen Konjugatspezies auf Basis von MaxEnt Deconvolution berechnet. Nach Signal Integration der verschiedenen Spezies wurde dann die DAR (= Drug/Antibody Ratio) berechnet. Hierzu wurde die Summe der Toxophor-Anzahl gewichteten Integrationsergebnisse aller Spezies geteilt durch die Summe der einfach gewichteten Integrationsergebnisse aller Spezies.

Die Bestimmung der Toxophorbeladung von Cystein-verknüpften Konjugaten wurde über Reversed-Phase-Chromatographie des reduzierten und denaturierten ADCs bestimmt. Zur ADC-Lösung (1mg/mL, 50µL) wurde Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und über HPLC analysiert.

Die HPLC-Analyse wurde auf einem Agilent 1260 HPLC-System mit Detektion bei 220 nm durchgeführt. Es wurde eine Polymer Laboratories PLRP-S Polymeric Reversed Phase Säule (Katalognummer PL1912-3802) (2.1 x150 mm, 8 µm particle size, 1000 Å) bei einer Flussrate von 1 mL/min mit folgendem Gradienten verwendet: 0 min, 25 %B; 3 min, 25 %B; 28 min, 50 %B. Laufmittel A bestand aus 0.05 % Trifluoressigsäure (TFA) in Wasser, Laufmittel B aus 0.05 % Trifluoressigsäure in Acetonitril.

Die detektierten Peaks wurden durch Retentionszeitvergleich mit der leichten Kette (L0) und der schweren Kette (H0) des nicht konjugierten Antikörpers zugeordnet. Peaks, die ausschließlich in der konjugierten Probe detektiert wurden, wurden der leichten Kette mit einem Toxophor (L1) und den schweren Ketten mit einem, zwei und drei Toxophoren (H1, H2, H3) zugeordnet.

Die durchschnittliche Beladung des Antikörpers mit Toxophoren (genannt DAR, drug-to-antibody ratio) wurde aus den durch Integration bestimmten Peakflächen als die zweifache Summe der HC-Beladung und der LC-Beladung bestimmt, wobei sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks berechnete, und wobei sich die HC-Beladung aus der Summe der Toxophor-Anzahl gewichteten Integrationsergebnisse aller HC-Peaks geteilt durch die Summe der einfach gewichteten Integrationsergebnisse aller HC- Peaks berechnete. In vereinzelten Fällen konnte es vorkommen, dass die Toxophorbeladung aufgrund von Ko-Elutionen einiger Peaks nicht exakt möglich war.

In den Fällen, in denen keine ausreichende HPLC-Trennung von leichter und schwerer Kette möglich war, erfolgte die Bestimmung der Toxophorbeladung von Cystein verknüpften Konjugaten mittels massenspektrometrischer Bestimmung der Molekulargewichte der einzelnen Konjugatspezies an leichter und schwerer Kette.

Dazu wurde zur ADC-Lösung (1mg/mL, 50µL) Guanidinium-Hydrochlorid (GuHCl) (28.6 mg) und eine Lösung von DL-Dithiothreitol (DTT) (500mM, 3 µL) gegeben. Die Mischung wurde für eine Stunde bei 55 °C inkubiert und massenspektrometrisch nach online Entsaltzung mittels ESI-MicroTof_{Q} (Bruker Daltonik) analysiert.

Zur DAR-Bestimmung wurden alle Spektren über das Signal im TIC (Total Ion Chromatogramm) addiert und das Molekulargewicht der verschiedenen Konjugatspezies an leichter und schwerer Kette auf Basis von MaxEnt Deconvolution berechnet. Die durchschnittliche Beladung des Antikörpers mit Toxophoren wurde aus den durch Integration bestimmten Peakflächen als die zweifache Summe der HC-Beladung und der LC-Beladung bestimmt. Hierbei berechnete sich die LC-Beladung aus der Summe der Toxophor-Anzahl gewichteten Integrationsergebnisse aller LC-Peaks geteilt durch die Summe der einfach gewichteten Integrationsergebnisse aller LC- Peaks und die HC-Beladung aus der Summe der Toxophor-Anzahl gewichteten Integrationsergebnisse aller HC-Peaks geteilt durch die Summe der einfach gewichteten Integrationsergebnisse aller HC- Peaks.

Bei den geöffneten Konstukten wurde zur Bestimmung des Anteils des geöffneten Cystein-Addukts das Molekulargewichtsflächenverhältnis vom geschlossenen und offenen Cystein-Addukt (Molekulargewichtsdelta 18Dalton) aller einfach konjugierten leichten und schweren Kettenvarianten bestimmt. Der Mittelwert über alle Varianten ergab den Anteil des geöffneten Cystein-Addukts.

### B-8. Überprüfung der Antigen-Bindung des ADCs

Die Bindefähigkeit des Binders an das Zielmolekül wurde nach erfolgter Kopplung überprüft. Dazu sind dem Fachmann vielfältige Methoden bekannt, beispielsweise kann die Affinität des Konjugats mittels ELISA-Technologie oder Oberflächenplasmonresonanzanalyse (BIAcore™ Messungen) überprüft werden. Die Konjugatkonzentration kann der Fachmann mit gängigen Methoden messen, beispielsweise für Antikörper-Konjugate mittels Proteinbestimmung. (siehe auch Doronina et al.; Nature Biotechnol. 2003; 21:778-784 und Polson et al., Blood 2007; 1102:616-623).

### Ausführungsbeispiele ADCs

Die in den Strukturformen der Ausführungsbeispiele und der Referenzbeispiele dargestellten ADCs, die über Maleinimid-Reste an Cysteinseitenketten der Antikörper gekuppelt wurden, liegen in Abhängigkeit vom Linker und von dem Kupplungsprotokoll zum überwiegenden Teil in der dargestellten ring-geöffneten Form vor. Zu einem kleinen Anteil kann jedoch die ringgeschlossene Form in der Präparation enthalten sein.

### Beispiele 1:

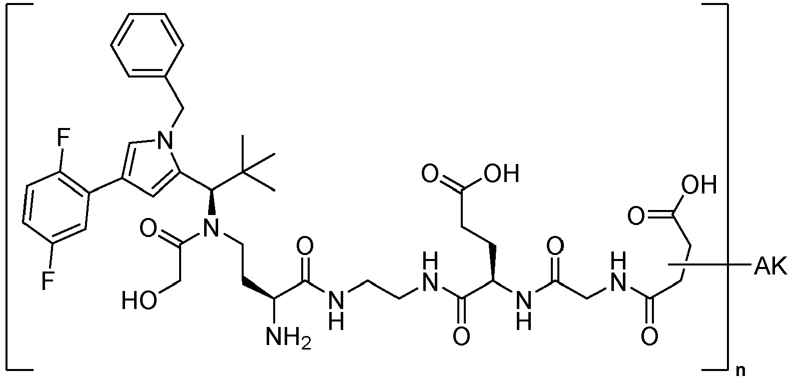

### Exemplarische Vorschrift A:

5 mg des entsprechenden Antikörpers in 0.5 ml PBS (c=10 mg/mL) wurden unter Argon mit einer Lösung aus 0.029 mg TCEP in 0.05 ml PBS-Puffer versetzt. Der Ansatz wurde 30min bei RT gerührt und anschließend wurden 0.26 mg (0.00023 mmol) von Intermediat F325 gelöst in 50 µl DMSO zugegeben. Nach weiteren 90 min Rühren bei RT wurde der Ansatz mit PBS-Puffer, der zuvor auf pH 8 eingestellt wurde, auf ein Volumen von 2.5 mL verdünnt, dann über eine mit PBS-Puffer pH8 equillibrierte PD 10-Säule (Sephadex^{®} G-25, GE Healthcare) gegeben und mit PBS-Puffer pH8 eluiert. Das Eluat wurde anschließend über Nacht bei RT unter Argon gerührt. Anschließend wurde durch Ultrazentrifugation aufkonzentriert und rückverdünnt mit PBS-Puffer (pH 7.2).

### Exemplarische Vorschrift B:

30 mg des entsprechenden Antikörpers in 3 ml PBS (c=10 mg/mL) wurden unter Argon mit einer Lösung aus 0.172 mg TCEP in 0.3 ml PBS-Puffer versetzt. Der Ansatz wurde 30min bei RT gerührt und anschließend wurden 1.57 mg (0.0014 mmol) von Intermediat F325 gelöst in 300 µl DMSO zugegeben. Nach weiteren 90 min Rühren bei RT wurde der Ansatz mit PBS-Puffer, der zuvor auf pH 8 eingestellt wurde, auf ein Volumen von 5 mL verdünnt, dann über eine mit PBS-Puffer pH8 equillibrierte PD 10-Säule (Sephadex^{®} G-25, GE Healthcare) gegeben und mit PBS-Puffer pH8 eluiert. Das Eluat wurde anschließend über Nacht bei RT unter Argon gerührt. Diese Lösung wurde dann über eine mit PBS-Puffer pH7.2 equillibrierte PD 10-Säule (Sephadex^{®} G-25, GE Healthcare) gegeben und mit PBS-Puffer pH7.2 eluiert. Anschließend wurde durch Ultrazentrifugation aufkonzentriert, rückverdünnt mit PBS-Puffer (pH 7.2), erneut aufkonzentriert und anschließend steril filtriert.

Folgende ADCs wurden in Analogie zu diesen Vorschriften hergestellt und wie in der Tabelle B angegeben charakterisiert:

**Tabelle B:**

| **Beispiel** | | **Target** | **Antikörper (TPP-)** | **Vorschrift** | **C [mg/mL]** | **DAR** |
|---|---|---|---|---|---|---|
| 1c-8987 | | CD123 | 8987 | B | 7.63 | 3.7 |
| 1c-8988 | | CD123 | 8988 | B | 9.10 | 3.5 |
| 1c-9476 | | CD123 | 9476 | B | 8.5 | 3.1 |
| 1x-9024 | | CXCR5 | 9024 | B | 8.76 | 3.8 |
| 1x-9574 | | CXCR5 | 9574 | B | 9.48 | 4.1 |
| 1x-9580 | | CXCR5 | 9580 | B | 9.25 | 4.2 |

### Ausführungsbeispiele Metabolite

### Beispiel M1

N-(3-{[(2R)-2-Amino-2-carboxyethyl]sulfanyl}-3-carboxypropanoyl)glycyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino] butanoyl}amino)ethyl]-D-alpha-glutamin -Trifluoressigsäure Salz

Regioisomer 1, Epimerengemisch

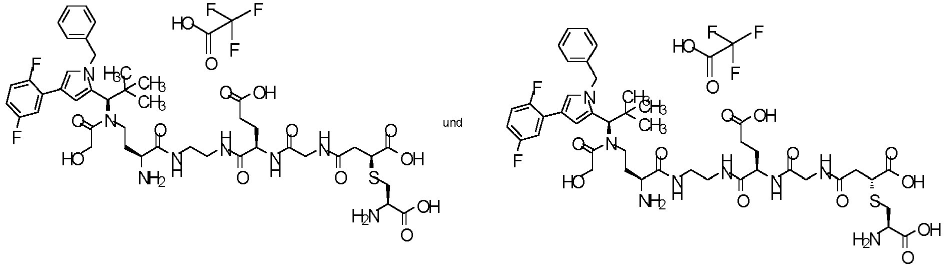

Zu einer Lösung von Methyl-L-cysteinathydrochlorid (1:1) (5.00 g, 29.1 mmol) in 1,4-Dioxan (200 ml) wurde Triethylamin (10 ml, 73 mmol) und anschließend 1-({[2-(Trimethylsilyl)ethoxy] carbonyl}oxy)pyrrolidin-2,5-dion (8.31 g, 32.0 mmol) gegeben. Die Reaktion wurde 20 h bei Raumtemperatur gerürht. Anschließend wurde der Feststoff abfiltriert und das Filtrat wurde im Hochvakkum eingeengt. Der Rückstand wurde über präparative HPLC gereinigt.

Zu einer Lösung des erhaltenen Methyl-N-{[2-(trimethylsilyl)ethoxy]carbonyl}-L-cysteinat (130 mg, 465 µmol) und 3-Brom-4-methoxy-4-oxobutansäure (393 mg, 1.86 mmol) in DMF (6.5 ml) wurden 210 µl (1.4 mmol) 1,8-Diazabicyclo(5.4.0)undec-7-en gegeben und die Reaktion wurde für 10 min bei Raumtemperatur gerührt. Anschließend wurde im Vakuum eingeengt und der Rückstand durch präparative HPLC gereinigt. Das Lösemittel wurde im Vakuum abgedampft und der Rückstand im Hochvakuum getrocknet.

Dieses erhaltene Intermediat wurde nach klassischen Methoden der Peptidchemie in Gegenwart von HATU mit Intermediat C119 gekuppelt. Anschließend wurden die Methylester durch Behandlung mit einer Lithiumhydroxid-Lösung in THF/Wasser (1:1) verseift.

Im letzten Schritt wurden 22 mg von dem erhaltenen Intermediat in 10 mL 2,2,2-Trifluorethanol gelöst. Es wurden 34 mg (0.252 mmol) Zinkchlorid zugegeben und der Ansatz 1 h bei 50°C gerührt. Anschließend wurden 74 mg (0.252 mmol) Ethylendiamin-N,N,N',N'-tetraessigsäure 10 mL Wasser und 500 µL TFA zugesetzt. Die Mischung wurde filtriert und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 13 mg (72% d.Th.) der Titelverbindung erhalten.
LC-MS (Methode 5): Rt = 2.44 min; MS (ESIneg): m/z = 959 [M-H]⁻

### Beispiel M2

N-(2-{[(2R)-2-Amino-2-carboxyethyl]sulfanyl}-3-carboxypropanoyl)glycyl-N-[2-({(2S)-2-amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]-D-alpha-glutamin-trifluoressigsäure Salz

Regioisomer 2, Epimerengemisch

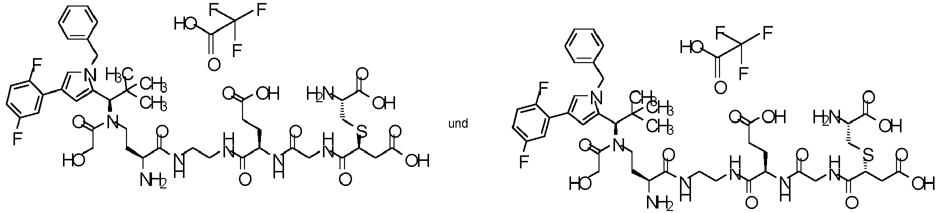

Die Titelverbindungen M2 wurden als Epimerengemisch analog Beispiel M1 hergestellt:
Zu einer Lösung aus Methyl-N-{[2-(trimethylsilyl)ethoxy]carbonyl}-L-cysteinat ( 1000 mg, 3.58 mmol) und 2-Brom-4-ethoxy-4-oxobutansäure ( 926 mg, 4.11 mmol) in DMF (40 ml) wurden801 µl, 5.4 mmol) 1,8-Diazabicyclo(5.4.0)undec-7-en gegeben und die Reaktion wurde für 2 h bei Raumtemperatur gerührt. Anschließend wurde im Vakuum eingeengt und der Rückstand durch präparative HPLC gereinigt.

Das erhaltene Intermediat wurde nach klassischen Methoden der Peptidchemie in Gegenwart von HATU und Methylmorpholin mit Intermediat C119 gekuppelt. Anschließend wurden der Methylester sowie der Ethylester durch Behandlung mit einer Lithiumhydroxid-Lösung in THF/Wasser (1:1) verseift.

Im letzten Schritt wurden 48 mg von diesem Intermediat in 5 mL 2,2,2-Trifluorethanol gelöst. Es wurden 75 mg (0.550 mmol) Zinkchlorid zugegeben und der Ansatz 3 h bei 50°C gerührt. Anschließend wurden 160 mg (0.550 mmol) Ethylendiamin-N,N,N',N'-tetraessigsäure 2 mL Wasser und 20 µL TFA zugesetzt. Das Lösemittel wurde im Vakuum eingeengt und der Rückstand wurde mittels präparativer HPLC gereinigt. Nach Einengen der entsprechenden Fraktionen und Lyophilisation des Rückstands aus Acetonitril/Wasser wurden 14 mg (39% d. Th.) der Titelverbindung erhalten.
LC-MS (Methode 5): Rt = 2.41 min; MS (ESIneg): m/z = 959 [M-H]⁻

Zu Vergleichszwecken wurden die Referenz-ADCs R1 hergestellt. Die Überlegenheit der erfindungsgemäßen ADCs aus Beispiel 1 gegenüber den entsprechenden Referenz-ADCs R1 werden im Teil C exemplarisch gezeigt.

### Referenzbeispiele R1

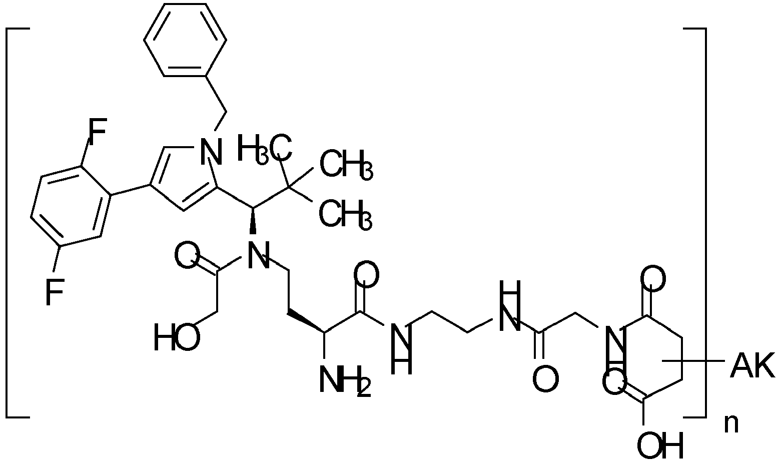

### Exemplarische Vorschrift

30 mg des entsprechenden AK in 3 mL PBS (c=10 mg/mL) wurden unter Argon mit einer Lösung aus 0.172 mg TCEP in 300 µl PBS-Puffer versetzt. Der Ansatz wurde 30min bei RT gerührt und anschließend wurden 1.291 mg (1.6 µmol) von Intermediat F104 gelöst in 300 µl DMSO zugegeben. Nach weiteren 90 min Rühren bei RT wurde der Ansatz mit 1.4 ml PBS-Puffer, der zuvor auf pH 8 eingestellt wurde, verdünnt.

Diese Lösung wurde dann über mit PBS-Puffer pH8 equillibrierte PD 10-Säulen (Sephadex^{®} G-25, GE Healthcare) gegeben und mit PBS-Puffer pH8 eluiert. Das Eluat wurde mit PBS-Puffer pH 8 auf ein Gesamtvolumen von 7.5 ml verdünnt. Diese Lösung wurde über Nacht bei RT unter Argon gerührt und anschließend wieder mittel PD-10 Säulen auf pH 7.2 umgepuffert. Das Eluat wurde auf 14 mL Gesamtvolumen verdünnt Anschließend wurde durch Ultrazentrifugation auf 2 mL aufkonzentriert, auf 14 mL rückverdünnt mit PBS-Puffer (pH 7.2) und erneut auf ein Volumen von 3 mL aufkonzentriert. Die Probe wird über ein Zentrifugen-Röhrchen (Microsep Advance Centrifugal Device 0,2µm Supor Membrane/Fa. PALL) filtriert. Der erhaltene ADC Batch wurde folgendermaßen charakterisiert:
Folgende ADCs wurden in Analogie zu diesen Vorschriften hergestellt und wie in der Tabelle C angegeben charakterisiert:

**Tabelle C:**

| **Beispiel** | **Target** | **Antikörper (TPP-)** | **C [mg/mL]** | **DAR** |
|---|---|---|---|---|
| R1c-8987 | CD123 | 8987 | 9.11 | 1.5 |
| R1c-8988 | CD123 | 8988 | 6.45 | 3.4 |
| R1c-9476 | CD123 | 9476 | 7.0 | 3.1 |
| R1x-9024 | CXCR5 | 9024 | 9.47 | 3.7 |
| R1x-9574 | CXCR5 | 9574 | 7.57 | 3.9 |
| R1x-9580 | CXCR5 | 9580 | 9.10 | 3.7 |

Ebenso wurden zu Vergleichszwecken die aus den Refenzbeispielen R1 gebildeten Metabolite Rm1 und Rm2 hergestellt:

### Referenzbeispiel Rm1

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-2-{[(2R)-2-amino-2-carboxyethyl]sulfanyl}-4-oxobutansäure -trifluoressigsäure Salz Regioisomer 1 als Epimerengemisch:

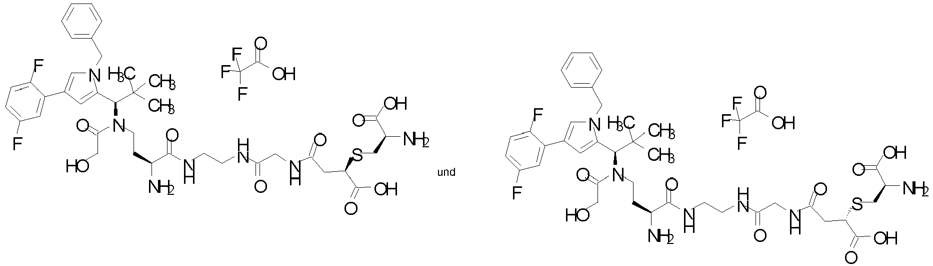

Zunächst wurde Methyl-L-cysteinathydrochlorid (1:1) mit 1-({[2-(Trimethylsilyl)ethoxy] carbonyl}oxy)pyrrolidine-2,5-dion in DMF in Gegenwart von *N,N*-Diisopropylethylamin in Methyl-N-{[2-(trimethylsilyl)ethoxy]carbonyl}-L-cysteinat überführt.

Zu einer Lösung aus Methyl-N-{[2-(trimethylsilyl)ethoxy]carbonyl}-L-cysteinat (130 mg, 465 µmol) und 3-Brom-4-methoxy-4-oxobutansäure (393 mg, 1.86 mmol) in DMF (6.5 ml) wurde portionsweiße (208 µl, 1.4 mmol) 1,8-Diazabicyclo(5.4.0)undec-7-en gegeben und die Reaktion wurde für 10 min bei Raumtemperatur gerührt. Anschließend wurde im Vakuum eingeengt und der Rückstand durch präparative HPLC gereinigt. Das Lösemittel wurde im Vakuum abgedampft und der Rückstand im Hochvakuum getrocknet.

Das erhaltene Intermediat wurde nach klassischen Methoden der Peptidchemie in Gegenwart von HATU mit Intermediat C66 gekuppelt. Anschließend wurden die Methylester durch Behandlung mit einer Lithiumhydroxid-Lösung in THF/Wasser (1:1) verseift.

Im letzten Schritt wurden 18 mg von diesem Intermediat in 10.6 mL 2,2,2-Trifluorethanol gelöst. Es wurden 22 mg (0.16 mmol) Zinkchlorid zugegeben und der Ansatz 2 h bei 50°C gerührt. Anschließend wurden 47 mg (0.16 mmol) Ethylendiamin-N,N,N',N'-tetraessigsäure und 2 mL Wasser und 2-3 Tropfen TFA zugesetzt. Die Mischung wurde filtriert und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 10.5 mg (78.5% d.Th.) der Titelverbindung (Isomer 2 als Regioisomerengemisch erhalten.
LC-MS (Methode 5): Rt = 2.43 min; MS (ESI-pos): m/z = 832 [M+H]⁺

### Referenzbeispiel Rm2

4-[(2-{[2-({(2S)-2-Amino-4-[{(1R)-1-[1-benzyl-4-(2,5-difluorphenyl)-1H-pyrrol-2-yl]-2,2-dimethylpropyl}(glycoloyl)amino]butanoyl}amino)ethyl]amino}-2-oxoethyl)amino]-3-{[(2R)-2-amino-2-carboxyethyl]sulfanyl}-4-oxobutansäure-Trifluoressigsäure Salz Isomer 2 als Epimerengemisch:

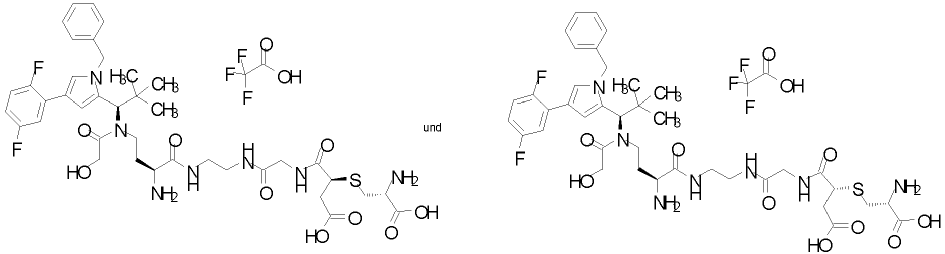

Zunächst wurde Methyl-L-cysteinathydrochlorid (1:1) mit 1-({[2-(Trimethylsilyl)ethoxy] carbonyl}oxy)pyrrolidin-2,5-dion in DMF in Gegenwart von *N,N*-Diisopropylethylamin in Methyl-N-{[2-(trimethylsilyl)ethoxy]carbonyl}-L-cysteinat überführt.

Zu einer Lösung aus Methyl-N-{[2-(trimethylsilyl)ethoxy]carbonyl}-L-cysteinat ( 1000 mg, 3.58 mmol) und 2-Brom-4-ethoxy-4-oxobutansäure ( 926 mg, 4.11 mmol) in DMF (40 ml) wurden 801 µl (5.4 mmol) 1,8-Diazabicyclo(5.4.0)undec-7-en gegeben und die Reaktion wurde für 2 h bei Raumtemperatur gerührt. Anschließend wurde im Vakuum eingeengt und der Rückstand durch präparative HPLC gereinigt. Das Lösemittel wurde im Vakuum abgedampft und der Rückstand im Hochvakuum getrocknet.

Das erhaltene Intermediat wurde nach klassischen Methoden der Peptidchemie in Gegenwart von HATU mit Intermediat C66 gekuppelt. Anschließend wurden der Methylester und der Ethylester durch Behandlung mit einer Lithiumhydroxid-Lösung in THF/Wasser (1:1) verseift.

Im letzten Schritt wurden 24 mg von diesem Intermediat in 6.4 mL 2,2,2-Trifluorethanol gelöst. Es wurden 28.5 mg (0.21 mmol) Zinkchlorid zugegeben und der Ansatz 2 h bei 50°C gerührt. Anschließend wurden 61 mg (0.21 mmol) Ethylendiamin-N,N,N',N'-tetraessigsäure und 2 mL Wasser und 2-3 Tropfen TFA zugesetzt. Die Mischung wurde filtriert und das Lösemittel im Vakuum verdampft. Der Rückstand wurde mittels präparativer HPLC gereinigt. Nach Einengen der entsprechenden Fraktionen und Lyophilisation des Rückstands aus Acetonitril/Wasser wurden 14.5 mg (71% d.Th.) der Titelverbindung erhalten.
LC-MS (Methode 5): Rt = 2.41 min; MS (ESI-pos): m/z = 832 [M+H]⁺

### C: Bewertung der biologischen Wirksamkeit

Die biologische Wirkung der erfindungsgemäßen Verbindungen kann durch die nachstehend beschriebenen Assays gezeigt werden:

### C-1a: Bestimmung der zytotoxischen Wirkung der gegen CD123 und CXCR5 gerichteten ADCs

Die Analyse der cytototoxischen Wirkung der beispielhaften ADCs erfolgt auf verschiedenen Zelllinien:
NCI-H292: humane mukoepidermoide Lungenkarzinomzellen, ATCC-CRL-1848, Standardmedium: RPMI 1640 (Biochrom; #FG1215, stab. Glutamin) + 10% FCS (Sigma #F2442), TWEAKR-positiv,
KPL4: humane Brustkrebszelllinie, Bayer Pharma AG (identity checked and confirmed on 19.7.2012 at DSMZ), Standardmedium: RPMI 1640 (Fa. Gibco; #21875- 059, stab. L-Glutamin) + 10% heat inactivated FCS (Fa. Gibco, No. 10500-064); HER2-positiv.
SK-HEP-1: humane Leberkrebszelllinie, ATCC No. HTB-52, Standardmedium: MEM mit Earle's Salzen + Glutamax I (Invitrogen 41090) + 10% heat inactivated FCS (Fa. Gibco, No. 10500-064); TWEAKR positiv
MOLM-13: humane akute monozytäre Leukämiezellen (AML-M5a), DSMZ, No. ACC 554, Standardmedium: RPMI 1640 (Fa. gibco; #21875- 059, stab. L-Glutamin) + 20% heat inactivated FCS (Fa. Gibco, No. 10500-064); CD123-positiv.
MV-4-11: humane biphenotypische B myelomonozytische Leukämiezellen gewonnen aus peripheren Blut, ATCC-CRL-9591, Standardmedium: IMDM (ATCC:30-2005), + 10% heat inactivated FCS (Gibco, No. 10500-064); CD123-positiv
NB4: humane akute promyelozytische Leukämiezellen gewonnen aus Knochenmark , DSMZ , No. ACC 207, Standardmedium: RPMI 1640 + GlutaMAX I (Invitrogen 61870) + 10% heat inactivated FCS (Gibco, No. 10500-064) + 2.5 g of Glukose (20% Glucose Lösung, Gibco, No.19002) + 10 mM Hepes (Invitrogen 15630) + 1 mM Sodium Pyruvat (Invitrogen 11360) ; CD123-negativ
Rec-1: humane Mantelzell Lymphomzellen (B Zell non-Hodgkin's Lymphoma) ATCC CRL-3004, Standardmedium: RPMI 1640 + GlutaMAX I (Invitrogen 61870) + 10% heat inactivated FCS (Gibco, No. 10500-064) CXCR5-positiv
Die Kultivierung der Zellen erfolgt nach Standard-Methode, wie bei der American Tissue Culture Collection (ATCC) oder des Leibniz-Institut DSMZ-Deutsche Sammlung von Mikroorganismen und Zellkulturen GmbH (DSMZ) für die jeweiligen Zelllinien angegeben.

### MTT-Assay

Die Kultivierung der Zellen erfolgte nach Standardmethode, mit den in Kapitel C-1 angegebenen Wachstumsmedien. Zur Durchführung wurden die Zellen mit einer Lösung von Accutase in PBS (Fa. Biochrom AG #L2143) abgelöst, pelletiert, in Kulturmedium resuspendiert, gezählt und in eine 96-Loch Kulturplatte mit weißem Boden (Fa. Costar #3610) ausgesät (NCI H292: 2500 Zellen/well; SK-HEP-1: 1000 Zellen/well; KPL-4: 1200 Zellen/well; in 100µL Gesamtvolumen). Anschließend wurden die Zellen im Brutschrank bei 37°C und 5% Kohlendioxid inkubiert. Nach 48h wurde ein Mediumwechsel durchgeführt. Dann wurden die Antikörper-Wirkstoff-Konjugate in 10µl Kulturmedium in Konzentrationen von 10⁻⁵M bis 10⁻¹³M zu den Zellen (Triplikate) pipettiert, bevor der Ansatz im Brutschrank bei 37°C und 5% Kohlendioxid inkubierte. Die Suspensionszellen wurden gezählt und in eine 96-Loch Kulturplatte mit weißem Boden (Fa. Costar #3610) ausgesät (#3610) (MOLM-13: 2000 Zellen/well; NB4: 7000 Zellen/well; MV-4-11: 5000 Zellen/well in einem Gesamtvolumen von 100 µl). Nach 6-stündiger Inkubation im Inkubator bei 37°C und 5% Kohlendioxid wurde das Medium gewechselt und die Antikörper-Wirkstoff Konjugate oder Metabolite in 10µl Kulturmedium in Konzentrationen von 10⁻⁵M bis 10⁻¹³M zu den Zellen (Triplikate) in 90µl zupipettiert. Die Inkubation des Ansatzes im Inkubator erfolgte bei 37°C und 5% Kohlendioxid. Nach 96h erfolgte die Detektion der Zellproliferation mit Hilfe des MTT-Assays (ATCC, Manassas, Virginia, USA, Katalog-Nr. 30-1010K). Hierzu wurde das MTT Reagens für 4h mit den Zellen inkubiert, bevor durch Zugabe des Detergenzes die Lyse der Zellen über Nacht erfolgte. Die Detektion des gebildeten Farbstoffs erfolgte bei 570nm (Infinite M1000 pro, Fa. Tecan). Aus den gemessenen Daten wurde die IC₅₀ der Wachstumshemmung unter Verwendung der DRC (Dose Response Curve) berechnet. Die Proliferation ohne Testsubstanz, aber ansonsten identisch behandelten Zellen, wird als 100% Wert definiert.

In der folgenden Tabelle 1a sind die IC₅₀-Werte repräsentativer Ausführungsbeispiele aus diesem Assay aufgeführt:

**Tabelle 1a**

| **Beispiel** | **MV-4-11 IC₅₀ [M]** | **MOLM-13 IC₅₀ [M]** | **Rec-1 IC₅₀ [M]** |
|---|---|---|---|
| 1c-8987 | 1.26E-09 | 2.10E-10 | |
| 1c-8988 | 2.94E-08 | 3.02E-11 | |
| 1c-9476 | 2.78E-09 | 2.85E-10 | |
| 1x-9024 | | | 1.34E-10 |
| 1x-9574 | | | 2.94E-11 |
| 1x-9580 | | | 2.78E-09 |

In Tabelle 1b sind die IC₅₀-Werte repräsentativer Referenzbeispiele aus diesem Assay aufgeführt:

**Tabelle 1b**

| **Beispiel** | **MV-4-11 IC₅₀ [M]** | **MOLM-13 IC₅₀ [M]** | **Rec-1 IC₅₀ [M]** |
|---|---|---|---|
| R1c-8987 | 1.33E-07 | 7.57E-08 | |
| R1c-8988 | 3.27E-08 | 4.26E-09 | |
| R1c-9476 | 5.30E-09 | 3.04E-10 | |
| R1x-9024 | | | 1.84E-10 |
| R1x-9574 | | | 2.62E-10 |
| R1x-9580 | | | 7.16E-11 |

Die angegebenen Wirkdaten beziehen sich auf die im vorliegenden experimentellen Teil beschriebenen Ausführungsbeispiele mit den angegebenen Drug/mAB-Ratios. Die Werte können bei anderen Drug/mAB-Ratios gegebenenfalls abweichen. Bei den IC50-Werten handelt es sich um Mittelwerte aus mehreren unabhängigen Exprimenten oder um Einfachwerte. Die Wirkung der Antikörper-Wirkstoffkonjugate war selektiv versus der jeweiligen Isotypkontrolle, die den jeweils entsprechenden Linker und Toxophor enthielt. Zusätzlich wurde die Targetspezifität der Antikörper-Wirkstoff Konjugate, die gegen CD123 gerichtet waren, durch Testung an einer CD123 negativen Zelle (NB4) gezeigt.

### C-1b; Bestimmung der Inhibition des Kinesinspindelproteins KSP/ Eg5 durch ausgewählte Beispiele

Die Motordomäne des humanen Kinesinspindelproteins KSP /Eg5 (Fa. tebu-bio/ Cytoskeleton Inc, No. 027EG01-XL) wurde in einer Konzentration von 10nM mit 50µg/mL Taxol (Fa. Sigma No. T7191-5MG) stabilisierten Microtubuli (bovine oder porcine, Fa. tebu-bio/ Cytoskeleton Inc) für 5 min bei RT in 15mM PIPES, pH 6,8 (5mM MgCl₂ und 10mM DTT, Fa. Sigma) inkubiert. Die frisch hergestellte Mischung wurde in eine 384 MTP (Fa. Corning) aliquotiert. Es folgte die Zugabe der zu untersuchenden Inhibitoren in Konzentration von 1.0x10-6M bis 1.0x 10-13M und ATP (finale Konzentration 500µM; Fa. Sigma). Die Inkubation erfolgte über 2h bei RT. Die ATPase-Aktivität wurde durch den Nachweis des entstehenden anorganischen Phosphats mit Malachit-Grün detektiert (Fa. Biomol). Nach der Zugabe des Reagenz erfolgte eine 50 min Inkubation bei RT, bevor die Detektion der Absorption bei einer Wellenlänge von 620nm erfolgte. Als Positivkontrolle wurden Monastrol (Fa. Sigma, M8515-1mg) und Ispinesib (Fa. AdooQ Bioscience A10486) verwendet. Die Einzeldaten der Dosis-Wirkungskurve stellen achtfach Bestimmungen dar. Bei den IC₅₀-Werten handelt es sich um Mittelwerte aus zwei unabhängigen Experimenten. Als 100% Kontrolle diente die nicht mit Inhibitoren behandelte Probe.

In der folgenden Tabelle 2 sind die IC₅₀-Werte repräsentativer Ausführungsbeispiele aus dem beschriebenen Assay und die korrespondierenden zytotoxischen Daten (MTT-Assay) zusammengefasst:

**Tabelle 2**

| **Beispiel** | **KSP-Assay IC₅₀ [M]** | **NCI-H292 IC₅₀ [M]** | **SKHep-1 IC₅₀ [M]** | **KPL-4 IC₅₀ [M]** | **MV 4--11 IC₅₀ [M]** | **MOLM-**13 **IC₅₀ [M]** |
|---|---|---|---|---|---|---|
| Rm1 | 9.44E-10 | 6.36E-08 | 1.90E-08 | | | |
| M1 | 2.13E-09 | 5.00E-07 | 9.88E-08 | 5.00E-07 | 3.02E-07 | 1.25E-07 |
| Rm2 | 2.03E-09 | 2.76E-07 | 8.90E-08 | | | |
| M2 | 4.67E-10 | | | | 1.85E-07 | 2.15E-07 |

Die angegebenen Wirkdaten beziehen sich auf die im vorliegenden experimentellen Teil beschriebenen Ausführungsbeispiele.

### C-2a Internalisierungsassay

Internalisierung ist der Schüsselprozess, um eine spezifische und effiziente Bereitstellung der zytotoxischen Payload in Antigen-exprimierenden Krebszellen durch Antikörper-Drug-Konjugate (ADC) zu ermöglichen. Dieser Prozess wird über Fluoreszenzmarkierung von spezifischen Antikörpern und einem Isotyp-Kontrollantikörper verfolgt. Hierzu wurde zunächst die Konjugation des Fluoreszenzfarbstoffes an Lysine des Antikörpers durchgeführt. Die Konjugation erfolgte mit zweifach molarem Überschuss von CypHer 5E mono NHS ester (Batch 357392, GE Healthcare) bei pH 8,3. Nach erfolgter Kupplung wurde die Reaktionsmischung gelchromatographisch aufgereinigt (Zeba Spin Desalting Columns, 40K, Fa. Thermo Scientific, No. 87768; Elutionspuffer: DULBECCO'S PBS, Fa. Sima-Aldrich, No. D8537), um überschüssigen Farbstoff zu eliminieren und den pH-Wert zu adjustieren. Die Ankonzentrierung der Proteinlösung erfolgte mittels VIVASPIN 500 Säulen (Fa Sartorius stedim biotec). Die Bestimmung der dye load des Antikörpers erfolgte mittels spektrophotometrischer Analyse (Fa. NanoDrop) und anschließender Berechnung (D/P = A_{dye} εₚᵣₒₜₑᵢₙ:(A₂₈₀-0,16A_{dye})ε_{dye}).

Die dye load der hier untersuchten Antikörpern sowie der Isotyp-Kontrolle lagen in vergleichbarer Größenordnung. In Zellbindungs-Assays wurde getestet, dass die Kupplung zu keiner Affinitätsänderung der Antikörper führte.

Die markierten Antikörper wurden im Internalisierungs-Assays eingesetzt. Vor dem Behandlungsstart wurden Zellen (2×10⁴/well) in 100µL Medium in einer 96-MTP ausgesät (fat, black, clear bottom No 4308776, Fa. Applied Biosystems). Nach 18h Inkubation bei 37°C/5%CO₂ wurde das Medium gewechselt und markierte Antikörper in variierender Konzentration hinzugefügt (10, 5, 2.5, 1, 0.1µg/mL). Das gleiche Behandlungsschema erfolgte mit der markierten Isotyp-Kontrolle (negative control). Die gewählten Inkubationszeiten waren 0h, 0,25h, 0,5h, 1h, 1,5h 2h, 3h, 6h and 24h. Die Fluoreszenz- Messung wurde mit Hilfe des InCellAnalyzer 1000 (Fa. GE Healthcare) durchgeführt. Es erfolgte eine kinetische Evaluierung über die Messung der Parameter granule counts/cell und totale granule intensity/cell.

Die Antikörper wurden nach Bindung an den Rezeptor auf ihre Internalisierungsfähigkeit hin untersucht. Hierzu wurden Tumorzellen mit verschiedenen Expressionsleveln des Rezeptors gewählt. Es konnte Target-vermittelte hochspezifische Internalisierung mit den Antikörpern der Erfindung beobachtet werden, wohingegen die Isotyp-Kontrolle keine Internalisierung zeigte.

### C-2b: Internalisierungsassay mit Suspensionszellen

Die Kupplung des Fluoreszenzfarbstoffs erfolgte wie in Kapitel C-2 beschrieben. Das zu untersuchende Antigen wird von hämatopoetischen Suspensionszellen exprimiert, deshalb wurde die Internalisierung in einem FACS basierten Internalisierungsassay untersucht.

Es wurden Zellen mit verschiedenen Target Expressionslevel untersucht. Die Zellen (5x10⁴/well) wurden in eine 96-MTP (Greiner bio-one, CELLSTAR, 650 180, U-bottom). in 100 µl Gesamtvolumen ausgesät. Nach Zugabe des targetspezifischen Antikörpers in einer Endkonzentration von 10µg/ml, wurden die Ansätze bei 37°C unterschiedlich lang inkubiert (1h, 2h, 6h, Dreifachbestimmung). Die Isotyp-Kontrolle wurde unter identischen Bedingungen behandelt..Ein paralleler Ansatz wurde konstant bei 4°C behandelt.und inkubiert (negative Kontrolle). Die FACS Analyse wurde mit Hilfe des Guava flow Cytometers (Millipore) durchgeführt.. Die kinetische Evaluierung erfolgte über Messung der Fluoreszenzintensität, und die Auswertung wurde mit Hilfe der guavaSoft 2.6 software (Millipore) durchgeführt. Für die hier beschriebenen Targets und targetspezifischen Antikörper konnte eine signifikante und spezifische Internalisierung in verschiedenen Zellen detektiert werden, Die Isotyp-Kontrollen zeigten keine Internalisierung.

### C-2c: Co-Lokalisation: Untersuchungen der anti- CD123 Antikörper

Die Generation des aktiven Metaboliten des Antikörper-Wirkstoff Konjugates erfolgt aufgrund des Linkers durch lysosomale Degradation. Dementsprechend ist das intrazelluläre trafficking nach erfolgter Internalisierung von essentieller Bedeutung. Die Untersuchungen zur Co-Lokalisation des Antikörpers mit Markern spezifisch für das lysosomale Organell (z.B.. Oberflächenmoleküle oder kleine GTPasen), ermöglichen die Selektion von Antikörpern mit gewünschtem Profil. Hierzu wurden target positive Zellen (5x10⁴/well) in 100 µl Gesamtvolumen in einer 96-MTP (Greiner bio-one, CELLSTAR, 650 180, U-bottom) ausgesät. Nach Zugabe des CypHer5E-markierten anti-target Antikörpers (finale Konzentration 20 µg/ml), wurden die Ansätze (Dublikate pro Zeitpunkt) bei 37°C für 30min, 2h und 6h im Inkubator (5%CO₂) inkubiert.. 30 min vor Beendigung der gewählten Inkubationszeit wurde den zu untersuchenden Ansätzen der Lysosom spezifische Marker hinzugefügt. Die Lysosomen wurden mit CytoPainter LysoGreen indicator reagent (finale Konzentration 1:2000; abcam, ab176826) gefärbt.. Nach der Inkubation wurden 200 µl eiskalter FACS Puffer (DULBECCO'S PBS, Sigma-Aldrich, No. D8537 + 3 % FBS heat inactivated FBS, Gibco, No. 10500-064) hinzugefügt und die Zellsuspension bei 400 x g, 4°C für 5 min zentrifugiert. Das Zellpellet wurde in 300µl eiskaltem FACS-Puffer resuspendiert und erneuet zentrifugiert (4 min, 400 x g at 4°C). Nach erfolgter Zentrifugation wurde der Überstand verworfen und das Zellpellet in 30 µl eiskalten FACS Puffer aufgenommen. Die Proben wurden dann einer sofortigen FACS/image Analyse (FlowSight amnis, Millipore) unterzogen. Die Co-Lokalisation wurde mittels einer spezifischen Software ausgewertet (Co-Lokalisation software IDEAS Application v6.1). In Tablle 3 sind die Ergebnisse aus diesem Assay für Beispiele der anti-CD123 Antikörper zusammengefasst.

**Tabelle 3**

| **Beispiel** | **Co-localisation [%]** |
|---|---|
| TPP-9476 | 29 |
| TPP-8987 | 28 |
| TPP-8988 | 41 |
| 7G3 | 10 |
| Isotype control | 0.2 |

Die Antikörper TPP-8987 und TPP-9476 zeigen ein deutlich verbessertes Profil im Vergleich zu dem parentalen murinen Antikörper.

### C-3 In vitro Tests zur Bestimmung der Zell-Permeabilität

Die Zell-Permeabilität einer Substanz kann mittels *in vitro*-Testung in einem Flux-Assay unter Verwendung von Caco-2-Zellen untersucht werden [M.D. Troutman und D.R. Thakker, Pharm. Res. 20 (8), 1210-1224 (2003)]. Hierzu wurden die Zellen auf 24-Loch-Filterplatten für 15-16 Tage kultiviert. Zur Bestimmung der Permeation wurde die jeweilige Testsubstanz in einem HEPES-Puffer entweder apikal (A) oder basal (B) auf die Zellen gegeben und für 2 h inkubiert. Nach 0 h und nach 2 h wurden Proben aus den Cis- und Transkompartimenten entnommen. Die Proben wurden mittels HPLC (Agilent 1200, Böblingen, Deutschland) unter Verwendung von reverse phase-Säulen aufgetrennt. Das HPLC-System war über ein Turbo Ion Spray-Interface an ein Triple Quadropol-Massenspektrometer API 4000 (AB SCIEX Deutschland GmbH, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als aktiv transportiert klassifiziert, wenn das Verhältnis von Pₐₚₚ (B-A) zu Pₐₚₚ (A-B) (efflux ratio) >2 oder <0.5 war.

Von entscheidender Bedeutung für Toxophore, die intrazellulär freigesetzt werden, sind die Permeabilität von B nach A [Pₐₚₚ (B-A)] und das Verhältnis von Pₐₚₚ (B-A) zu Pₐₚₚ (A-B) (efflux ratio): Je niedriger diese Permeabilität ist, desto langsamer sind die aktiven und passiven Transportvorgänge der Substanz durch die Monoschicht von Caco-2-Zellen. Gibt das efflux ratio zudem keine Hinweise auf aktiven Transport, kann die Substanz nach intrazellulärer Freisetzung länger in der Zelle verweilen. Damit steigt auch die Zeit, die für eine Interaktion mit dem biochemischen Target (hier: Kinesin-Spindelprotein, KSP / Eg5) zur Verfügung steht.

In der folgenden Tabelle 4 sind Permeabilitätsdaten repräsentativer Ausführungsbeispiele aus diesem Assay aufgeführt:

**Tabelle 4**

| **Ausführungsbeispiel** | **Pₐₚₚ (B-A) [nm/s]** | **Efflux ratio** |
|---|---|---|
| M1 | 1.2 | 0.8 |
| M2 | 1.1 | 1.6 |
| Rm1 | 13.0 | 9.6 |
| Rm2 | 13.2 | 11.9 |

Die Metabolite M1 und M2, die aus den erfindungsgemäßen ADCs in Beispiel 1 gebildet werden können, zeigen sowohl einen deutlich reduzierten Transport aus der Zelle als auch eine reduzierte Efflux-Ratio gegenüber den Referenzmetaboliten RM1 und RM2 auf, die aus dem ADC in Referenzbeispiel 1 gebildet werden.

### C-4 In vitro Tests zur Bestimmung der Substrateigenschaften für P-Glycoprotein (P-gρ)

Viele Tumorzellen exprimieren Transporterproteine für Wirkstoffe, was häufig mit einer Resistenzentwicklung gegenüber Cytostatika einhergeht. Substanzen, die keine Substrate von solchen Transporterproteinen wie beispielsweise P-Glycoprotein (P-gp) oder BCRP sind, könnten somit ein verbessertes Wirkprofil aufzeigen.

Die Substrateigenschaften einer Substanz für P-gp (ABCB1) wurden mittels eines Flux-Assays unter Verwendung von LLC-PK1-Zellen, die P-gp überexprimieren (L-MDR1-Zellen), bestimmt [A.H. Schinkel et al., J. Clin. Invest. 96, 1698-1705 (1995)]. Hierzu wurden die LLC-PK1- oder L-MDR1-Zellen auf 96-Loch-Filterplatten für 3-4 Tage kultiviert. Zur Bestimmung der Permeation wurde die jeweilige Testsubstanz allein oder in Gegenwart eines Inhibitors (wie z.B. Ivermectin oder Verapamil) in einem HEPES-Puffer entweder apikal (A) oder basal (B) auf die Zellen gegeben und für 2 h inkubiert. Nach 0 h und nach 2 h wurden Proben aus den Cisund Transkompartimenten entnommen. Die Proben wurden mittels HPLC unter Verwendung von reverse phase-Säulen aufgetrennt. Das HPLC-System war über ein Turbo Ion Spray-Interface an ein Triple Quadropol-Massenspektrometer API 3000 (Applied Biosystems Applera, Darmstadt, Deutschland) gekoppelt. Die Permeabilität wurde anhand eines Pₐₚₚ-Wertes bewertet, welcher mittels der von Schwab *et al.* publizierten Formel berechnet wurde [D. Schwab et al., J. Med. Chem. 46, 1716-1725 (2003)]. Eine Substanz wurde als P-gp-Substrat klassifiziert, wenn das Efflux-Verhältnis Pₐₚₚ (B-A) zu Pₐₚₚ (A-B) >2 war.

Als weitere Kriterien zur Bewertung der P-gp-Substrateigenschaften können die Efflux-Verhältnisse in L-MDR1- und LLC-PK1-Zellen oder das Efflux-Verhältnis in An- oder Abwesenheit eines Inhibitors miteinander verglichen werden. Wenn sich diese Werte um mehr als einen Faktor 2 unterscheiden, so handelt es sich bei der betreffenden Substanz um ein P-gp-Substrat.

### C-5a: Identifizierung der ADC-Metabolite nach Internalisierung in vitro

### Methodenbeschreibung:

Internalisierungsuntersuchungen mit Immunkonjugaten wurden durchgeführt, um intrazellulär entstandene Metabolite zu analysieren. Hierzu wurden humane Lungentumorzellen NCI H292 (3x10⁵/well) in 6-well Platten ausgesät und über Nacht inkubiert (37 °C, 5% CO₂). Es erfolgte eine Behandlung mit 10 µg/mL (66 nM) des zu untersuchenden ADCs. Die Internalisierung wurde bei 37 °C und 5% CO₂ durchgeführt. Zu verschiedenen Zeitpunkten (0, 4, 24, 48, 72h) wurden Zellproben zur weiteren Analyse genommen. Zunächst wurden die Überstände (ca. 5 mL) geerntet und nach erfolgter Zentrifugation (2 min, RT, 1000 rpm Heraeus Variofuge 3.0R) bei -80 °C gelagert. Die Zellen wurden mit PBS gewaschen, mit Accutase abgelöst und die Zellzahl bestimmt. Nach erneutem Waschen wurde eine definierte Zellzahl (2×10⁵) mit 100 mL Lysis Puffer (Mammalian Cell Lysis Kit (Sigma MCL1) versetzt und unter ständigem Schütteln (Thermomixer, 15min, 4°C, 650 rpm) in Protein LoBind tubes (eppendorf Cat.No. 0030 108.116) inkubiert. Nach der Inkubation wurde das Lysat zentrifugiert (10min, 4°C, 12000g, eppendorf 5415R) und der Überstand geerntet. Der gewonnene Überstand wurde bei -80 °C gelagert. Alle Proben wurden anschließend wie folgt analysiert.

Die Messung der Verbindungen im Kulturüberstand bzw. Zelllysat erfolgte nach Fällung der Proteine mit Methanol oder Acetonitril durch eine Hochdruck-Flüssigkeits-Chromatographie (HPLC) gekoppelt an ein Triple-Quadrupol-Massenspektrometer (MS).

Zur Aufarbeitung von 50 µL Kulturüberstand/Zelllysat werden diese mit 150 µL Fällungsreagenz (Methanol) versetzt und für 10 Sekunden geschüttelt. Das Fällungsreagenz enthält einen internen Standard (ISTD) in geeigneter Konzentration (in der Regel im Bereich 20-100 µg/L). Nach dem 10minütigen Zentrifugieren bei 1881g wird der Überstand in ein Autosampler-Vial überführt, mit 300 µL eines auf das Laufmittel abgestimmten Puffers aufgefüllt und erneut geschüttelt und 10 min bei 1881g zentrifugiert.

Die Messung der Zelllysat- und Überstandsproben erfolgt schließlich an dem mit einer HPLC gekoppelten Triple-Quadrupol-Massenspektrometer API4200 der Firma AB SCIEX Deutschland GmbH.

Zur Kalibrierung wird Leerlysat bzw. Leerüberstand mit entsprechenden Konzentrationen (0.1 - 1000 µg/L) versetzt. Die Nachweisgrenze (LLOQ) liegt bei ca. 0.2 µg/L.

Qualitätskontrollen zur Gültigkeitsprüfung enthalten 4 und 40 µg/L.

### C-5b: Identifizierung der ADC-Metabolite in vivo

### Analytik zur Quantifizierung der potentiell auftretenden Metabolite

Nach i.v. Applikation von 10 mg/kg verschiedener erfindungsgemäßer Konjugate in Xenograft Mäuse können 24h nach Applikation dieser Konjugate, die Plasma-, Tumor-, Leber-, Milz- und Nierenkonzentrationen des Antikörpers sowie potentiell auftretenden Metabolite gemessen werden. Unter C-6 ist eine genauere Methodenbeschreibung bezüglich der Xenograft Modelle zu finden. Hier soll nur auf die Metabolitenkonzentrationen der erfindungsgemäßen Konjugate eingegangen werden. Die Messwerte der Metabolite in den genannten Matrizes geben darüber Aufschluss, wie stark die Belastung mit Metabolit in Plasma, Niere, Milz und Leber ausgeprägt ist im Vergleich zur Belastung im Tumor.

### Analytik zur Quantifizierung der potentiell auftretenden Metabolite

Die Messung der Verbindungen in Plasma, Tumor, Leber, Milz und Niere erfolgt nach Fällung der Proteine mit in der Regel Methanol durch eine Hochdruck-Flüssigkeits-Chromatographie (HPLC) gekoppelt an ein Triple-Quadrupol-Massenspektrometer (MS).

Zur Aufarbeitung von 50 µL Plasma werden diese mit 150 µL Fällungsreagenz (in der Regel Methanol) versetzt und für 10 sec geschüttelt. Das Fällungsreagenz enthält einen internen Standard (ISTD) in geeigneter Konzentration (in der Regel im Bereich 20-100 µg/ L). Nach dem 10minütigen Zentrifugieren bei 1881g wird der Überstand in ein Autosampler-Vial überführt, mit 300 µL eines auf das Laufmittel abgestimmten Puffers aufgefüllt und erneut geschüttelt.

Bei der Aufarbeitung von Tumor- oder Organmaterial wird das jeweilige Material mit der 3-20 fachen Menge an Extraktionspuffer versetzt. Der Extraktionspuffer enthält 50 mL Tissue Protein Extraction Reagent (Pierce, Rockford, IL), zwei Pellets Complete-Protease-Inhibitor-Cocktail (Roche Diagnostics GmbH, Mannheim, Deutschland) und Phenylmethylsulfonylfluorid (Sigma, St. Louis, MO) in einer finaler Konzentration von 1 mM. Je nach Gewebetyp (hart: Tumor; weich: Leber, Niere, Milz) wird das Lyse und Homogeniserungsprogramm des Prescellys 24 Lysis and Homogenization Gerätes (Bertin Technologies) ausgewählt (www.prescellys.com). Die homogenisierten Proben werden über Nacht bei 4°C stehen gelassen. 50 µL des Homogenats werden in ein Autosampler-Vial überführt und mit 150 µL Methanol inklusive ISTD aufgefüllt und 10 sec geschüttelt und darauf 5 min stehen gelassen. Nach Zugabe von 300 µL Ammoniumacetat Puffer (pH6.8) und kurzem Schütteln wird die Probe 10min bei 1881g zentrifugiert.

Zur Kalibrierung wird für Plasmaproben Plasma und für Gewebeproben entsprechende Leermatrix mit Konzentrationen von 0.6 ― 1000 µg/L versetzt. Die Nachweisgrenze (LOQ) liegt je nach Probentyp bzw. Gewebetyp zwischen 1 und 20 µg/L.

Die Messung der Plasma und Matrixproben erfolgt schließlich an dem mit einer HPLC gekoppelten Triple-Quadrupol-Massenspektrometer API4200 der Firma AB SCIEX Deutschland GmbH.

Qualitätskontrollen zur Gültigkeitsprüfung enthalten 4, 40 und 400 µg/L.

Tabelle 5: Konzentrationen des Metaboliten M1 in Tumor, Leber, Niere, Milz und Plasma 24h nach einmaliger 10mg/kg i.v. Applikation des Beispiels 1x-9024 im Vergleich zur Isotypkontrolle in REC-1 Xenograft nunu Mäusen.

**Tabelle 5**

| Beispiel | Gewebe | Beispiel M1 | | LLOQ (µg/L) |
|---|---|---|---|---|
| | | Mittelwert (µg/L) | SD (µg/L) | |
| Isotyp Kontrolle | Tumor | 54.2 | 0.7 | 20.0 |
| | Leber | 26.1 | 1.2 | 4.0 |
| | Niere | 93.3 | 9.3 | 10.0 |
| | Milz | 31.5 | 2.4 | 10.0 |
| | Plasma | 4.2 | 0.8 | 1.0 |
| Beispiel | Gewebe | Beispiel M1 | | LLOQ (µg/L) |
| | | Mittelwert (µg/L) | SD (µg/L) | |
| 1x-9024 | Tumor | 186.4 | 32.4 | 20.0 |
| | Leber | 19.8 | 3.6 | 4.0 |
| | Niere | 28.0 | 7.0 | 10.0 |
| | Milz | 20.3 | 6.3 | 10.0 |
| | Plasma | 2.9 | 0.9 | 1.0 |

### C-6 Wirksamkeitstest in vivo

Die Wirksamkeit der erfindungsgemäßen Konjugate können in vivo beispielsweise mittels Xenograft-Modellen getestet werden. Der Fachmann kennt Methoden im Stand der Technik, anhand derer die Wirksamkeit der erfindungsgemäßen Verbindungen getestet werden kann (siehe z.B. WO 2005/081711; Polson et al., Cancer Res. 2009 Mar 15;69(6):2358-64).

Humane Tumorzellen, die das Antigen für das Antikörper-Wirkstoffkonjugat exprimieren, werden subkutan in die Flanke von immunsupprimierten Mäusen, beispielsweise NMRi Nudeoder SCID-Mäusen, inokuliert. 1-10 Millionen Zellen werden aus der Zellkultur abgelöst, zentrifugiert und mit Medium oder Medium / Matrigel resuspendiert. Die Zellsuspension wird unter die Haut der Maus gespritzt.

Innerhalb von einigen Tagen wächst ein Tumor heran. Die Behandlung beginnt nach Etablierung des Tumors, ungefähr bei einer Tumorgröße von 40 mm². Um die Wirkung auf größere Tumoren zu untersuchen, kann die Behandlung auch erst bei einer Tumorgröße von 50-100 mm² begonnen werden.

Die Behandlung mit den ADCs erfolgt über die intravenöse (i.v.) Route in die Schwanzvene der Maus. Das ADC wird mit einem Volumen von 5 mL / kg appliziert.

Das Behandlungsschema richtet sich nach der Pharmakokinetik des Antikörpers-Konjugates. Als Standard wurde drei Mal in Folge jeden siebten Tag behandelt. Für eine zeitnahe Beurteilung kann sich auch ein Schema mit einer Einmalbehandlung eignen. Die Behandlung kann aber auch weiter fortgesetzt werden oder es kann sich zu einem späteren Zeitpunkt ein zweiter Zyklus mit weiteren Behandlungstagen anschließen.

Standardmäßig werden 8 Tiere pro Behandlungsgruppe eingesetzt. Neben den Gruppen, die die Wirksubstanzen bekommen, wird eine Gruppe als Kontrollgruppe nur mit dem Puffer bzw. isotonischer Salzlösung nach dem gleichen Schema behandelt.

Im Verlauf des Experiments wird die Tumorfläche regelmäßig mit einer Schieblehre in zwei Dimensionen (Länge / Breite) gemessen. Die Tumorfläche wird mittels Länge x Breite bestimmt. Der Vergleich der mittleren Tumorfläche der Behandlungsgruppe mit der Kontrollgruppe wird als T/C area angegeben.

Werden alle Gruppen des Experimentes nach Behandlungsende gleichzeitig beendet, können die Tumore entnommen und gewogen werden. Der Vergleich der mittleren Tumorgewichte der Behandlungsgruppe mit der Kontrollgruppe wird als T/C weight angegeben.

### C-6a. Wachstumshemmung / Regression von experimentellen Tumoren in der Maus

Die Tumorzellen (REC-1, MOLM-13 oder MV-4-11) werden subkutan in die Flanke von weiblichen NMRI-nude Mäusen (Janvier) inokuliert. Bei einer Tumorgröße von 40-50 mm² wird intravenös mit dem Antikörper-Wirkstoffkonjugat einmal pro Woche für zwei oder drei Wochen behandelt.

Die Behandlung mit den erfindungsgemäßen ADCs führt zu einer deutlichen Wachstumshemmung der Tumore im Vergleich zur Kontrollgruppe. Die Tabelle 6 gibt die T/C Werte an, ermittelt über die Tumorfläche am jeweiligen Tag des Versuchsendes gerechnet nach Behandlungsstart.

**Tabelle 6**

| **Beispiel** | **Tumor Modell** | **Dosis** | **Dosisschema** | **T/C area** |
|---|---|---|---|---|
| R1x-9024 | REC1 | 10 mg/kg | Q7dx3 | 0.48 (Tag 23) |
| 1x-9024 | | | | 0.19 (Tag 23) |
| 1x-9024 | REC1 | 10 mg/kg | Q7dx3 | 0.22 (Tag 24) |
| 1x-9574 | | | | 0.20 (Tag 24) |
| 1c-8988 | MV-4-11 | 5mg/kg | Q7dx2 | 0.16 (Tag 20) |
| 1c-9476 | | | | 0.21 (Tag 20) |
| 1c-8988 | MOLM-13 | 5mg/kg | Q7dx2 | 0.33 (Tag14) |
| 1c-9476 | | | | 0.5 (Tag 14) |
| 1c-8987 | | | | 0.35 (Tag 14) |

## Patentansprüche

1. Antikörper-Wirkstoff-Konjugate (ADCs) der Formel (I) 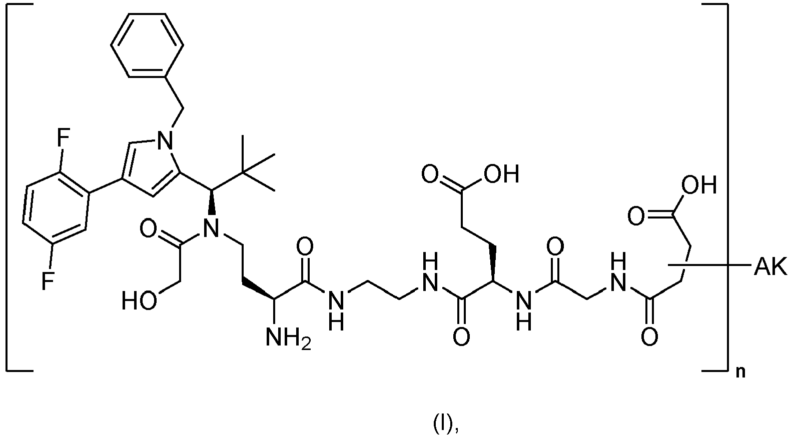 in der
n für 1 bis 8 steht,
AK für einen anti-CD123 Antikörper, ausgewählt aus der Gruppe bestehend aus TPP-8987, TPP-9476 und TPP-8988 steht, oder
AK für einen anti-CXCR5 Antikörper, bevorzugt ausgewählt aus der Gruppe bestehend aus TPP-9574, TPP-9580 und TPP-9024 steht, oder
AK für ein Antigen-bindendes Fragment von diesen Antikörpern steht,
wobei der Antikörper oder das Antigen-bindende Fragment über ein Schwefelatom einer Cystein-Seitengruppe gebunden ist,
sowie deren Salze, Solvate und Salze dieser Solvate.

2. Antikörper-Wirkstoff-Konjugate (ADCs) der Formel (I), gemäß Anspruch 1, in der n für 2 bis 8 steht.

3. Antikörper-Wirkstoff-Konjugate (ADCs) der Formel (I), gemäß Anspruch 1, in der n für 4 bis 8 steht.

4. Antikörper-Wirkstoff-Konjugate (ADCs) gemäß einem der Ansprüche 1 bis 3, wobei AK
(i) für einen anti-CD123 Antikörper umfassend eine variable Region der schwere Kette (VH) umfassend die variable CDR1-Sequenz der schweren Kette (H-CDR1), wie dargestellt durch SEQ ID NO: 2, die variable CDR2-Sequenz der schweren Kette (H-CDR2), wie dargestellt durch SEQ ID NO: 3 und die variable CDR3-Sequenz der schweren Kette (H-CDR3), wie dargestellt durch SEQ ID NO: 4, sowie eine variable Region der leichte Kette (VL) umfassend die variable CDR1-Sequenz der leichten Kette (L-CDR1), wie dargestellt durch SEQ ID NO: 6, die variable CDR2-Sequenz der leichten Kette (L-CDR2), wie dargestellt durch SEQ ID NO: 7 und die variable CDR3-Sequenz der leichten Kette (L-CDR3), wie dargestellt durch SEQ ID NO: 8,
(ii) für einen anti-CD123 Antikörper umfassend eine variable Region der schwere Kette (VH) umfassend die variable CDR1-Sequenz der schweren Kette (H-CDR1), wie dargestellt durch SEQ ID NO: 12, die variable CDR2-Sequenz der schweren Kette (H-CDR2), wie dargestellt durch SEQ ID NO: 13 und die variable CDR3-Sequenz der schweren Kette (H-CDR3), wie dargestellt durch SEQ ID NO: 14, sowie eine variable Region der leichte Kette (VL) umfassend die variable CDR1-Sequenz der leichten Kette (L-CDR1), wie dargestellt durch SEQ ID NO: 16, die variable CDR2-Sequenz der leichten Kette (L-CDR2), wie dargestellt durch SEQ ID NO: 17 und die variable CDR3-Sequenz der leichten Kette (L-CDR3), wie dargestellt durch SEQ ID NO: 18,
(iii) für einen anti-CXCR5 Antikörper umfassend eine variable Region der schwere Kette (VH) umfassend die variable CDR1-Sequenz der schweren Kette (H-CDR1), wie dargestellt durch SEQ ID NO: 22, die variable CDR2-Sequenz der schweren Kette (H-CDR2), wie dargestellt durch SEQ ID NO: 23 und die variable CDR3-Sequenz der schweren Kette (H-CDR3), wie dargestellt durch SEQ ID NO: 24, sowie eine variable Region der leichte Kette (VL) umfassend die variable CDR1-Sequenz der leichten Kette (L-CDR1), wie dargestellt durch SEQ ID NO: 26, die variable CDR2-Sequenz der leichten Kette (L-CDR2), wie dargestellt durch SEQ ID NO: 27 und die variable CDR3-Sequenz der leichten Kette (L-CDR3), wie dargestellt durch SEQ ID NO: 28,
(iv) für einen anti-CD123 Antikörper umfassend eine variable Region der schwere Kette (VH) umfassend die variable CDR1-Sequenz der schweren Kette (H-CDR1), wie dargestellt durch SEQ ID NO: 32, die variable CDR2-Sequenz der schweren Kette (H-CDR2), wie dargestellt durch SEQ ID NO: 33 und die variable CDR3-Sequenz der schweren Kette (H-CDR3), wie dargestellt durch SEQ ID NO: 34, sowie eine variable Region der leichte Kette (VL) umfassend die variable CDR1-Sequenz der leichten Kette (L-CDR1), wie dargestellt durch SEQ ID NO: 36, die variable CDR2-Sequenz der leichten Kette (L-CDR2), wie dargestellt durch SEQ ID NO: 37 und die variable CDR3-Sequenz der leichten Kette (L-CDR3), wie dargestellt durch SEQ ID NO: 38,
(v) für einen anti-CXCR5 Antikörper umfassend eine variable Region der schwere Kette (VH) umfassend die variable CDR1-Sequenz der schweren Kette (H-CDR1), wie dargestellt durch SEQ ID NO: 42, die variable CDR2-Sequenz der schweren Kette (H-CDR2), wie dargestellt durch SEQ ID NO: 43 und die variable CDR3-Sequenz der schweren Kette (H-CDR3), wie dargestellt durch SEQ ID NO: 44, sowie eine variable Region der leichte Kette (VL) umfassend die variable CDR1-Sequenz der leichten Kette (L-CDR1), wie dargestellt durch SEQ ID NO: 46, die variable CDR2-Sequenz der leichten Kette (L-CDR2), wie dargestellt durch SEQ ID NO: 47 und die variable CDR3-Sequenz der leichten Kette (L-CDR3), wie dargestellt durch SEQ ID NO: 48, oder
(vi) für einen anti-CXCR5 Antikörper umfassend eine variable Region der schwere Kette (VH) umfassend die variable CDR1-Sequenz der schweren Kette (H-CDR1), wie dargestellt durch SEQ ID NO: 52, die variable CDR2-Sequenz der schweren Kette (H-CDR2), wie dargestellt durch SEQ ID NO: 53 und die variable CDR3-Sequenz der schweren Kette (H-CDR3), wie dargestellt durch SEQ ID NO: 54, sowie eine variable Region der leichte Kette (VL) umfassend die variable CDR1-Sequenz der leichten Kette (L-CDR1), wie dargestellt durch SEQ ID NO: 56, die variable CDR2-Sequenz der leichten Kette (L-CDR2), wie dargestellt durch SEQ ID NO: 57 und die variable CDR3-Sequenz der leichten Kette (L-CDR3), wie dargestellt durch SEQ ID NO: 58,
oder für ein Antigen-bindendes Fragment von diesen Antikörpern steht.

5. Antikörper-Wirkstoff-Konjugate (ADCs) gemäß einem der Ansprüche 1 bis 4, wobei AK
(i) für einen anti-CD123 Antikörper umfassend eine variable Region der schweren Kette (VH) wie dargestellt durch SEQ ID NO: 1 sowie eine variable Region der leichten Kette (VL) wie dargestellt durch SEQ ID NO: 5,
(ii) für einen anti-CD123 Antikörper umfassend eine variable Region der schweren Kette (VH) wie dargestellt durch SEQ ID NO: 11 sowie eine variable Region der leichten Kette (VL) wie dargestellt durch SEQ ID NO: 15,
(iii) für einen anti-CXCR5 Antikörper umfassend eine variable Region der schweren Kette (VH) wie dargestellt durch SEQ ID NO: 21 sowie eine variable Region der leichten Kette (VL) wie dargestellt durch SEQ ID NO: 25,
(iv) für einen anti-CD123 Antikörper umfassend eine variable Region der schweren Kette (VH) wie dargestellt durch SEQ ID NO: 31 sowie eine variable Region der leichten Kette (VL) wie dargestellt durch SEQ ID NO: 35,
(v) für einen anti-CXCR5 Antikörper umfassend eine variable Region der schweren Kette (VH) wie dargestellt durch SEQ ID NO: 41 sowie eine variable Region der leichten Kette (VL) wie dargestellt durch SEQ ID NO: 45, oder
(vi) für einen anti-CXCR5 Antikörper umfassend eine variable Region der schweren Kette (VH) wie dargestellt durch SEQ ID NO: 51 sowie eine variable Region der leichten Kette (VL) wie dargestellt durch SEQ ID NO: 55,
oder für ein Antigen-bindendes Fragment von diesen Antikörpern steht.

6. Antikörper-Wirkstoff-Konjugate (ADCs) gemäß einem der Ansprüche 1 bis 5, wobei AK
(i) für einen anti-CD123 Antikörper umfassend eine Region der schweren Kette wie dargestellt durch SEQ ID NO: 9 sowie eine Region der leichten Kette wie dargestellt durch SEQ ID NO: 10,
(ii) für einen anti-CD123 Antikörper umfassend eine Region der schweren Kette wie dargestellt durch SEQ ID NO: 19 sowie eine Region der leichten Kette wie dargestellt durch SEQ ID NO: 20,
(iii) für einen anti-CXCR5 Antikörper umfassend eine Region der schweren Kette wie dargestellt durch SEQ ID NO: 29 sowie eine Region der leichten Kette wie dargestellt durch SEQ ID NO: 30,
(iv) für einen anti-CD123 Antikörper umfassend eine Region der schweren Kette wie dargestellt durch SEQ ID NO: 39 sowie eine Region der leichten Kette wie dargestellt durch SEQ ID NO: 40,
(v) für einen anti-CXCR5 Antikörper umfassend eine Region der schweren Kette wie dargestellt durch SEQ ID NO: 49 sowie eine Region der leichten Kette wie dargestellt durch SEQ ID NO: 50, oder
(vi) für einen anti-CXCR5 Antikörper umfassend eine Region der schweren Kette wie dargestellt durch SEQ ID NO: 59 sowie eine Region der leichten Kette wie dargestellt durch SEQ ID NO: 60,
oder für ein Antigen-bindendes Fragment von diesen Antikörpern steht.

7. Pharmazeutische Zusammensetzung umfassend mindestens ein Antikörper-Wirkstoff-Konjugat (ADC) nach einem oder mehreren der vorhergehenden Ansprüche in Kombination mit einem inerten, nicht-toxischen, pharmazeutisch geeigneten Hilfsstoff.

8. Antikörper-Wirkstoff-Konjugate (ADCs) nach einem oder mehreren der vorhergehenden Ansprüche zur Verwendung in einem Verfahren zur Behandlung und/oder Prophylaxe von Krankheiten.

9. Antikörper-Wirkstoff-Konjugate (ADCs) nach einem oder mehreren der vorhergehenden Ansprüche zur Verwendung in einem Verfahren zur Behandlung von hyperproliferativen und/oder angiogenen Erkrankungen.

10. Antikörper -Wirkstoff-Konjugate (ADCs) nach einem oder mehreren der vorhergehenden Ansprüche zur Verwendung in einem Verfahren zur Behandlung von Krebs und Tumoren.

11. Antikörper -Wirkstoff-Konjugate (ADCs) nach einem oder mehreren der vorhergehenden Ansprüche zur Verwendung in einem Verfahren zur Behandlung von Krebs und Tumoren in Kombination mit einem oder mehreren therapeutischer Ansätzen zur Krebs-Immuntherapie oder mit einem oder mehreren Wirkstoffen gerichtet gegen ein molekulares Target aus der Krebs-Immuntherapie.

## Claims

1. Antibody Drug Conjugations (ADCs) with the formula (I) 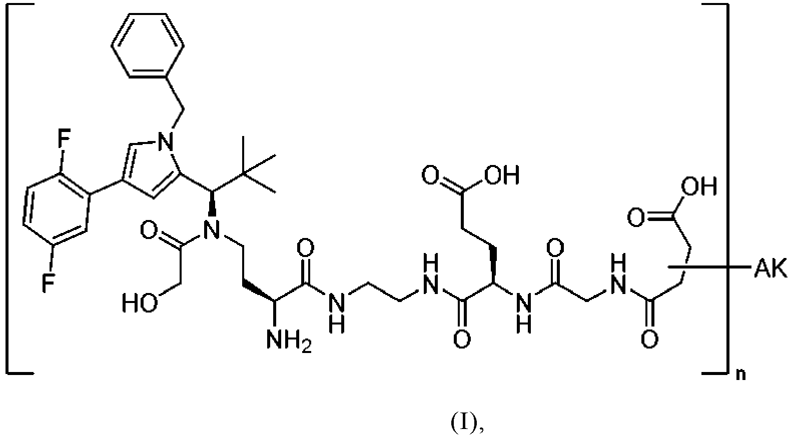 in which
n stands for 1 to 8,
AK stands for an anti-CD123 antibody, selected from the group consisting of TPP-8987, TPP-9476 and TPP-8988 ,
or
AK stands for an anti-CXCR5 antibody, preferably selected from the group consisting of TPP-9574, TPP-9580 and TPP-9024 ,
or
AK stands for an antigen-binding fragment of these antibodies,
wherein the antibody or the antigen-binding fragment is bound by a sulphur atom of a cystein side group,
as well as their salts, solvates and salts of these solvates.

2. Antibody drug conjugations (ADCs) of the formula (I), according to claim 1, in which n stands for 2 to 8.

3. Antibody drug conjugations (ADCs) of the formula (I), according to claim 1, in which n stands for 4 to 8.

4. Antibody drug conjugations (ADCs) according to any one of claims 1 to 3, wherein AK stands
(i) for an anti-CD123 antibody comprising a variable region of the heavy chain (VH) comprising the variable CDR1 sequence of the heavy chain (H-CDR1), as illustrated by SEQ ID NO: 2, the variable CDR2 sequence of the heavy chain (H-CDR2), as illustrated by SEQ ID NO: 3 and the variable CDR3 sequence of the heavy chain (H- CDR3), as illustrated by SEQ ID NO: 4, as well as a variable region of the light chain (VL) comprising the variable CDR1 sequence of the light chain (L-CDR1), as illustrated by SEQ ID NO: 6, the variable CDR2 sequence of the light chain (L-CDR2), as illustrated by SEQ ID NO: 7 and the variable CDR3 sequence of the light chain (L-CDR3), as illustrated by SEQ ID NO: 8,
(ii) for an anti-CD123 antibody comprising a variable region of the heavy chain (VH) comprising the variable CDR1 sequence of the heavy chain (H-CDR1), as illustrated by SEQ ID NO: 12, the variable CDR2 sequence of the heavy chain (H- CDR2), as illustrated by SEQ ID NO: 13 and the variable CDR3 sequence of the heavy chain (H-CDR3), as illustrated by SEQ ID NO: 14, as well as a variable region of the light chain (VL) comprising the variable CDR1 sequence of the light chain (L-CDR1), as illustrated by SEQ ID NO: 16, the variable CDR2 sequence of the light chain (L-CDR2), as illustrated by SEQ ID NO: 17 and the variable CDR3 sequence of the light chain (L-CDR3), as illustrated by SEQ ID NO: 18,
(iii) for an anti-CXCR5 antibody comprising a variable region of the heavy chain (VH) comprising the variable CDR1 sequence of the heavy chain (H-CDR1), as illustrated by SEQ ID NO: 22, the variable CDR2 sequence of the heavy chain (H- CDR2), as illustrated by SEQ ID NO: 23 and the variable CDR3 sequence of the heavy chain (H-CDR3), as illustrated by SEQ ID NO: 24, as well as a variable region of the light chain (VL) comprising the variable CDR1 sequence of the light chain (L-CDR1), as illustrated by SEQ ID NO: 26, the variable CDR2 sequence of the light chain (L-CDR2), as illustrated by SEQ ID NO: 27 and the variable CDR3 sequence of the light chain (L-CDR3), as illustrated by SEQ ID NO: 28,
(iv) for an anti-CD123 antibody comprising a variable region of the heavy chain (VH) comprising the variable CDR1 sequence of the heavy chain (H-CDR1), as illustrated by SEQ ID NO: 32, the variable CDR2 sequence of the heavy chain (H- CDR2), as illustrated by SEQ ID NO: 33 and the variable CDR3 sequence of the heavy chain (H-CDR3), as illustrated by SEQ ID NO: 34, as well as a variable region of the light chain (VL) comprising the variable CDR1 sequence of the light chain (L-CDR1), as illustrated by SEQ ID NO: 36, the variable CDR2 sequence of the light chain (L-CDR2), as illustrated by SEQ ID NO: 37 and the variable CDR3 sequence of the light chain (L-CDR3), as illustrated by SEQ ID NO: 38,
(v) for an anti-CXCR5 antibody comprising a variable region of the heavy chain (VH) comprising the variable CDR1 sequence of the heavy chain (H-CDR1), as illustrated by SEQ ID NO: 42, the variable CDR2 sequence of the heavy chain (H- CDR2), as illustrated by SEQ ID NO: 43 and the variable CDR3 sequence of the heavy chain (H-CDR3), as illustrated by SEQ ID NO: 44, as well as a variable region of the light chain (VL) comprising the variable CDR1 sequence of the light chain (L-CDR1), as illustrated by SEQ ID NO: 46, the variable CDR2 sequence of the light chain (L-CDR2), as illustrated by SEQ ID NO: 47 and the variable CDR3 sequence of the light chain (L-CDR3), as illustrated by SEQ ID NO: 48, or
(vi) for an anti-CXCR5 antibody comprising a variable region of the heavy chain (VH) comprising the variable CDR1 sequence of the heavy chain (H-CDR1), as illustrated by SEQ ID NO: 52, the variable CDR2 sequence of the heavy chain (H- CDR2), as illustrated by SEQ ID NO: 53 and the variable CDR3 sequence of the heavy chain (H-CDR3), as illustrated by SEQ ID NO: 54, as well as a variable region of the light chain (VL) comprising the variable CDR1 sequence of the light chain (L-CDR1), as illustrated by SEQ ID NO: 56, the variable CDR2 sequence of the light chain (L-CDR2), as illustrated by SEQ ID NO: 57 and the variable CDR3 sequence of the light chain (L-CDR3), as illustrated by SEQ ID NO: 58,
or stands for an antigen-binding fragment of these antibodies.

5. Antibody drug conjugations (ADCs) according to any one of claims 1 to 4, wherein AK stands
(i) for an anti-CD 123 antibody comprising a variable region of the heavy chain (VH) as illustrated by SEQ ID NO: 1 as well as a variable region of the light chain (VL) as illustrated by SEQ ID NO: 5,
(ii) for an anti-CD123 antibody comprising a variable region of the heavy chain (VH) as illustrated by SEQ ID NO: 11 as well as a variable region of the light chain (VL) as illustrated by SEQ ID NO: 15,
(iii) for an anti-CXCR5 antibody comprising a variable region of the heavy chain (VH) as illustrated by SEQ ID NO: 21 as well as a variable region of the light chain (VL) as illustrated by SEQ ID NO: 25,
(iv) for an anti-CD 123 antibody comprising a variable region of the heavy chain (VH) as illustrated by SEQ ID NO: 31 as well as a variable region of the light chain (VL) as illustrated by SEQ ID NO: 35,
(v) for an anti-CXCR5 antibody comprising a variable region of the heavy chain (VH) as illustrated by SEQ ID NO: 41 as well as a variable region of the light chain (VL) as illustrated by SEQ ID NO: 45, or
(vi) for an anti-CXCR5 antibody comprising a variable region of the heavy chain (VH) as illustrated by SEQ ID NO: 51 as well as a variable region of the light chain (VL) as illustrated by SEQ ID NO: 55,
or stands for an antigen-binding fragment of these antibodies.

6. Antibody drug conjugations (ADCs) according to any one of claims 1 to 5, wherein AK stands
(i) for an anti-CD123 antibody comprising a region of the heavy chain as illustrated by SEQ ID NO: 9 as well as a region of the light chain as illustrated by SEQ ID NO: 10,
(ii) for an anti-CD123 antibody comprising a region of the heavy chain as illustrated by SEQ ID NO: 19 as well as a region of the light chain as illustrated by SEQ ID NO: 20,
(iii) for an anti-CXCR5 antibody comprising a region of the heavy chain as illustrated by SEQ ID NO: 29 as well as a region of the light chain as illustrated by SEQ ID NO: 30,
(iv) for an anti-CD123 antibody comprising a region of the heavy chain as illustrated by SEQ ID NO: 39 as well as a region of the light chain as illustrated by SEQ ID NO: 40,
(v) for an anti-CXCR5 antibody comprising a region of the heavy chain as illustrated by SEQ ID NO: 49 as well as a region of the light chain as illustrated by SEQ ID NO: 50, or
(vi) for an anti-CXCR5 antibody comprising a region of the heavy chain as illustrated by SEQ ID NO: 59 as well as a region of the light chain as illustrated by SEQ ID NO: 60,
or stands for an antigen-binding fragment of these antibodies.

7. Pharmaceutical composition comprising at least one antibody drug conjugate (ADC) according to one or more of the preceding claims in combination with an inert, non-toxic, pharmaceutically suitable excipient.

8. Antibody drug conjugations (ADCs) according to one or more of the preceding claims for use in a method for treating and/or preventing illnesses.

9. Antibody drug conjugations (ADCs) according to one or more of the preceding claims for use in a method for treating hyperproliferative and/or angiogenic diseases.

10. Antibody drug conjugations (ADCs) according to one or more of the preceding claims for use in a method for treating cancer and tumours.

11. Antibody drug conjugations (ADCs) according to one or more of the preceding claims for use in a method for treating cancers and tumours in combination with one of more therapeutic approaches for cancer immunotherapy or with one or more active ingredients directed against a molecular target from cancer immunotherapy.

## Revendications

1. Conjugués anticorps-médicaments (ADC) de la formule (I) 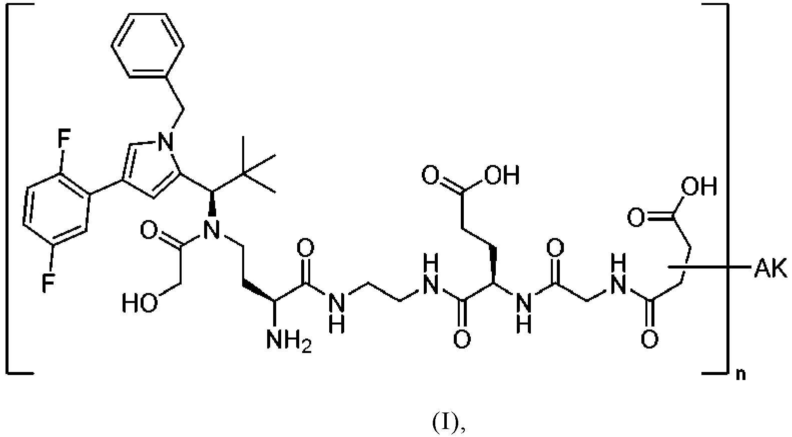 dans laquelle
n signifie 1 à 8,
AK signifie un anticorps anti-CD123 choisi dans le groupe constitué de TPP-8987, TPP-9476 et TPP-8988,
ou
AK signifie un anticorps anti-CXCR5, de préférence choisi dans le groupe constitué de TPP-9574, TPP-9580 et TPP-9024,
ou
AK signifie un fragment de liaison à l'antigène de ces anticorps,
dans lequel l'anticorps ou le fragment de liaison à l'antigène est lié via un atome de soufre d'un groupe latéral de cystéine,
et leurs sels, solvates et sels de ces solvates.

2. Conjugués anticorps-médicaments (ADC) de la formule (I) selon la revendication 1, dans laquelle n signifie 2 à 8.

3. Conjugués anticorps-médicaments (ADC) de la formule (I) selon la revendication 1, dans laquelle n signifie 4 à 8.

4. Conjugués anticorps-médicaments (ADC) selon l'une quelconque des revendications 1 à 3, dans lesquels AK
(i) signifie un anticorps anti-CD123 comprenant une région variable de la chaîne lourde (VH) comprenant la séquence variable CDR1 de la chaîne lourde (H-CDR1) telle que représentée par SEQ ID NO : 2, la séquence variable CDR2 de la chaîne lourde (H-CDR2) telle que représentée par SEQ ID NO : 3 et la séquence variable CDR3 de la chaîne lourde (H-CDR3) telle que représentée par SEQ ID NO : 4, ainsi qu'une région variable de la chaîne légère (VL) comprenant la séquence variable CDR1 de la chaîne légère (L-CDR1) telle que représentée par SEQ ID NO : 6, la séquence variable CDR2 de la chaîne légère (L-CDR2) telle que représentée par SEQ ID NO : 7 et la séquence variable CDR3 de la chaîne légère (L-CDR3) telle que représentée par SEQ ID NO : 8,
(ii) signifie un anticorps anti-CD123 comprenant une région variable de la chaîne lourde (VH) comprenant la séquence variable CDR1 de la chaîne lourde (H-CDR1) telle que représentée par SEQ ID NO : 12, la séquence variable CDR2 de la chaîne lourde (H-CDR2) telle que représentée par SEQ ID NO : 13 et la séquence variable CDR3 de la chaîne lourde (H-CDR3) telle que représentée par SEQ ID NO : 14, ainsi qu'une région variable de la chaîne légère (VL) comprenant la séquence variable CDR1 de la chaîne légère (L-CDR1) telle que représentée par SEQ ID NO : 16, la séquence variable CDR2 de la chaîne légère (L-CDR2) telle que représentée par SEQ ID NO : 17 et la séquence variable CDR3 de la chaîne légère (L-CDR3) telle que représentée par SEQ ID NO : 18,
(iii) signifie un anticorps anti-CXCR5 comprenant une région variable de la chaîne lourde (VH) comprenant la séquence variable CDR1 de la chaîne lourde (H-CDR1) telle que représentée par SEQ ID NO : 22, la séquence variable CDR2 de la chaîne lourde (H-CDR2) telle que représentée par SEQ ID NO : 23 et la séquence variable CDR3 de la chaîne lourde (H-CDR3) telle que représentée par SEQ ID NO : 24, ainsi qu'une région variable de la chaîne légère (VL) comprenant la séquence variable CDR1 de la chaîne légère (L-CDR1) telle que représentée par SEQ ID NO : 26, la séquence variable CDR2 de la chaîne légère (L-CDR2) telle que représentée par SEQ ID NO : 27 et la séquence variable CDR3 de la chaîne légère (L-CDR3) telle que représentée par SEQ ID NO : 28,
(iv) signifie un anticorps anti-CD123 comprenant une région variable de la chaîne lourde (VH) comprenant la séquence variable CDR1 de la chaîne lourde (H-CDR1) telle que représentée par SEQ ID NO : 32, la séquence variable CDR2 de la chaîne lourde (H-CDR2) telle que représentée par SEQ ID NO : 33 et la séquence variable CDR3 de la chaîne lourde (H-CDR3) telle que représentée par SEQ ID NO : 34, ainsi qu'une région variable de la chaîne légère (VL) comprenant la séquence variable CDR1 de la chaîne légère (L-CDR1) telle que représentée par SEQ ID NO : 36, la séquence variable CDR2 de la chaîne légère (L-CDR2) telle que représentée par SEQ ID NO : 37 et la séquence variable CDR3 de la chaîne légère (L-CDR3) telle que représentée par SEQ ID NO : 38,
(v) signifie un anticorps anti-CXCR5 comprenant une région variable de la chaîne lourde (VH) comprenant la séquence variable CDR1 de la chaîne lourde (H-CDR1) telle que représentée par SEQ ID NO : 42, la séquence variable CDR2 de la chaîne lourde (H-CDR2) telle que représentée par SEQ ID NO : 43 et la séquence variable CDR3 de la chaîne lourde (H-CDR3) telle que représentée par SEQ ID NO : 44, ainsi qu'une région variable de la chaîne légère (VL) comprenant la séquence variable CDR1 de la chaîne légère (L-CDR1) telle que représentée par SEQ ID NO : 46, la séquence variable CDR2 de la chaîne légère (L-CDR2) telle que représentée par SEQ ID NO : 47 et la séquence variable CDR3 de la chaîne légère (L-CDR3) telle que représentée par SEQ ID NO : 48, ou
(vi) signifie un anticorps anti-CXCR5 comprenant une région variable de la chaîne lourde (VH) comprenant la séquence variable CDR1 de la chaîne lourde (H-CDR1) telle que représentée par SEQ ID NO : 52, la séquence variable CDR2 de la chaîne lourde (H-CDR2) telle que représentée par SEQ ID NO : 53 et la séquence variable CDR3 de la chaîne lourde (H-CDR3) telle que représentée par SEQ ID NO : 54, ainsi qu'une région variable de la chaîne légère (VL) comprenant la séquence variable CDR1 de la chaîne légère (L-CDR1) telle que représentée par SEQ ID NO : 56, la séquence variable CDR2 de la chaîne légère (L-CDR2) telle que représentée par SEQ ID NO : 57 et la séquence variable CDR3 de la chaîne légère (L-CDR3) telle que représentée par SEQ ID NO : 58,
ou un fragment de liaison à l'antigène de ces anticorps.

5. Conjugués anticorps-médicaments (ADC) selon l'une des revendications 1 à 4, dans lesquels AK
(i) signifie un anticorps anti-CD123 comprenant une région variable de la chaîne lourde (VH) telle que représentée par SEQ ID NO : 1 ainsi qu'une région variable de la chaîne légère (VL) telle que représentée par SEQ ID NO : 5,
(ii) signifie un anticorps anti-CD123 comprenant une région variable de la chaîne lourde (VH) telle que représentée par SEQ ID NO : 11 ainsi qu'une région variable de la chaîne légère (VL) telle que représentée par SEQ ID NO : 15,
(iii) signifie un anticorps anti-CXCR5 comprenant une région variable de la chaîne lourde (VH) telle que représentée par SEQ ID NO : 21 ainsi qu'une région variable de la chaîne légère (VL) telle que représentée par SEQ ID NO : 25,
(iv) signifie un anticorps anti-CD123 comprenant une région variable de la chaîne lourde (VH) telle que représentée par SEQ ID NO : 31 ainsi qu'une région variable de la chaîne légère (VL) telle que représentée par SEQ ID NO : 35,
(v) signifie un anticorps anti-CXCR5 comprenant une région variable de la chaîne lourde (VH) telle que représentée par SEQ ID NO : 41 ainsi qu'une région variable de la chaîne légère (VL) telle que représentée par SEQ ID NO : 45, ou
(vi) signifie un anticorps anti-CXCR5 comprenant une région variable de la chaîne lourde (VH) telle que représentée par SEQ ID NO : 51 ainsi qu'une région variable de la chaîne légère (VL) telle que représentée par SEQ ID NO : 55,
ou un fragment de liaison à l'antigène de ces anticorps.

6. Conjugués anticorps-médicaments (ADC) selon l'une quelconque des revendications 1 à 5, dans lesquels AK
(i) signifie un anticorps anti-CD123 comprenant une région de la chaîne lourde telle que représentée par SEQ ID NO : 9 ainsi qu'une région de la chaîne légère telle que représentée par SEQ ID NO : 10,
(ii) signifie un anticorps anti-CD123 comprenant une région de la chaîne lourde telle que représentée par SEQ ID NO : 19 ainsi qu'une région de la chaîne légère telle que représentée par SEQ ID NO : 20,
(iii) signifie un anticorps anti-CXCR5 comprenant une région de la chaîne lourde telle que représentée par SEQ ID NO : 29 ainsi qu'une région de la chaîne légère telle que représentée par SEQ ID NO : 30,
(iv) signifie un anticorps anti-CD123 comprenant une région de la chaîne lourde telle que représentée par SEQ ID NO : 39 ainsi qu'une région de la chaîne légère telle que représentée par SEQ ID NO : 40,
(v) signifie un anticorps anti-CXCR5 comprenant une région de la chaîne lourde telle que représentée par SEQ ID NO : 49 ainsi qu'une région de la chaîne légère telle que représentée par SEQ ID NO : 50, ou
(vi) signifie un anticorps anti-CXCR5 comprenant une région de la chaîne lourde telle que représentée par SEQ ID NO : 59 ainsi qu'une région de la chaîne légère telle que représentée par SEQ ID NO : 60,
ou un fragment de liaison à l'antigène de ces anticorps.

7. Composition pharmaceutique comprenant au moins un conjugué anticorps-médicament (ADC) selon l'une ou plusieurs des revendications précédentes en combinaison avec un excipient inerte, non toxique, pharmaceutiquement acceptable.

8. Conjugués anticorps-médicaments (ADC) selon l'une ou plusieurs des revendications précédentes pour une utilisation dans un procédé destiné au traitement et/ou à la prophylaxie de maladies.

9. Conjugués anticorps-médicaments (ADC) selon l'une ou plusieurs des revendications précédentes pour une utilisation dans un procédé destiné au traitement de maladies hyperprolifératives et/ou angiogéniques.

10. Conjugués anticorps-médicaments (ADC) selon l'une ou plusieurs des revendications précédentes pour une utilisation dans un procédé destiné au traitement de cancers et tumeurs.

11. Conjugués anticorps-médicaments (ADC) selon l'une ou plusieurs des revendications précédentes pour une utilisation dans un procédé destiné au traitement de cancers et tumeurs en combinaison avec une ou plusieurs approches thérapeutiques pour l'immunothérapie anticancéreuse ou avec un ou plusieurs médicaments dirigés contre une cible moléculaire provenant de l'immunothérapie anticancéreuse.
